# EUROPEAN PATENT SPECIFICATION

(11) **EP 1 469 066 B1**
(45) Date of publication and mention of the grant of the patent: **09.04.2008**
(21) Application number: 04016461.8
(22) Date of filing: 10.01.1997
(51) Int. Cl.: C12N 9/26, C12N 1/20, C07H 21/04

(54) **Endoglucanase from Thermotoga**
Endoglukanase von Thermotoga
Endoglucanase de Thermotoga

(30) Priority: 11.01.1996 US 583787; 13.09.1996 US 56916 P
(43) Date of publication of application: 20.10.2004
(62) Divisional of application: 97906876.4
(73) Proprietor: Verenium Corporation, San Diego, CA 92121 (US)
(72) Inventor: Bylina, Edward J., Henderson, NV 89074 (US); Swanson, Ronald V., Del Mar, CA 92014 (US); Mathur, Eric J., Carlsbad, CA 92009 (US); Lam, David E., San Marcos, CA 92078 (US)
(74) Representative: Vossius & Partner

(56) References cited:
- EP-A- 0 606 008
- EP-A- 0 687 732
- WO-A-97/20918
- WO-A-97/25417
- WO-A-97/44361
- WO-A-98/24799
- DAKHOVA O N ET AL: "CLONING AND EXPRESSION IN ESCHERICHIA COLI OF THERMOTOGA NEAPOLITANA GENES CODING FOR ENZYMES OF CARBOHYDRATE SUBSTRATE DEGRADATION" BIOCHEMICAL AND BIOPHYSICAL RESEARCH COMMUNICATIONS,US,ACADEMIC PRESS INC. ORLANDO, FL, vol. 194, no. 3, 16 August 1993 (1993-08-16), pages 1359-1364, XP002026991 ISSN: 0006-291X -& DAKHOVA ET AL.: "Thermotoga neapolitana bgA1 gene" EMBL SEQUENCE DATABASE, 1 July 1997 (1997-07-01), XP002154622 HEIDELBERG DE -& DAKHOVA ET AL.: "T. neapolitana lamA gene and bg1B gene" EMBL SEQUENCE DATABASE, 31 December 1995 (1995-12-31), XP002154623 HEIDELBERG DE
- VOORHORST W G B ET AL: "CHARACTERIZATION OF THE CELB GENE CODING FOR BETA-GLUCOSIDASE FROM THE HYPERTHERMOPHILIC ARCHAEON PYROCOCCUS FURIOSUS AND ITS EXPRESSION AND SITE-DIRECTED MUTATION IN ESCHERICHIA COLI" JOURNAL OF BACTERIOLOGY,US,WASHINGTON, DC, vol. 177, no. 24, 1 December 1995 (1995-12-01), pages 7105-7111, XP002064570 ISSN: 0021-9193
- GRAEBNITZ F ET AL: "STRUCTURE OF THE BETA-GLUCOSIDASE GENE BGLA OF CLOSTRIDIUM THERMOCELLUM. SEQUENCE ANALYSIS REVEALS A SUPERFAMILY OF CELLULASES AND BETA-GLYCOSIDASES INCLUDING HUMAN LACTASE/PHLORIZIN HYDROLASE" EUROPEAN JOURNAL OF BIOCHEMISTRY,DE,BERLIN, vol. 200, no. 2, 1 September 1991 (1991-09-01), pages 301-309, XP000874574 ISSN: 0014-2956
- CANGANELLA F ET AL: "CHARACTERIZATION OF AMYLOLYTIC AND PULLULYTIC ENZYMES FROM THERMOPHILIC ARCHAEA AND FROM A NEW FERVIDOBACTERIUM SPECIES" APPLIED MICROBIOLOGY AND BIOTECHNOLOGY,DE,SPRINGER VERLAG, BERLIN, vol. 42, no. 2/03, 1994, pages 239-245, XP000602919 ISSN: 0175-7598
- BRONNEMEIER K ET AL: "PURIFICATION OF THERMOTOGA MARITIMA ENZYMES FOR THE DEGRADATION OF CELLULOSIC MATERIALS" APPLIED AND ENVIRONMENTAL MICROBIOLOGY, WASHINGTON,DC, US, vol. 61, no. 4, April 1995 (1995-04), pages 1399-1407, XP001005565 ISSN: 0099-2240
- CARANSA A ET AL: "A NOVEL ENZYME APPLICATION FOR CORN WET MILLING" STARCH STARKE, WILEY-VCH VERLAG, WEINHEIM, DE, vol. 40, no. 11, 1988, pages 409-411, XP002030182 ISSN: 0038-9056
- BAUER ET AL.: "Comparison of a beta-glucosidase and a beta-mannosidase from hyperthermophilic archaeon Pyrococcus furiosus" THE JOURNAL OF BIOLOGICAL CHEMISTRY, vol. 271, no. 39, 27 September 1996 (1996-09-27), pages 23749-23755, XP002154624 -& BAUER ET AL.: "Pyrococcus furiosus beta mannosidase (bmnA) gene, complete ccds" EMBL SEQUENCE DATABASE, 2 July 1996 (1996-07-02), XP002154625 HEIDELBERG DE -& BAUER ET AL.: "Beta-mannosidase" EMBL SEQUENCE DATABASE, 1 November 1996 (1996-11-01), XP002154626 HEIDELBERG DE

## Description

This invention relates to a newly identified polynucleotide, a polypeptide encoded by such polynucleotide, the use of such polynucleotide and polypeptide, as well as the production and isolation of such polynucleotide and polypeptide. More particularly, the polynucleotide and polypeptide of the present invention has been putatively identified as glucosidase, α-galactosidase, β-mannosidase, β-mannanase, or endoglucanase.

The glycosidic bond of β-galactosides can be cleaved by different classes of enzymes: (i) phospho-β-galactosidases (EC3.2.1.85) which are specific for a phosphorylated substrate generated via phosphoenolpyruvate phosphotransferase system (PTS)-dependent uptake; (ii) typical β-galactosidases (EC 3.2.1.23), represented by the Escherichia *coli* LacZ enzyme, which are relatively specific for β-galactosides; and (iii) β-glucosidases (EC 3.2.1.21) such as the enzymes of Agrobacterium *faecalis, Clostridium thermocellum, Pyrococcus furiosus* or Sulfolobus solfataricus (Day, A.G. and Withers, S..G., (1986) Purification and characterization of a β-glucosidase from Alcaligenes faecalis. Can. J. Biochem. Cell. Biol. 64, 914-922; Kengen, S.W.M., et al. (1993) Eur. J. Biochem., 213, 305-312; Ait, N., Cruezet, N. and Cattaneo, J. (1982) Properties of β-glucosidase purified from Clostridium thermocellum. J. Gen. Microbiol. 128, 569-577; Grogan D.W. (1991) Evidence that β-galactosidase of Sulfolobus solfataricus is only one of several activities of a thermostable β-D-glycosidase.Appl. Environ. Microbiol. 57, 1644-1649). Members of the latter group, although highly specific with respect to the β-anomeric configuration of the glycosidic linkage, often display a rather relaxed substrate specificity and hydrolyse. β-glucosides as well as β-fucosides and β-galactosides.

Generally, α-galactosidases are enzymes that catalyze the hydrolysis of galactose groups on a polysaccaride backbone or hydrolyze the cleavage of di- or oligosaccharides comprising galactose.

Generally, β-mannanases are enzymes that catalyze the hydrolysis of mannose groups internally on a polysaccaride backbone or hydrolyze the cleavage of di- or oligosaccaharides comprising mannose groups. β-mannasidases hydrolyze non-reducing, terminal mannose residues on a mannose -containing polysaccharide and cause the cleavage of di- or oligosaccaharides comprising mannose groups.

Guar gum is a branched galactomannan polysaccharide composed of β-1,4 linked mannose backbone with α-1,6 linked galactose sidechains. The enzymes required for the degradation of guar are β-mannazuLse, β-mannosidase and α-galactosidase. β-mmannase hydrolyses the mannose backbone internally and β-mannosidase hydrolyses non-reducing, terminal mannose residues. α-galactosidase hydrolyses α-linked galactose groups.

Galactomannan polysaccharides and the enzymes that degrade them have a variety of applications. Guar is commonly used as a thickening agent in food and is utilized in hydraulic fracturing in oil and gas recovery. Consequently, galactomannanases are industrially relevant for the degradation and modification of guar. Furthermore, a need exists for thermostable galactomannases that are active in extreme conditions associated with drilling and well stimulation.

There are other applications for these enzymes in various industries, such as in the beet sugar industry. 20-30% of the domestic U.S. sucrose consumption is sucrose from sugar beets. Raw beet sugar can contain a small amount of raffinose when the sugar beets are stored before processing and rotting begins to set in. Raffinose inhibits the crystallization of sucrose and also constitutes a hidden quantity of sucrose. Thus, there is merit to eliminating raffinose from raw beet sugar. α-Galactosidase has also been used as a digestive aid to break down raffinose, stachyose, and verbascose in such foods as beans and other gassy foods.

β-Galactosidases which are active and stable at high temperatures appear to be superior enzymes for the production of lactose-free dietary milk products (Chaplin, M.F. and Bucke, C. (1990) In: Enzyme Technology, pp. 159-160, Cambridge University Press, Cambridge, UK). Also, several studies have demonstrated the applicability of β-galactosidases to the enzymatic synthesis of oligosaccharides via transglycosylation reactions (Nilsson, R.G.I. (1988) Enzymatic synthesis of oligosaccharides. Trends Biotechnol. 6, 156-264; Cote, G.L. and Tao, B.Y. (1990) Oligosaccharide synthesis by enzymatic transglycosylation. Glycoconjugate J. 7, 145-162). Despite the commercial potential, only a few β-galactoaidases of thermophiles have been characterized so far. Two genes reported are β-galactoside-cleaving enzymes of the hyperthermophilic bacterium *Thermotoga maritima,* one of the most thermophilic organotrophic eubacteria described to date (Huber, R., Langworthy, T.A., König, H., Thomm, M., Woese, C.R., Sleytr, U.B. and Stetter, K.O. (1986) T. maritima sp. nov. represents a new genus of unique extremely thermophilic eubacteria growing up to 90°C, Arch. Microbiol. 144, 324-333) one of the most thermophilic organotrophic eubacteria described to date. The gene products have been identified as a β-galactosidaee and a β-glucouidase;

Pullulanase is well known as a debranching enzyme of pullulan and starch. The enzyme hydrolyzes α-1,6-glucosidic linkages on these polymers. Starch degradation for the production or sweeteners (glucose or maltose) is a very important industrial application of this enzyme. The degradation of starch is developed in two stages. The first stage involves the liquefaction of the substrate with α-amylase, and the second stage, or saccharification stage, in performed by β-amylase with pullalanase added as a debranching enzyme, to obtain better yields.

Endoglucanases can be used in a variety of industrial applications. For instance, the endoglucanases of the present invention can hydrolyze the internal β-1,4-glycosidic bonds in cellulose, which may be used for the conversion of plant biomass into fuels and chemicals. Endoglucanases also have applications in detergent formulations, the textile industry, in animal feed, in waste treatment, and in the fruit juice and brewing industry for the clarification and extraction of juices.

The polynucleotides and polypeptides described herein have been identified as glucosidases, α-galactosidases, β-galactosidases, β-mannosidases, β-mannanases, endoglucanases, and pullulanases as a result of their enzymatic activity.

In accordance with one aspect of the present invention, there is provided a novel enzyme, as well as active fragments, analogs and derivatives thereof.

In accordance with another aspect of the present invention, there are provided isolated nucleic acid molecules encoding the enzyme4 of the present invention including mRNAs, cDNAs, genomic DNAs as well as active analogs and fragments of such enzymes.

In accordance with another aspect of the present invention there are provided isolated nucleic acid molecules encoding mature polypeptides expressed by the DNA contained in ATCC Deposit No. 97379.

In accordance with yet a further aspect of the present invention, there is provided a process for producing such polypeptides by recombinant techniques comprising culturing recombinant prokaryotic and/or eukaryotic host cells, containing a nucleic acid sequence of the present invention, under conditions promoting expression of said enzymes and subsequent recovery of, said enzymes.

In accordance with yet a further aspect of the present invention, there is provided a process for utilizing such enzymes, or polynucleotides encoding such enzymes for hydrolyzing lactose to galactose and glucose for use in the food processing industry, the pharmaceutical industry, for example, to treat intolerance to lactose, as a diagnostic reporter molecule, in corn wet milling, in the fruit juice industry, in baking, in the textile industry and in the detergent industry.

In accordance with yet a further aspect of the present invention, there is provided a process for utilizing such enzymes for hydrolyzing guar gum (a galactomannan polysaccharide) to remove non-reducing terminal mannose residues. Further polysaccharides such as galactomannan and the enzymes according to the invention that degrade them have a varitey of applications. Guar gum is commonly used as a thickening agent in food and also is utilized in hydraulic fracturing in oil and gas recovery. Consequently, mannanases are industrially relevant for the degradation and modification of guar gums. Furthermore, a need exists for thermostable mannases that are active in extreme conditions associated with drilling and well stimulation.

In accordance with yet a further aspect of the present invention, there are also provided nucleic acid probes comprising nucleic acid molecules of sufficient length to specifically hybridize to a nucleic acid sequence of the present invention.

In accordance with yet a further aspect of the present invention, there is provided a process for utilizing such enzymes, or polynucleotides encoding such enzymes, for in vitro purposes related to scientific research, for example, to generate probes for identifying similar sequences which might encode similar enzymes from other organisms by using certain regions, i.e., conserved sequence regions, of the nucleotide sequence.

### Brief Description of the Drawings

Figure 1 is an illustration of the full-length Ma and corresponding deduced amino acid sequence of M11TL of the present invention. Sequencing was performed using a 378 automated DNA sequencer for all sequences of the present invention (Applied Biosystems, Inc.).

Figure 2 is an illustration of the full-length DNA and corresponding deduced amino acid sequence of OC1/4V-33B/G.

Figure 3 is an illustration of the full-length DNA and corresponding deduced amino acid sequence of F1-12G.

Figure 4 are illustrations of the full-length DNA and corresponding deduced amino acid sequence of 9N2-31B/G.

Figure 5 are illustrations of the full-length DNA and corresponding deduced amino acid sequence of MSB8-6G.

Figure 6 are illustrations of the full-length DNA and corresponding deduced amino acid sequence of AEDII12RA-18B/G.

Figure 7 is an illustration of the full-length DNA and corresponding deduced amino acid sequence of GC74-22G.

Figure 8 is an illustration of the full-length DNA and corresponding deduced amino acid sequence of VC1-7G1.

Figure 9 is an illustration of the full-length DNA and corresponding deduced amino acid sequence of 37GP1.

Figure 10 is an illustration of the full-length DNA and corresponding deduced amino acid sequence of 6GC2.

Figure 11 is an illustration of the full-length DNA and corresponding deduced amino acid sequence of 6GP2.

Figure 12 is an illustration of the full-length DNA and corresponding deduced amino acid sequence of 63GB1.

Figure 13 is an illustration of the full-length DNA and corresponding deduced amino acid sequence of OCl/4V.

Figure 14 is an illustration of the full-length DNA and corresponding deduced amino acid sequence of 6GP3.

### Definitions

The term "gene" means the segment of DNA involved in producing a polypeptide chain; it includes regions preceding and following the coding region (leader and trailer) as well as intervening sequences (introns) between individual coding segments (exons).

A coding sequence is "operably linked to" another coding sequence when RNA polymerase will transcribe the two coding sequences into a single IDRRA, which is then translated into a single polypeptide having amino acids derived from both coding sequences. The coding sequences need not be contiguous to one another as long as the expressed sequences ultimately process to produce the desired protein.

"Recombinant" enzymes refer to enzymes produced by recombinant DNA techniques; i.e., produced from cells transformed by an exogenous DNA construct encoding the desired enzyme. "Synthetic" enzymes are those prepared by chemical synthesis.

A DNA "coding sequence of" or a "nucleotide sequence encoding" a particular enzyme, is a DNA sequence which is transcribed and trani3lated into an enzyme when placed under the control of appropriate regulatory sequences.

### Summary of the Invention

In accordance with an aspect of the present invention there is provided an isolated nucleic acid (polynucleotide having SEQ ID NO: 13) which encodes for the mature enzyme having the deduced amino acid sequences of SEQ ID NO:27.

In accordance with another aspect of the present invention, there is provided an isolated polynucleotide encoding the enzyme of the present invention. The deposited material is a mixture of genomic clones comprising DNA encoding an enzyme of the present invention. Each genomic clone comprising the respective DNA has been inserted into a pBluescript vector (Stratagene, La Jolla, CA). The deposit has been deposited with the American Type Culture Collection. 12301 Parklawn Drive, Rockville, Maryland 20852, USA**,** on December 13, 1995 and assigned ATCC Deposit No. 97379.

The deposit (8) have been made under the terms of the Budapest Treaty on the International Recognition of the deposit of micro-organisms for purposes of patent procedure. The strains will be irrevocably and without restriction or condition released to the public upon the issuance of a patent. These deposits are provided merely as convenience to those of skill in the art and are not an admission that a deposit be required under 35 U.S.C. 5112. The sequences of the polynucleotides contained in the deposited materials, as well as the amino acid, sequences of the polypeptides encoded thereby, serve as controls in the event of any conflict with any description of sequences herein. A license may be required to make, use or sell the deposited materials, and no such license is hereby granted.

### Detailed Description of the Invention

The polynucleotides described herein were originally recovered from genomic gene libraries derived from the following organisms:
M11TL is a new species of *Desulfurococcus* isolated from Diamond Pool in Yellowstone National Park. The organism grows optimally at 85-88°C, pH 7.0 in a low salt medium containing yeast extract, peptone, and gelatin as substrates with a N₂/CO₂ gas phase.
OC1/4V is from the genus *Thermotoga*. The organism was isolated from Yellowstone National Park. It grows optimally at 75°C in a low salt medium with cellulose as a substrate and *N₂* in gas phase.
*Pyrococcus furiosus* VC1 is from the genus *Pyrococcus*. VC1 was isolated from Vulcano, Italy. It grows optimally at 100°C in a high salt medium (marine) containing elemental sulfur, yeast extract, peptone and starch as substrates and N₂ in gas phase.
*Staphylothermus marinus* F1, is a from the genus *Staphylothermus.* F1 was isolated from Vulcano, Italy. It grows optimally at 85°C, pH 6.5 in high salt medium (marine) containing elemental sulfur and yeast extract as substrates and N₂ in gas phase.
*Thermococcus* 9N-2 is from the genus *Thermococcus* 9N-2 was isolated from diffuse vent fluid in the East Pacific Rise. It is a strict anaerobe that grows optimally at 87°C.
*Thermotoga maritima* MSB8 is from the genus *Thermotogo,* and was isolated from Vulcano, Italy. MSB8 grows optimally at 85°C, pH 6.5 in a high salt medium (marine) containing starch and yeast extract as substrates and N₂ in gas phase.
*Thermococcus alcaliphilus* AEDII12RA is from the genus *Thermococcus*. AEDII12RA grows optimally at 85°C, pH 9.5 in a high salt medium (marine) containing polysulfides and yeast extract as substrates and N₂ in gas phase.
*Thermococcus chitonophagus* GC74 is from the genus *Thermococcus*. GC74 grows optimally at 85°C, pH 6.0 in a high salt medium (marine) containing chitin, meat extract, elemental sulfur and yeast extract as substrates and N₂ in gas phase. AEPII 1a grows optimally at 85°C at pH 6.5 in marine medium under anaerobic conditions. It has many substrates.

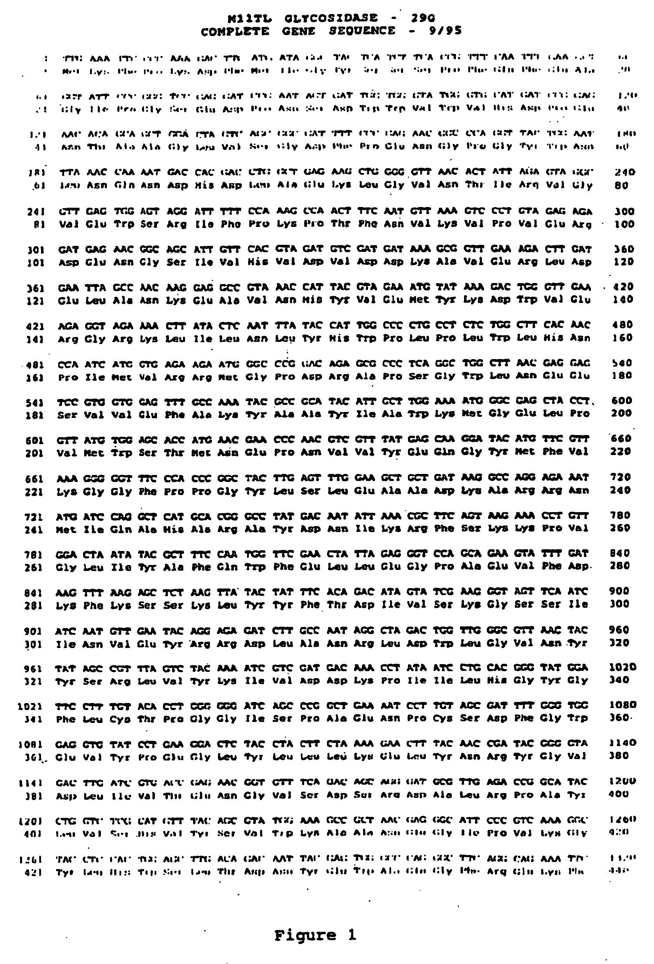

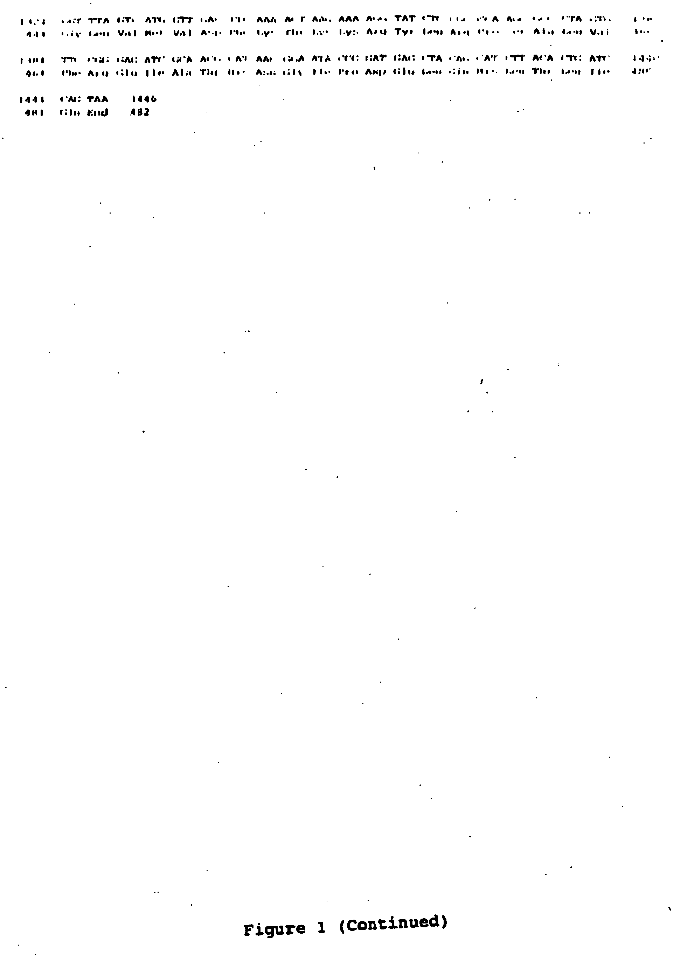

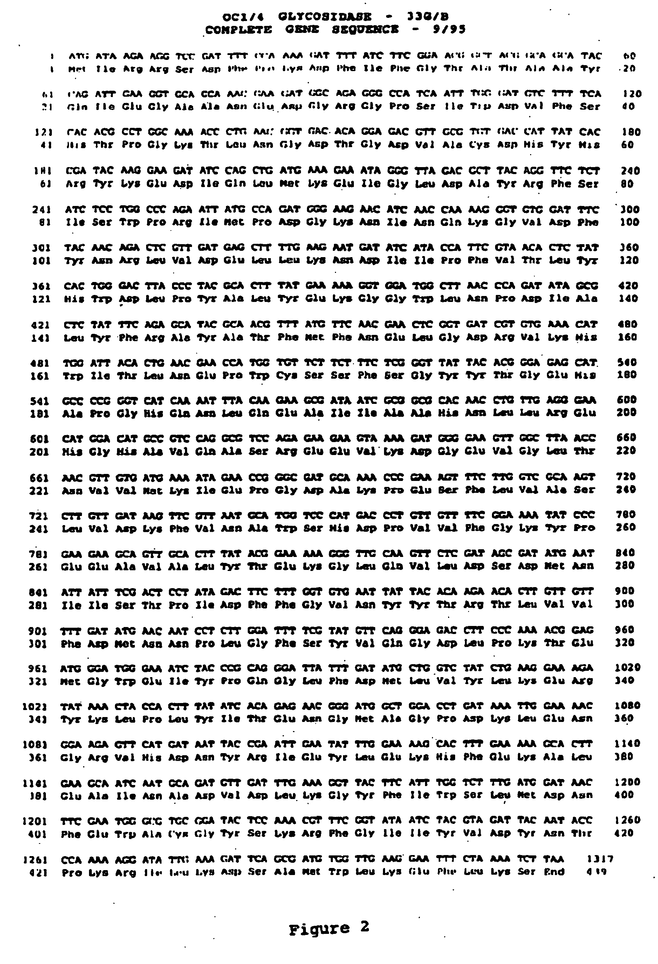

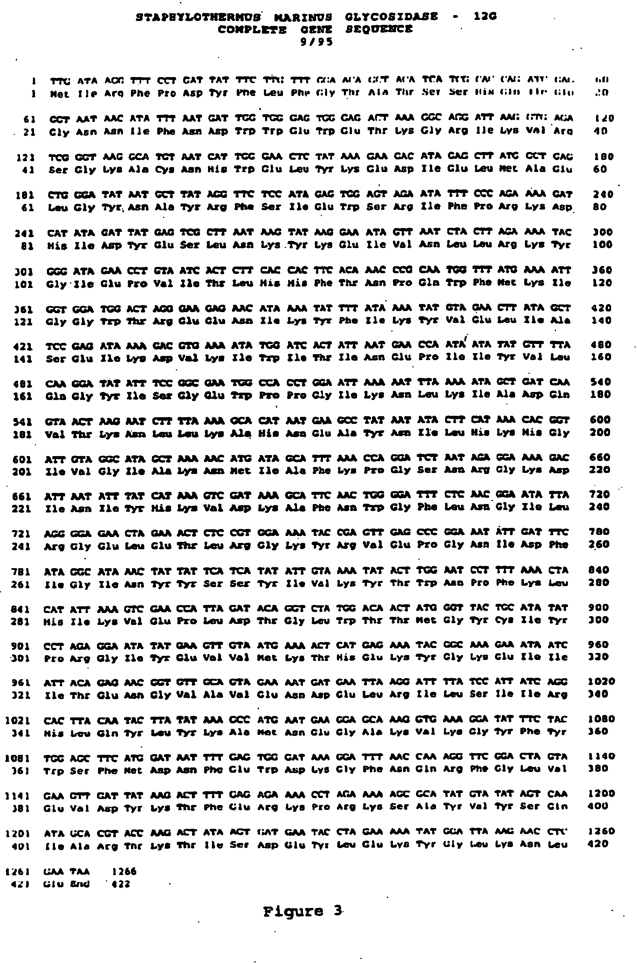

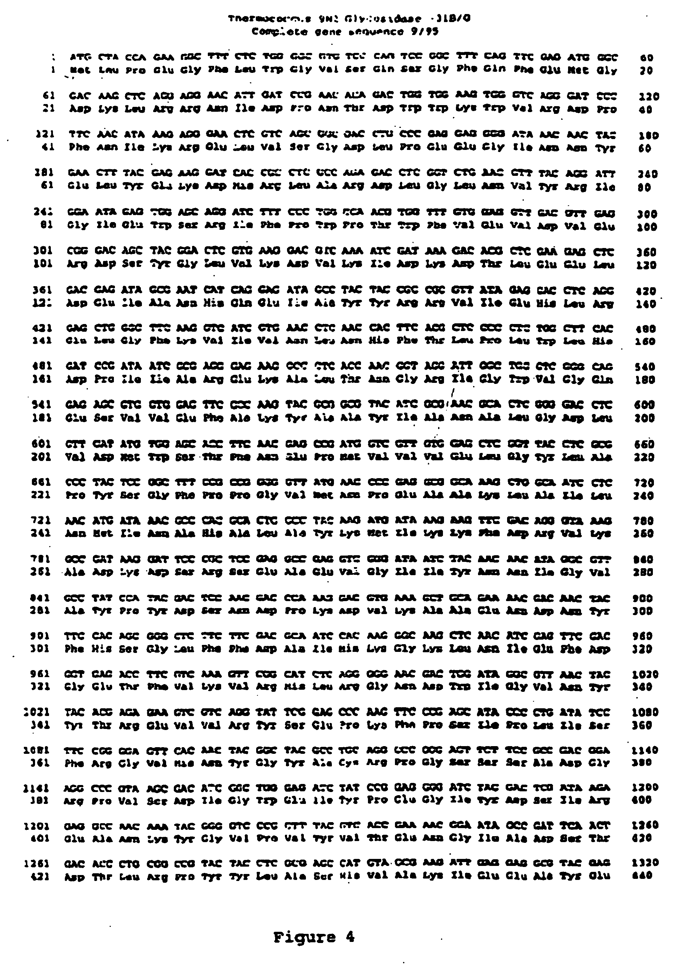

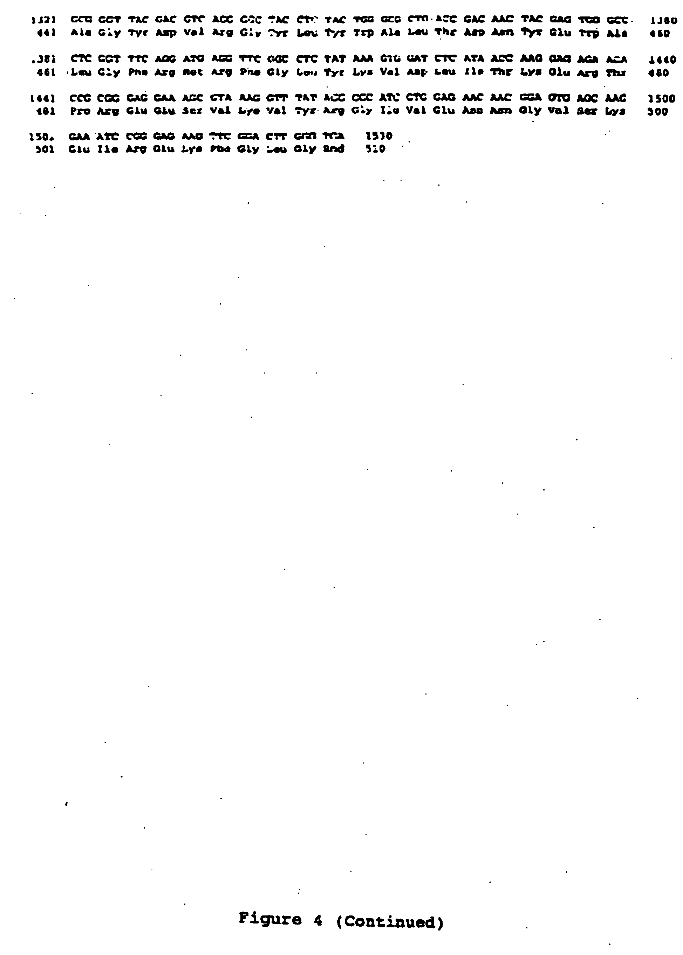

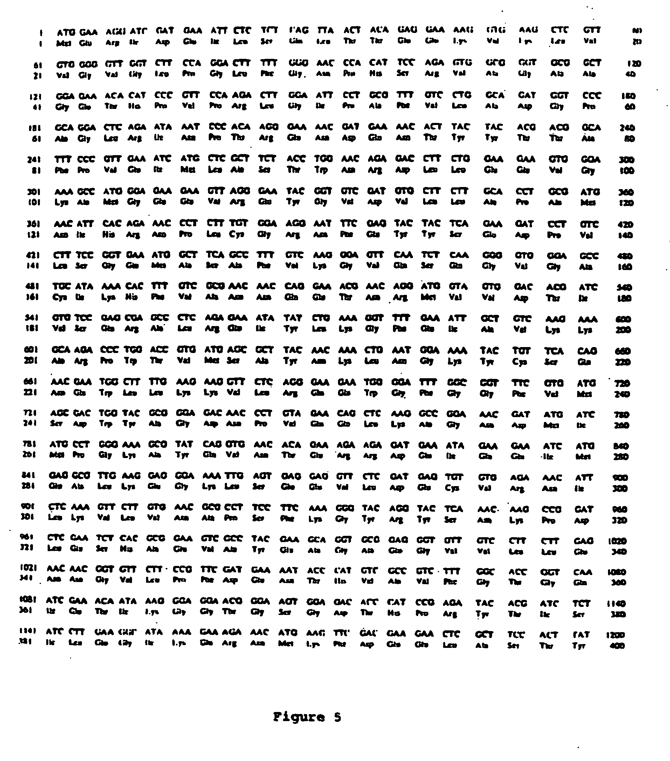

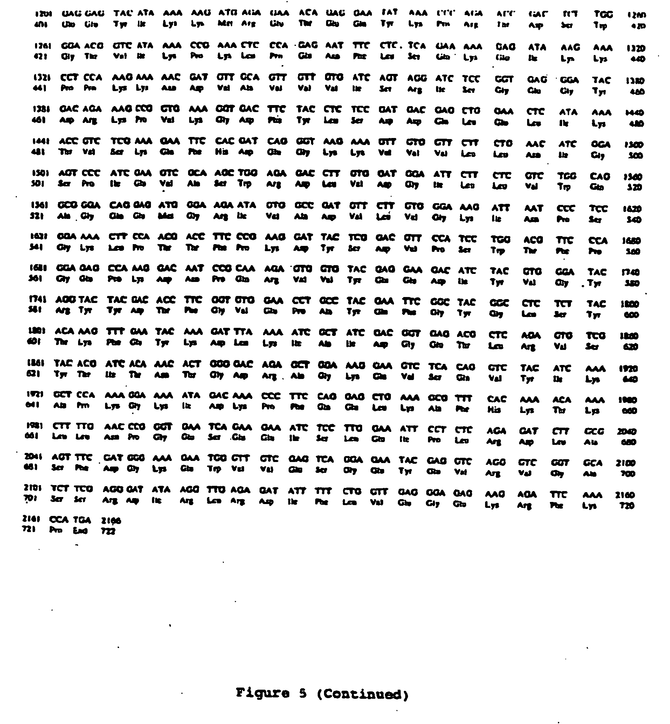

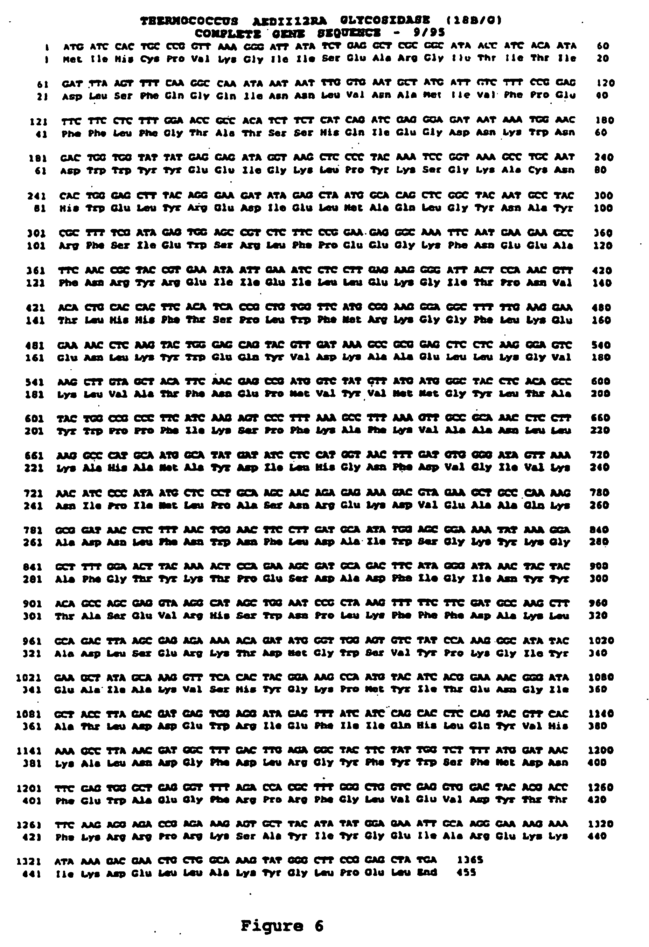

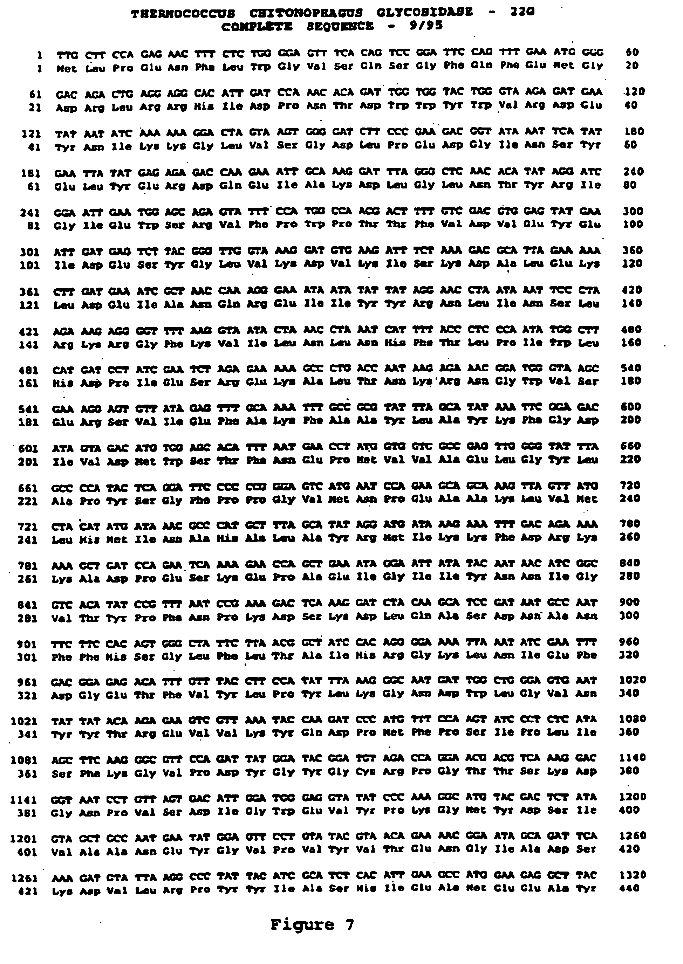

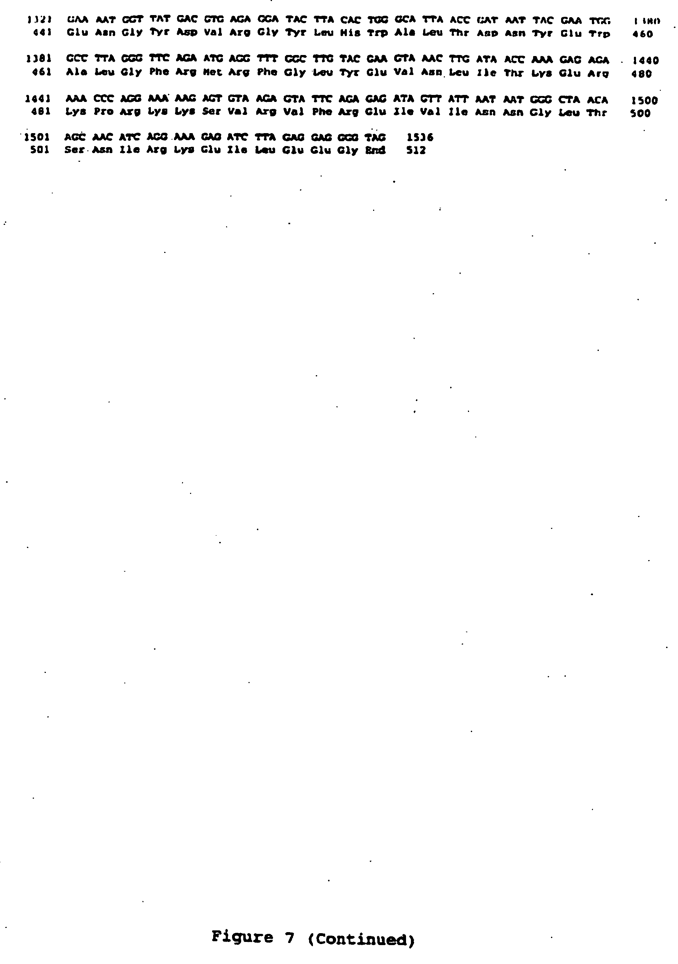

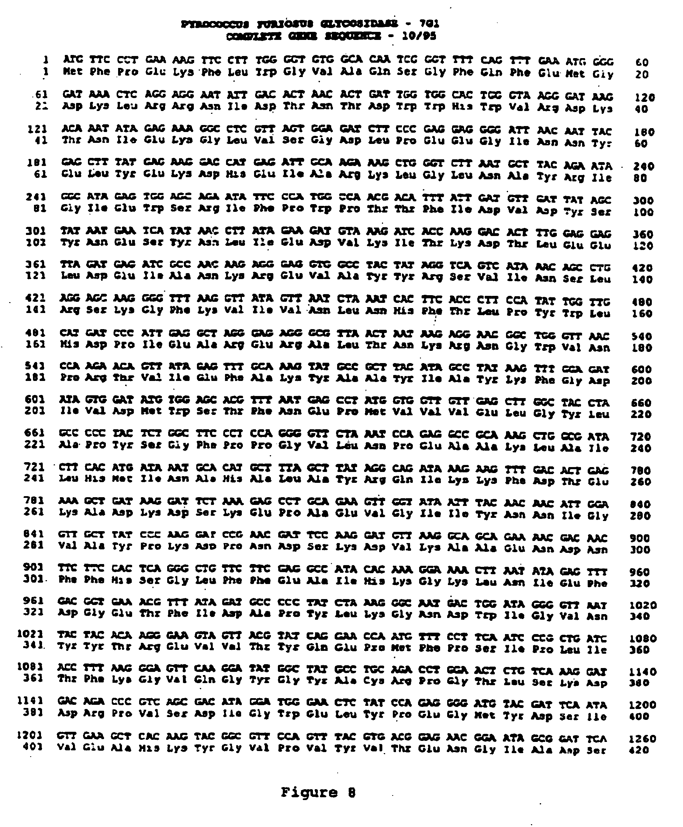

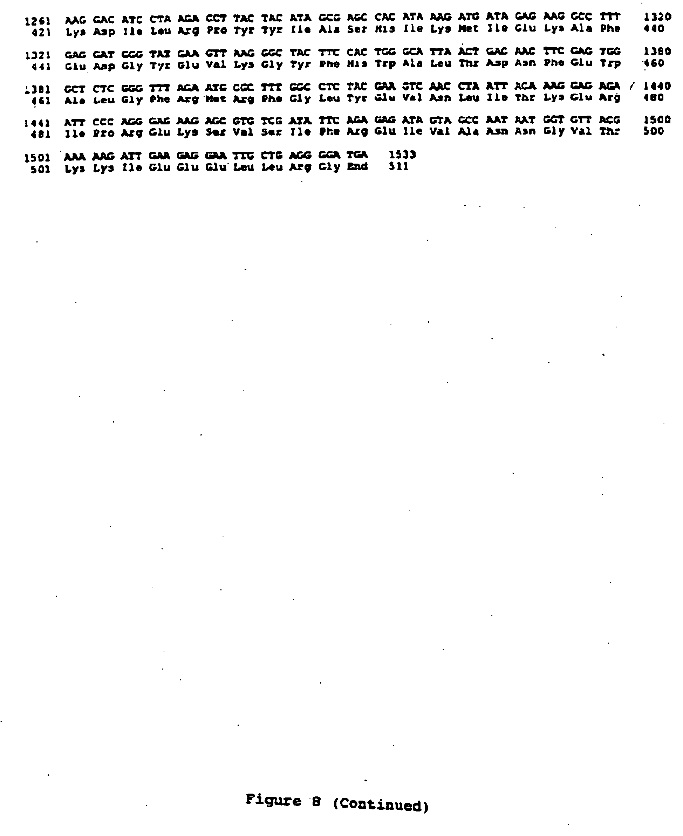

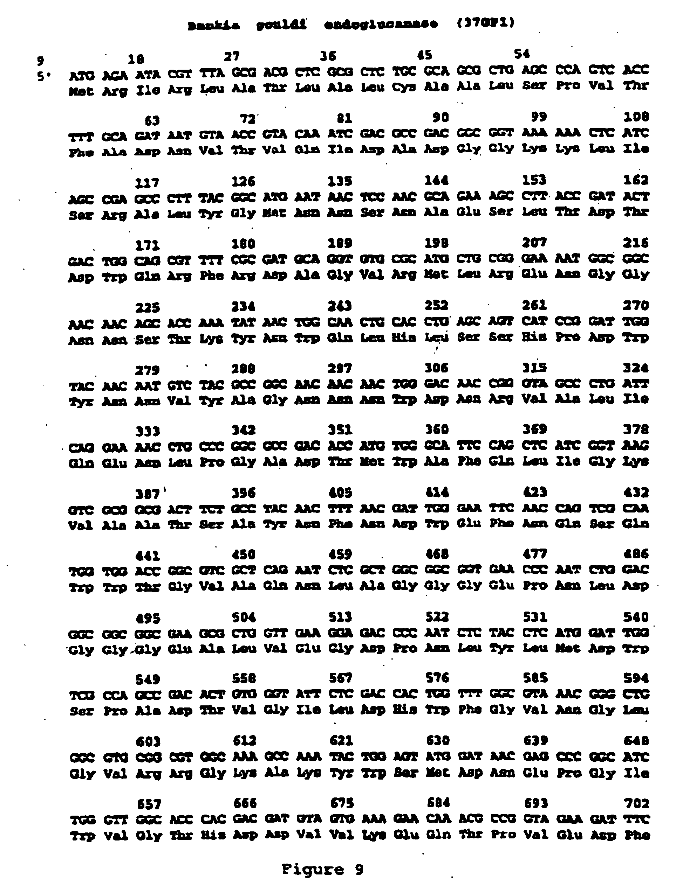

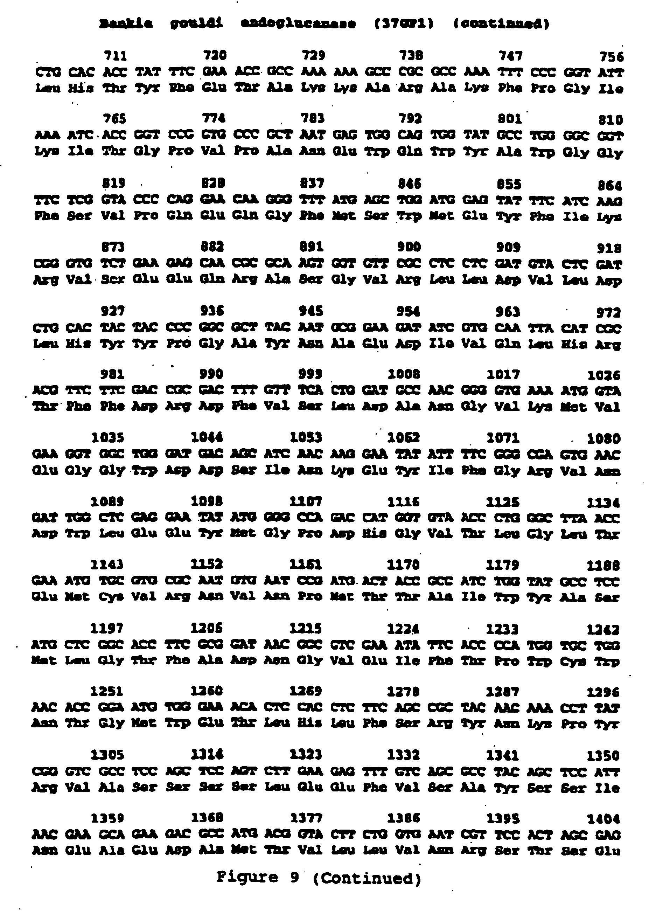

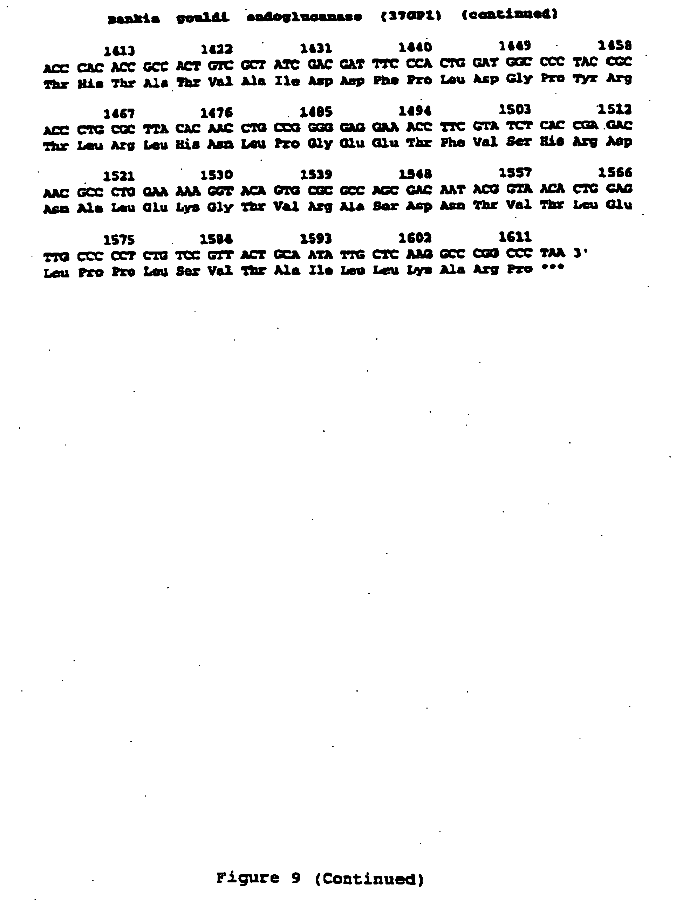

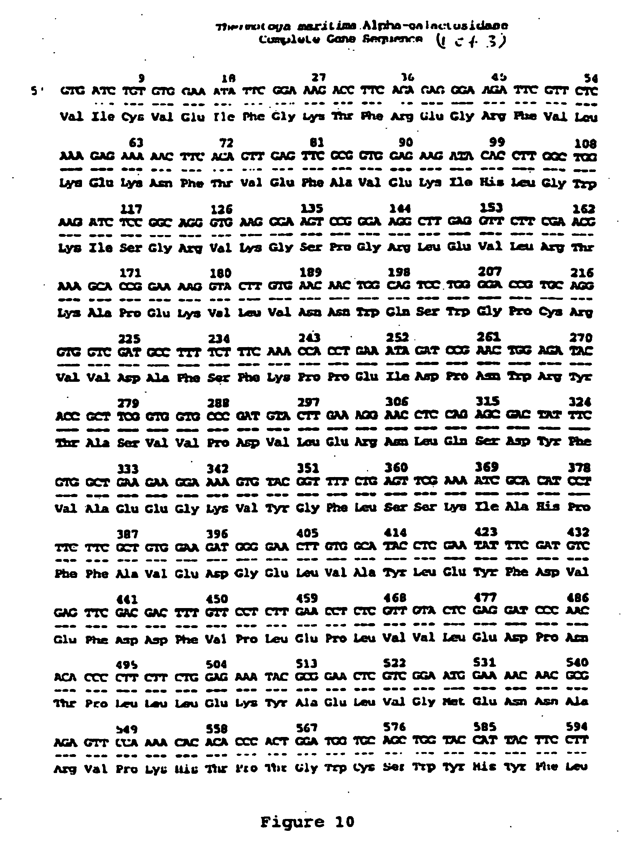

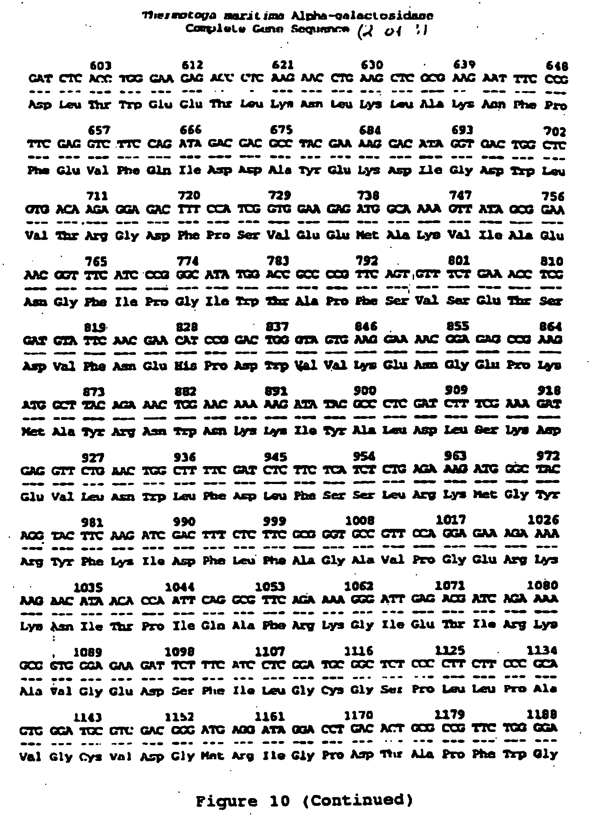

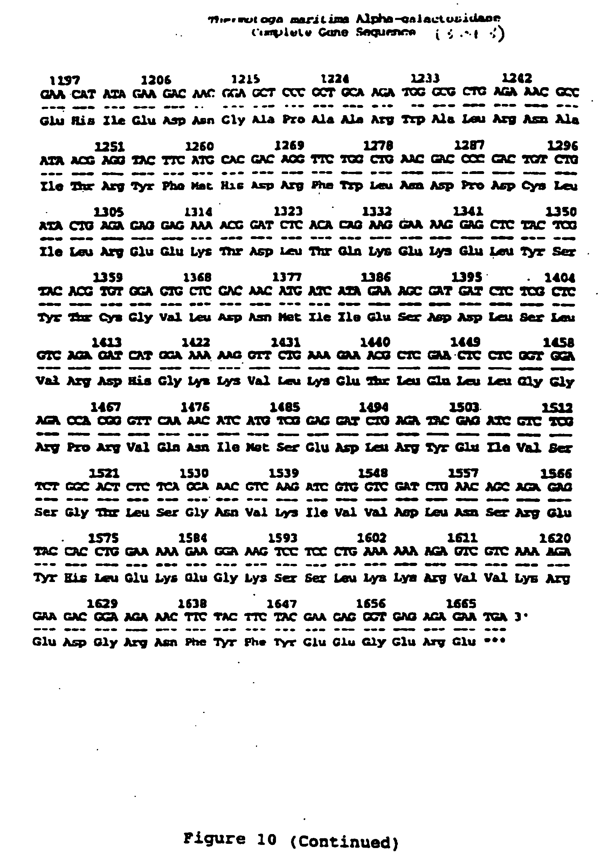

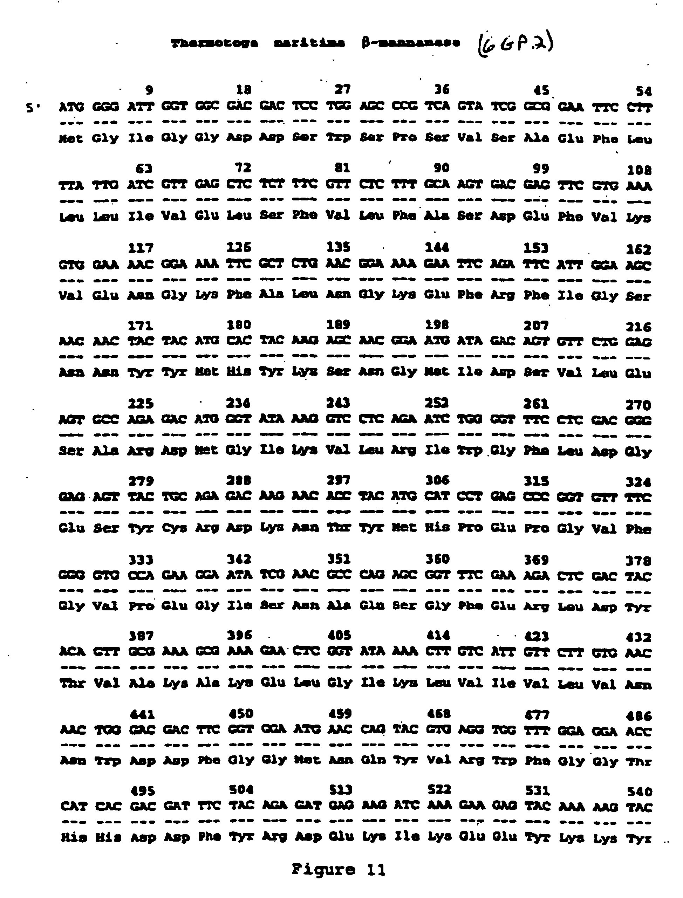

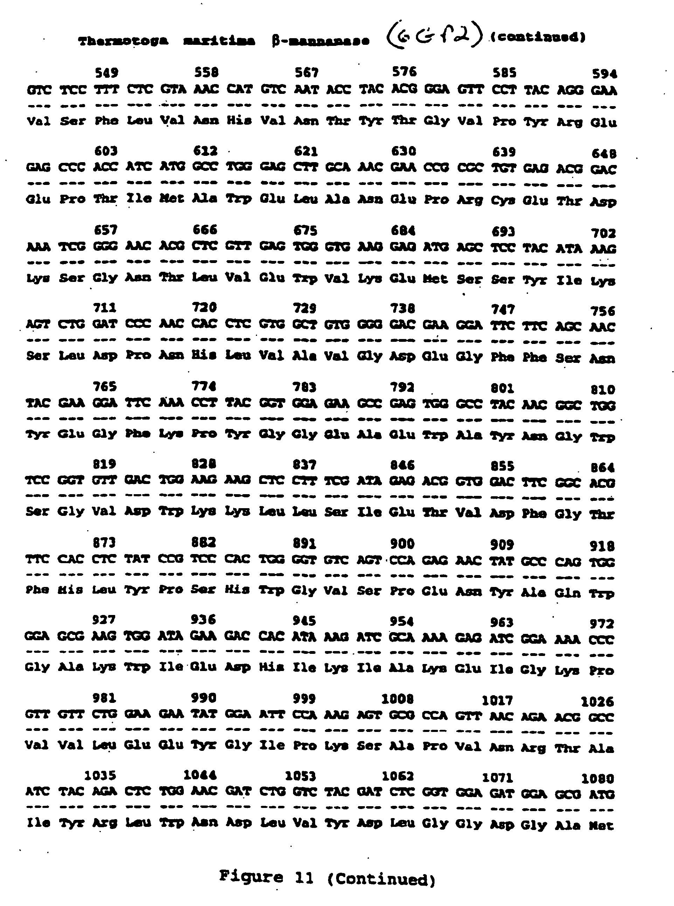

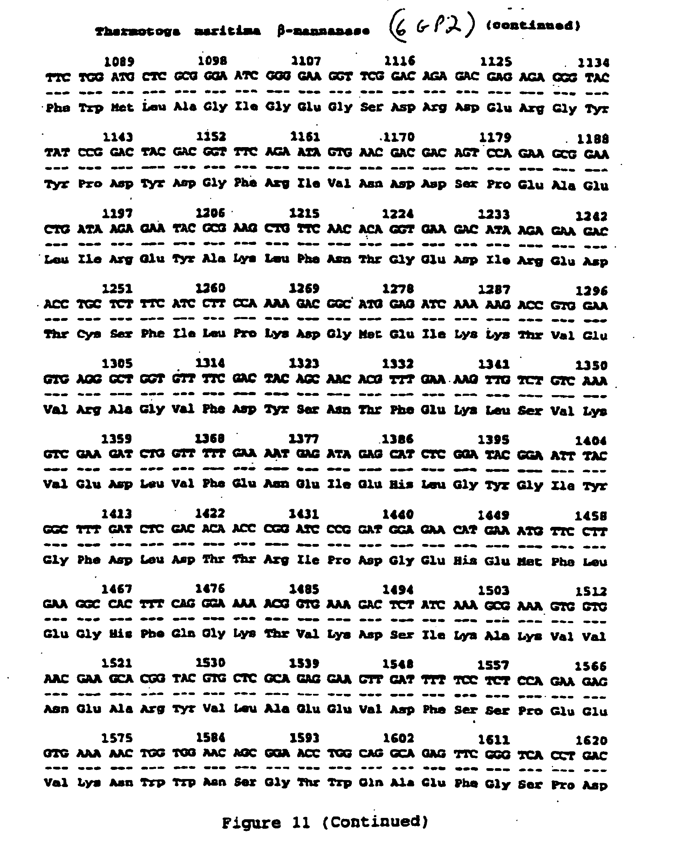

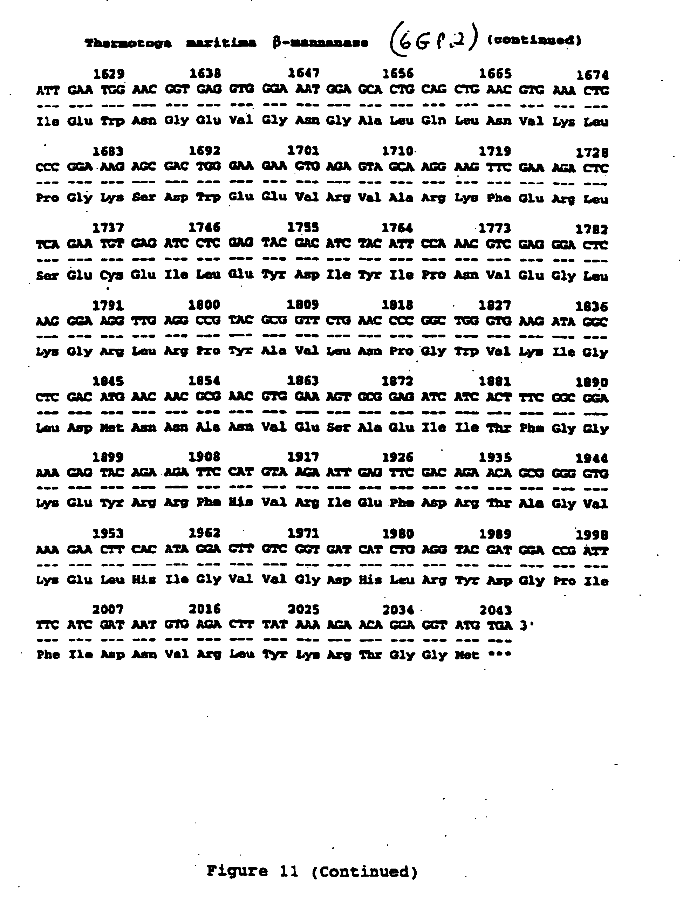

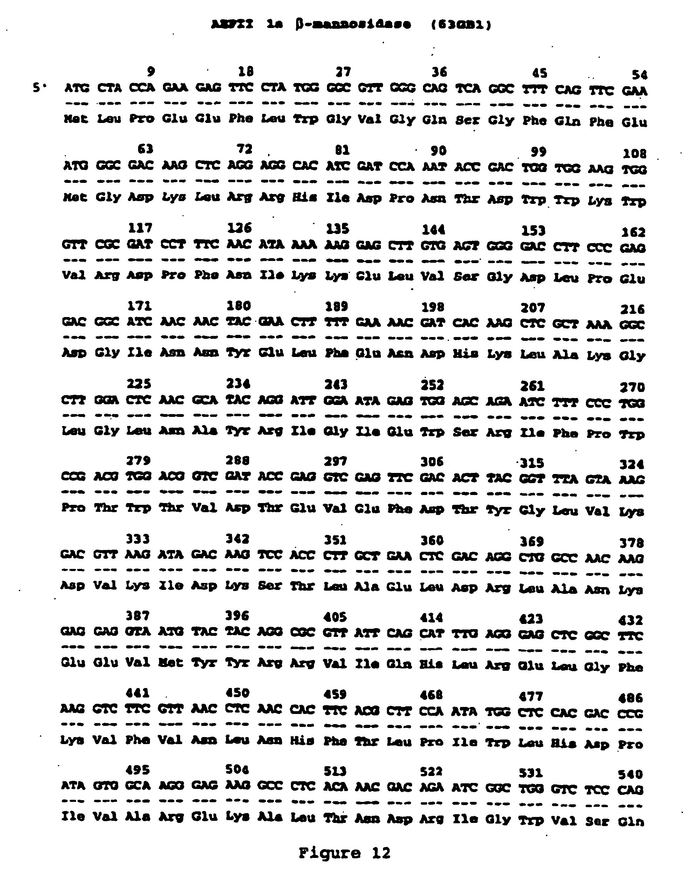

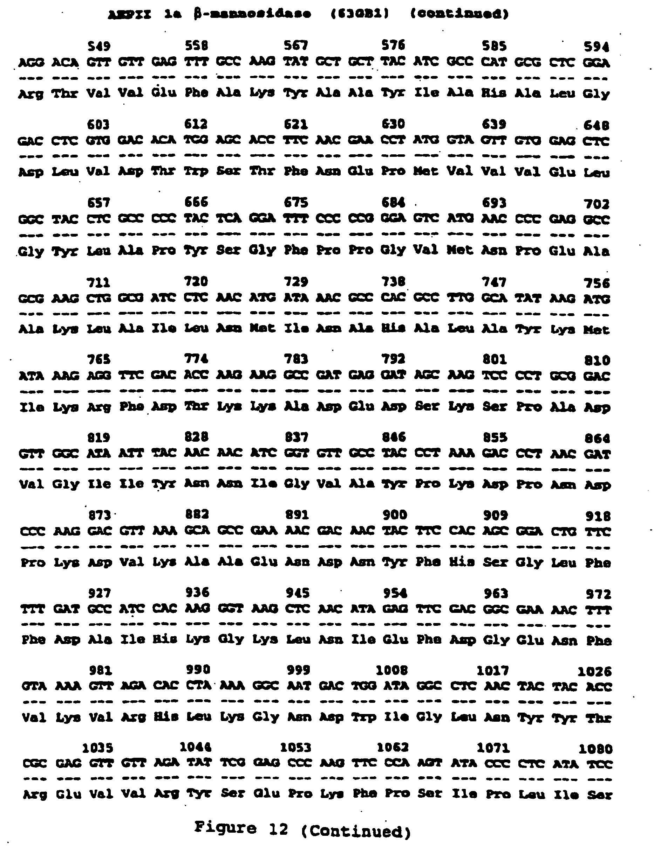

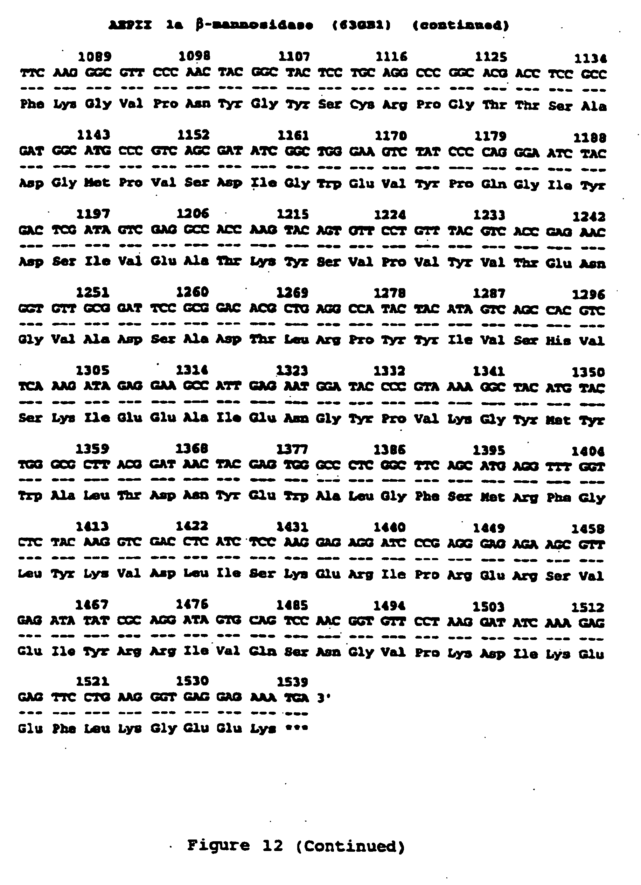

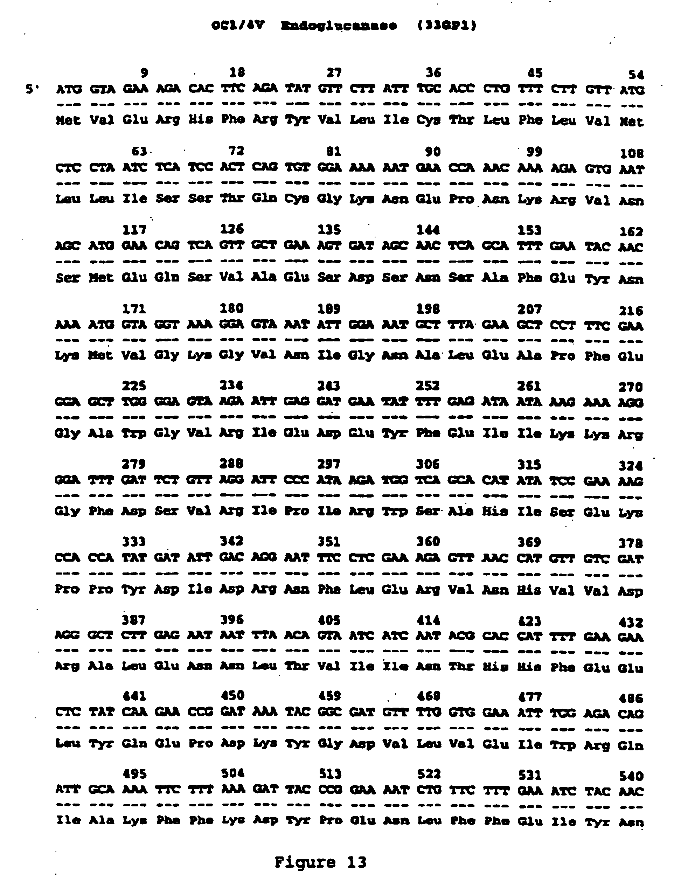

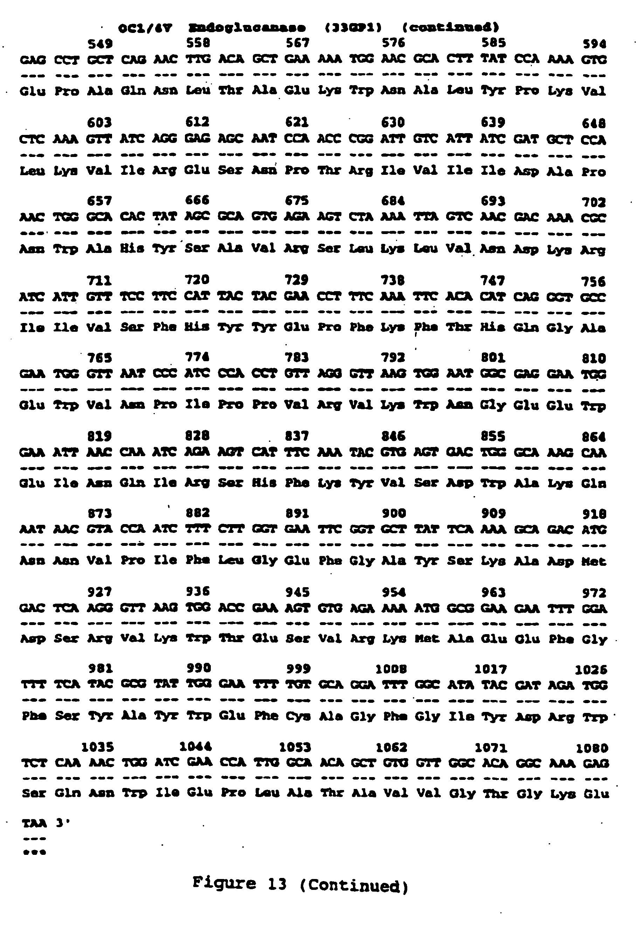

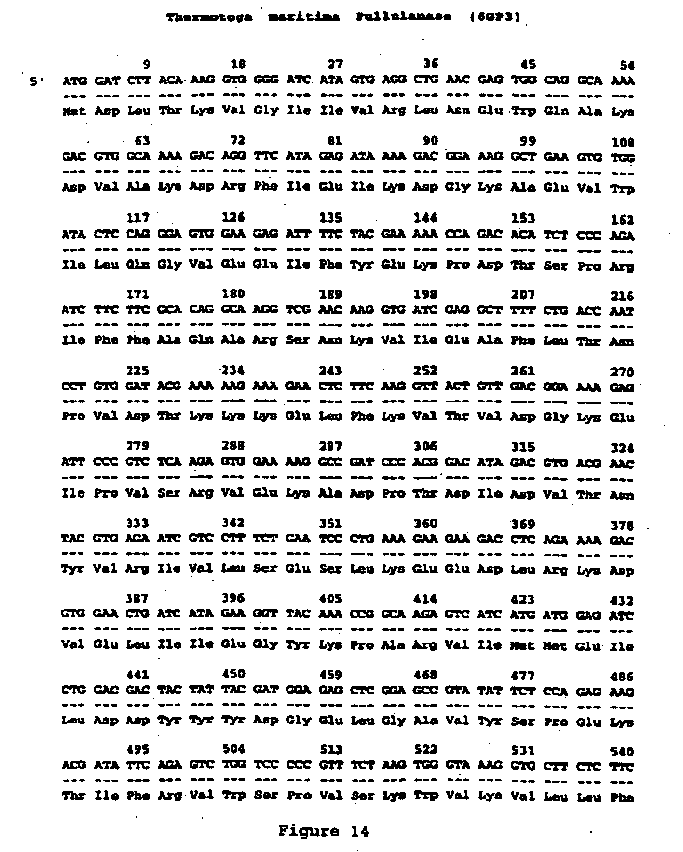

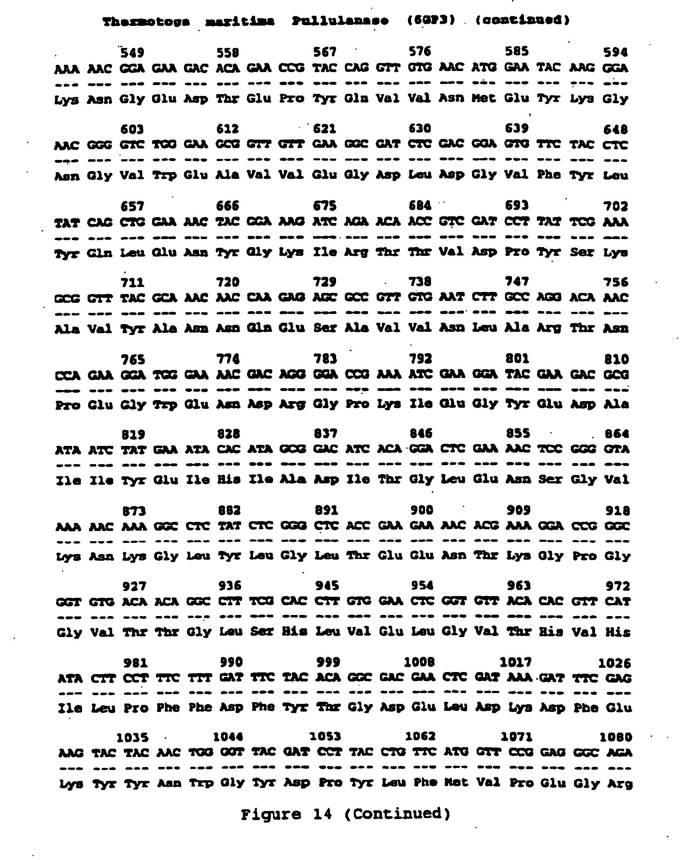

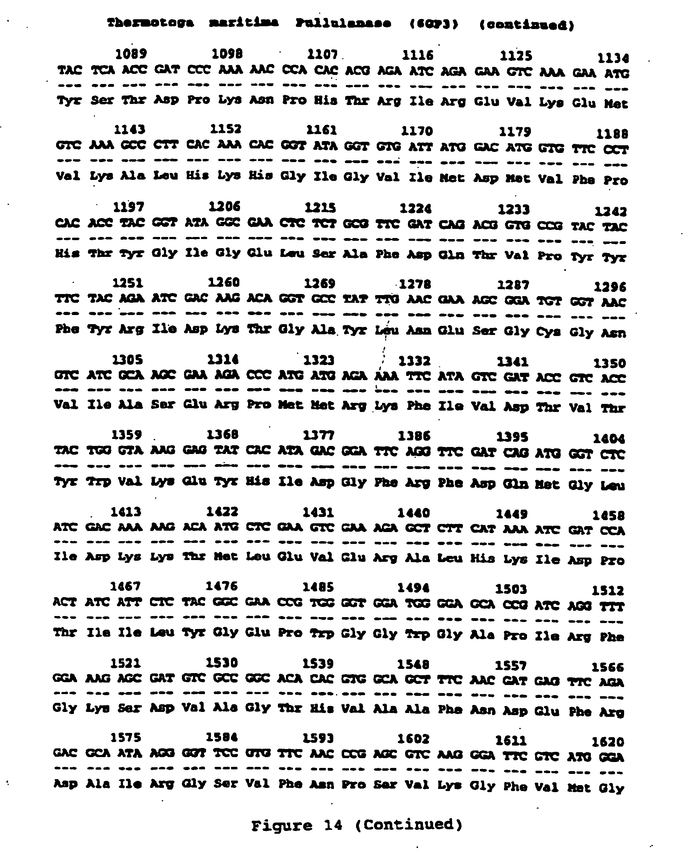

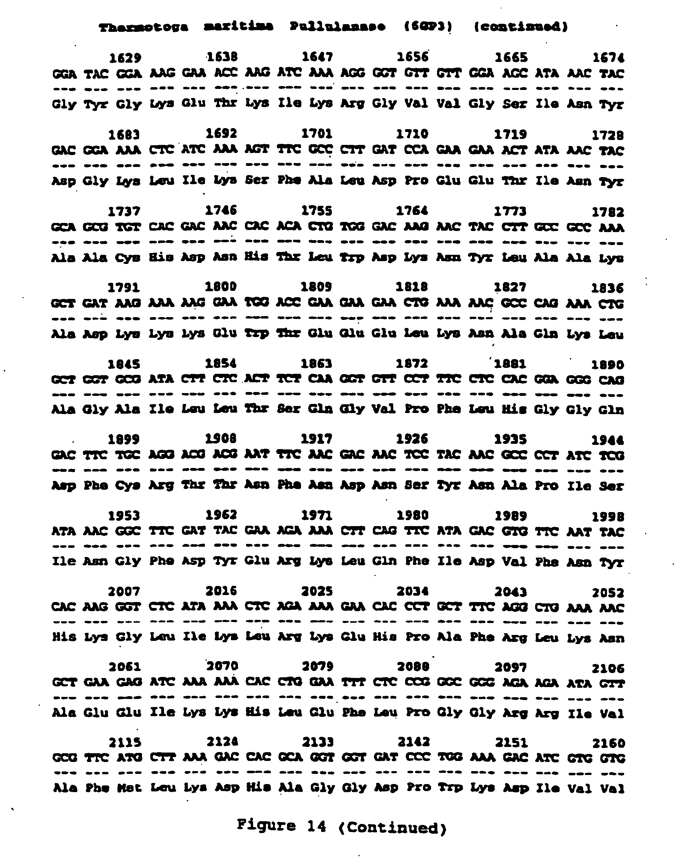

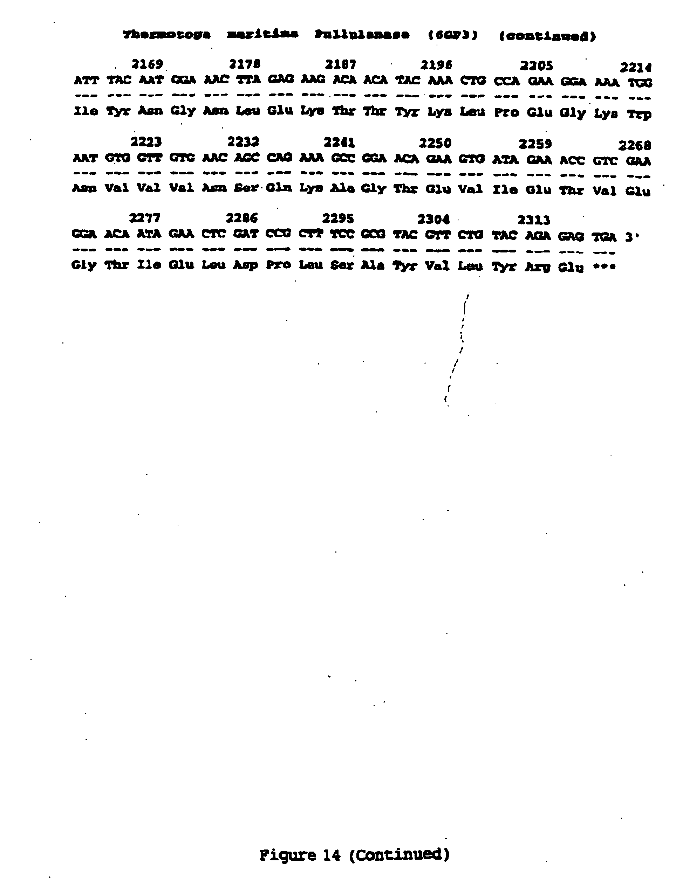

Accordingly, the polynucleotides and enzymes encoded thereby are identified by the organism from which they were isolated, and are sometimes hereinafter referred to as "M11TL" (Figure 1 and SEQ ID NOS:L and 15), "OC1/4V-33B/G" (Figure 2 and SBQ ID NOS:2 and 16), "F1-12G" (Figure 3 and SEQ ID MOS:3 and 17), "9N2-31B/G" (Figure 4 and SEQ ID NOS:4 and 18), "MSB8" (Figure 5 and SEQ ID NOS:5 and 19), "AEDII12RA-18B/G" (Figure 6 and SEQ ID 1108:6 and 20), "GC74-22G" (Figure 7 and SEQ ID NOS:7 and 21), "VC1-7G1" (Figure 8 and SEQ ID NOS:8 and 22), "37GP1" (Figure 9 and SEQ ID NOS: 9 and 23), "6GC2" (Figure 10 and SEQ ID NOS: 10 and 24), "6GP2'' (Figure 11 and SEQ ID NOS:11 and 25), "ASPII 1a" (Figure 12 and SEQ ID NOS:3.2 and 26), "OC1/4V" (Figure 13 and SBQ ID NOS:13 and 27), and "6GP3'' (Figure 14 and SEQ ID NOS:14 and 28).

The polynucleotides and polypeptides described show identity at the nucleotide and protein level to known genes and proteins encoded thereby as shown in Table 1.

**Table 1**

| Clone | Gene/Protein with Closest homology | Protein Identity | Nucleic Acid Identity |
|---|---|---|---|
| M11TL-29G | Sulfolobus Sulfataricus DSM 1616/P1, β-galactosidase | 51% | 55% |
| OC1/4V-33B/G | Caldocellum saccharolyticum, β-glucosidase | 52% | 57% |
| *Staphylothermus marinus* F1-12G | Bacillus polymyxa, β-galactosidase | 36% | 48% |
| *Thermococcus* 9N2-31B/G | Sulfolobus sulfataricus ATCC 4925S/MT4. β-galactosidase | 51% | 50% |
| *Thermotoga maritima* MSB8-6G | Clostridium thermocellum bglB | 45% | 53% |
| *Thermococcus* AEDII12RA-18B/G β-galactosidase | Bacillus polymyxa, | 34% | 48% |
| *Thermococcus chitonophagus* GC74-22G | Sulfolobus sulfataricus ATCC 49255/MT4, β-galactosidase | 46% | 54% |
| *Pyrococcus furiosus* VC1-7G1 | Sulfolobus sulfataricus/MT-4 β-galactosidase | 46.4% | 52.5% |
| *Thermotoga maritima* α-galactosidase (6GC2) | Pediococcus pentosaceaus α-galactosidase | 49% | 29% |
| *Thermotoga maritima* β-mannanase (6GP2) | Aspergillus aculeatus mannanase | 56% | 37% |
| AEPII 1a β-mannosidase (63GB1) | Sulfolobus solfactaricus β-galactosidase | 78% | 56% |
| OC1/4V endoglucanase (33BP1) | clostridium thermocellum endo-1,4-β-endoglucanase | 65% | 43% |
| *Thermotoga maritima* pullalanase (6GP3) | Caldocellum saccharolyticum α-destrom 6 glucanohydralase | 72 | 53 |
| *Bankia gouldi* mix Endoglucanase (37GP1) | None available | | |

The polynucleotide and enzyme of the present invention shows homology as shown in Table 2.

**Table 2**

| Clone | Gene/Protein with Closest Homology | Protein Identity | Nucleic Acid Identity |
|---|---|---|---|
| *Staphylothermus marinus* F1-12G | *Thermococcus* AEDII12RA-18B/G, β-galactosidase, glucosidase | 55% | 57% |
| *Thermococcus* 9N2-31B/G | *Thermococcus chitonophagus* GC74-22G-glucosidase | 74% | 66% |
| *Pyrococcus furiosus* VC1-7G1 | *Pyrococcus furiosus* VC1-7B/G β-galactosidase | 46.4% | 54% |

All the clones identified in Tables 1 and 2 encode polypeptides which have α-glycosidase or β-glycosidase activity.

This invention, in addition to the isolated nucleic acid molecule encoding the enzyme of the present invention, also provide substantially similar sequences. Isolated nucleic acid sequences are substantially similar if: (i) they are capable of hybridizing under conditions hereinafter described, to the polynucleotide of SEQ ID NO:13; (ii) or they encode DNA sequences which are degenerate to the polynucleotide of SEQ ID NO:13. Degenerate DNA sequences encode the amino acid sequence of SEQ ID NO:27, but have variations in the nucleotide coding sequences. As used herein, substantially similar refers to the sequences having similar identity to the sequences of the instant invention. The nucleotide sequences that are substantially the same can be identified by hybridization or by sequence comparison. Enzyme sequences that are substantially the same can be identified by one or more of the following: proteolytic digestion, gel electrophoresis and/or microsequencing.

One means for isolating the nucleic acid molecules encoding the enzymes of the present invention is to probe a gene library with a natural or artificially designed probe using art recognized procedures (see, for example: Current Protocols in Molecular Biology, Ausubel P.M. et al. (EDS.) Green Publishing Company Assoc. and John Wiley Interscience. New York, 1989, 1992). It is appreciated to one skilled in the art, that the polynucleotides of SEQ ID NOS:1-14 or fragments thereof (comprising at least 12 contiguous nucleotides), are particularly useful probes. Other particular useful probes for this purpose are hybridizable fragments to the sequences of SEQ ID NOS:1-14 (*i.e.*, comprising at least 12 contiguous nucleotides).

With respect to nucleic acid sequences which hybridize to specific nucleic acid sequences disclosed herein, hybridization may be carried out under conditions of reduced stringency, medium stringency or even stringent conditions. As an example of oligonucleotide hybridization, a polymer membrane containing immobilized denatured nucleic acids is first prehybridized for 30 minutes at 45°C in a solution consisting of 0.9 M NaCl, 50 mM NaH₂PO₄, pH 7.0, 5.0 mM Na₂EDTA, 0.5% SDS, 10X Denhardt's, and 0.5 mg/mL polyriboadenylic acid. Approximately 2 X 10⁷ cpm (specific activity 4-9 X 10⁸ cpm/ug) of ³²P end-labeled oligonucleotide probe are then added to the solution. After 12-16 hours of incubation, the membrane is washed for 30 minutes at room temperature in 1X SET (150 mM NaCl, 20 mM Tris hydrochloride, pH 7.8, 1 mM Na₂EDTA) containing 0.5% SDS, followed by a 30. minute wash in fresh 1X SET at Tm 10°C for the oligonucleotide probe. The membrane is then exposed to autoradiographic film for detection of hybridization signals.

Stringent conditions means hybridization will occur only if there is at least 90% identity, preferably at least 95% identity and most preferably at least 97% identity between the sequences. Further, it is understood that a section of a 100 bps sequence that is 95 bps in length has 95% identity with the 1090 bps sequence from which it is obtained. See J. Sambrook et al., Molecular Cloning, A Laboratory Manual, 2d Ed., Cold Spring Harbor Laboratory (1989). Also, it is understood that a fragment of a 100 bps sequence that is 95 bps in length has 95% identity with the 100 bps sequence from which it is obtained.

As used herein, a first DNA (RNA) sequence is at least 70% and preferably at least 80% identical to another DNA (RNA) sequence if there is at least 70% and preferably at least a 80% or 90% identity, respectively, between the bases of the first sequence and the bases of the another sequence, when properly aligned with each other, for example when aligned by BLASTN.

"Identity" as the term is used herein, refers to a polynucleotide sequence which comprises a percentage of the same bases as a reference polynucleotide (SBQ ID NO:13). For example, a polynucleotide which is at least 90% identical to a reference polynucleotide, has polynucleotide bases which are identical in 90% of the bases which make up the reference polynucleotide and may have different bases in 10% of the bases which comprise that polynucleotide sequence.

The present invention relates to polynucleotides which differ from the reference polynucleotide such that the changes are silent changes, for example the change do not alter the amino acid sequence encoded by the polynucleotide. The present invention also relates to nucleotide changes which result in amino acid substitutions, additions, deletions, fusions and truncations in the polypeptide encoded by the reference polynucleotide. In a preferred aspect of the invention these polypeptides retain the same biological action as the polypeptide encoded by the reference polynucleotide.

It is also appreciated that such probes can be and are preferably labeled with an analytically detectable reagent to facilitate identification of the probe. Useful reagents include but are not limited to radioactivity, fluorescent dyes or enzymes capable of catalyzing the formation of a detectable product. The probes are thus useful to isolate complementary copies of DNA from other sources or to screen such sources for related sequences.

The polynucleotides described herein were recovered from genomic gene libraries from the organisms listed in Table 1. For example, gene libraries can be generated in the Lambda ZAP II cloning vector (Stratagene Cloning Systems). Mass excisions can be performed on these libraries to generate libraries in the pBluescript phagemid. Libraries are thus generated and excisions performed according to the protocols/methods hereinafter described.

The excision libraries are introduced into the *E. coli* strain BW14B93 F'kanlA. Expression clones are then identified using a high temperature filter assay. Expression clones encoding several glucanases and several other glycosidases are identified and repurified. The polynucleotides, and enzymes encoded thereby, of the present invention, yield the activities as described above.

The coding sequences for the enzymes of the present invention were identified by screening the genomic DNAs prepared for the clones having glucosidase or galactosidase activity.

An example of such an assay is a high temperature filter assay wherein expression clones were identified by use of high temperature filter assays using buffer Z (see recipe below) containing 1 mg/ml of the substrate 5-bromo-4-chloro-3-indolyl-β-D-glueopyranoside (XGLU) (Diagnostic Chemicals Limited or Sigma) after introducing an excision library into the *E. coli* strain BW14893 F'kan1A**.** Expression clones encoding XGLUases were identified and repurified from MIITL, OC1/4V, Pyrococcus furiosus VC1, Staphylothemus marinus F1, Thermococcus 9N-2, Thermotoga maritima MSBS, Thermococcus alcaliphilus AEDII12RA, and Thermococcus chitonophagus GC74.

Z-buffer: (referenced in Miller, J.H. (1992) A Short Course in Bacterial Genetics, p. 445.)

| per liter: | |
|---|---|
| Na₂HPO₄-7H₂O | 16.1g |
| NaH₂PO₄-7H₂O | 5.5g |
| KCl | 0.75g |
| MgSO₄-7H₂O | 0.246g |
| β-mercaptoethanol | 2.7ml |
| Adjust pH to 7.0 | |

**High Temperature Filter Assay**
(1) The f factor f'kan (from *E. coli* strain CSH118)(1) was introduced into the pho-pnh-lac-strain BW14893(2). BW13893 (2) . The filamentous phage library was plated on the resulting strain, BW14893 F'kan. (Miller, J.H. (1992) A Short Course in Bacterial Genetics; Lee, K.S., Metcalf, et al., (1992) Evidence for two phosphonate degradative pathways in Enterobacter Aerogenes, J. Bacteriol., 174:2501-2510.
(2) After growth on 100 mm LB plates containing 100 µg/ml ampicillin, 80 µg/ml nethicillin and 1mM IPTG, colony lifts were performed using Millipore HATF membrane filters.
(3) The colonies transferred to the filters were lysed with chloroform vapor in 150 mm glass petri dishes.
(4) The filters were transferred to 100 mm glass petri dishes containing a piece of Whatman 3MM filter paper saturated with buffer.
   (a) when testing for galactosidase activity (XGALase), 3MM paper was saturated with Z buffer containing 1 mg/ml XGAL (ChemBridge Corporation). After transferring filter bearing lysed colonies to the glass petri dish, placed dish in oven at 80-85°C.
   (b) when testing for glucosidase (XGLUase), 3MM paper was saturated with Z buffer containing 1 mg/ml XGLU. After transferring filter bearing lysed colonies to the glass petri dish, placed dish in oven at 80-85°C.
(5) 'Positives' were observed as blue spots on the filter membranes. Used the following filter rescue technique to retrieve plasmid from lysed positive colony. Used pasteur pipette (or glass capillary tube) to core blue spots on the filter membrane. Placed the small filter disk in an Eppendorf tube containing 20 µl water. Incubated the Eppendorf tube at 75°C for 5 minutes followed by vortexing to elute plasmid DNA off filter. This DNA was transformed into electrocompetent *E. coli* cells DH10B for Thermatoga maritima MSDO-6G, Staphylothermus marinus F1-12G, Thermococcus AEDII12RA-18B/G, Thermococcus chitonophagus GC74-22G, M11T1 and OC1/4V. Electrocompetent BW14893 F'kanlA *E. coli* were used for Thermococcus 9N2-31B/G, and *Pyrococcus furiosus* VC1-7G1. Repeated filter-lift assay on transformation plates to identify 'positives'. Return transformation, plates to 37°C incubator after filter lift to regenerate colonies. Inoculate 3 ml LB liquid containing 100 µg/ml ampicillin with repurified positives and incubate at 37°C overnight. Isolate plasmid DNA from these cultures and sequence plasmid insert. In some instances where the plates used for the initial colony lifts contained non-confluent colonies, a specific colony corresponding to a blue spot on the filter could be identified on a regenerated plate and repurified directly, instead of using the filter rescue technique.

Another example of such an assay is a variation of the high temperature filter assay wherein colony-laden filters are heat-killed at different temperatures (for example, 105°C for 20 minutes) to monitor thermostability. The 3MM paper is saturated with different buffers (i.e., 100 mM NaCl, 5 mM MgCl₂, 100 mM Tris-Cl (pH 9.5)) to determine enzyme activity under different buffer conditions.

A β-glucosidase assay may also be employed, wherein GlcpβNp is used as an artificial substrate (aryl-β-glucosidase). The increase in absorbance at 405 nm as a result of p-nitrophenol (pNp) liberation was followed on a Hitachi U-1100 spectrophotometer, equipped with a thermostatted cuvette holder. The assays may be performed at 80°C or 90°C in closed 1-ml quartz cuvette. A standard reaction mixture contains 150 mM trisodium substrate, pH 5.0 (at 80°C), and 0.95 mM pNp derivative pNp - 0.561 mM⁻¹ • cm⁻¹). The reaction mixture is allowed to reach the desired temperature, after which the reaction is started by injecting an appropriate amount of enzyme (1.06 ml final volume).

1 U β-glucosidase activity is defined as that amount required to catalyze, the formation of 1.0 µmol pNp/min., D-cellobiose may also be used as a substrate.

An ONPG assay for β-galactosidase activity is described by Miller, J.H. (1992) A Short Course in Bacterial Genetics and Mill, J.H. (1992) Experiments in Molecular Genetics, the contents of which are hereby incorporated by reference in their entirety.

A quantitative fluorometric assay for β-galactosidase specific activity is described by : Youngman P., (1987) Plasmid Vectors for Recovering and Exploiting Tn917 Transpositions in Bacillus and other Gram-Positive Bacteria. In Plasmids: A Practical approach (ed. K. Hardy) pp 79-103. IRL Press, Oxford. A description of the procedure can be found in Miller (1992) p. 75-77, the contents of which are incorporated by reference herein in their entirety.

The polynucleotides of the present invention may be in the form of DNA which DNA includes cDNA, genomic DNA, and synthetic DNA. The DNA may be double-stranded or single-stranded, and if single stranded may be the coding strand or non-eoding (anti-sense) strand. The coding sequences which encodes the mature enzymes may be identical to the coding sequences shown in Figure 13 (SEQ ID NO:13) or may be a different coding sequence which coding sequence, as a result of the redundancy or degeneracy of the genetic code, encodes the same mature enzymes as the DNA of Figure 13 (SEQ ID NO:13).

The polynucleotide which encodes for the mature enzyme of Figure 13 (SEQ ID NO:27) may include, but is not limited to: only the coding sequence for the mature enzyme; the coding sequence for the mature enzyme and additional coding sequence such as a leader sequence or a proprotein sequence; the coding sequence for the mature enzyme (and optionally additional coding sequence) and non-coding sequence, such as introns or non-coding sequence 5' and/or 3' of the coding sequence for the mature enzyme.

Thus, the term "polynucleotide encoding an enzyme (protein) " encompasses a polynucleotide which includes only coding sequence for the enzyme as well as a polynucleotide which includes additional coding and/or non-coding sequence.

The present invention further relates to variants of the hereinabove described polynucleotides which encode for fragments, analogs and derivatives of the enzymes having the deduced amino acid sequences of Figure 13 (SEQ ID NO:27).

The variant of the polynucleotide may be a naturally occurring allelic variant of the polynucleotide or a non-naturally occurring variant of the polynucleotide.

Thus, the present invention includes polynucleotides encoding the same mature enzymes as shown in Figure 13 (SEQ ID NO:27) an well as variants of such polynucleotides which variants encode for a fragment, derivative or analog of the enzyme of Figure 13 (SEQ ID NO:27). Such nucleotide variants include deletion variants, substitution variants and addition or insertion variants.

As hereinabove indicated, the polynucleotides may have a coding sequence which is a naturally occurring allelic variant of the coding sequences shown in Figures 1-14 (SEQ ID **NOS:1-14)** . As known in the art, an allelic variant is an alternate form of a polynucleotide sequence which may have a substitution, deletion or addition of one or more nucleotides, which does not substantially alter the function of the encoded enzyme.

Fragments of the full length gene of the present invention may be used as a hybridization probe for a cDNA or a genomic library to isolate the full length DNA and to isolate other DNAs which have a high sequence similarity to the gene or similar biological activity. Probes of this type preferably have at least 10, preferably at least 15. and even more preferably at least 30 bases and may contain, for example, at least 50 or more bases. The probe may also be used to identify a DNA clone corresponding to a full length transcript and a genomic clone or clones that contain the complete gene including regulatory and promotor regions, exons, and introns. An example of a screen comprises isolating the coding region of the gene by using the known DNA sequence to synthesize an oligonucleotide probe. Labeled oligonucleotides having a sequence complementary to that of the gene of the present invention are used to screen a library of genomic DNA to determine which members, of the library the probe hybridizes to.

The present invention further relates to polynucleotides which hybridize to the hereinabove-described sequences if there is at least 70%, preferably at least 90%, and more preferably at least 95% identity between the sequences. The present invention particularly relates to polynucleotides which hybridize under stringent conditions to the hereinabove-deseribed polynucleotides. As herein used, the term "stringent conditions" means hybridization will occur only if there is at least 95% and preferably at least 97% identity between the sequences. The polynucleotides which hybridize to the hereinabove described polynucleotides in a preferred embodiment encode enzymes which either retain substantially the same biological function or activity as the mature enzyme encoded by the DNA of Figure 13 (SEQ ID NO:13).

The polynucleotide described may have at least 15 bases, at least 30 bases, and at least 50 bases which hybridize to any part of a polynucleotide of the present invention and which has an identity thereto, as hereinabove described, and which may or may not retain activity. For example, such polynucleotides may be employed as probes for the polynucleotides of SEQ ID NO:13, for example, for recovery of the polynucleotide or as a diagnostic probe or as a PCR primer.

Thus, the present invention is directed to polynucleotides having at least a 70% identity, preferably at least 90% identity and more preferably at least a 95% identity to a polynucleotide which encodes the enzymes of SEQ ID NO:27. Fragments thereof are described, which fragments have at least 15 bases, at least 30 bases and at least 50 bases, which fragments are at least 90% identical, at least 95% identical and at least 97% identical under stringent conditions to any portion of a polynucleotide of the present invention.

The present invention further relates to enzymes which have the deduced amino acid sequences of Figure 13 (SEQ ID NO:27) as well as fragments, analogs and derivatives of such enzyme.

The terms "fragment," "derivative" and "analog" when referring to the enzyme, of Figure 13 (SEQ ID NO:27) means enzymes which retain essentially the same biological function or activity as such enzyme. Thus, an analog includes a proprotein which can be activated by cleavage of the proprotein portion to produce an active mature enzyme.

The enzymes of the present, invention may be a recombinant enzyme, a natural enzyme or a synthetic enzyme, preferably a recombinant enzyme.

The fragment, derivative or analog of the enzyme of Figure 13 (SEQ ID NO:27) may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which the mature enzyme is fused with another compound, such as a compound to increase the half-life of the enzyme (for example, polyethylene glycol), or (iv) one in which the additional amino acids are fused to the mature enzyme, such as a leader or secretary sequence or a sequence which is employed for purification of the mature enzyme or a proprotein sequence. Such fragments, derivatives and analogs are deemed to be within the scope of those skilled in the art from the teachings herein.

The enzymes and polynucleotides of the present invention are preferably provided in an isolated form, and preferably are purified to homogeneity.

The term "solated" means that the material is removed from its original environment (e.g., the natural environment if it is naturally occurring). For example, a naturally-occurring polynucleotide or enzyme present in a living animal is not isolated, but the same polynucleotide or enzyme" separated from some or all of the coexisting materials in the natural system, is isolated. Such polynucleotides could be part of a vector and/or such polynucleotides or enzymes could be part of a composition, and still be isolated in that such vector or composition is not part of its natural environment.

The enzymes of the present invention include the enzyme of SEQ ID NO:27 (in particular the mature enzyme) as well as enzymes which have at least 70% similarity (preferably at least 70% identity) to the enzymes of SEQ ID NO:27 and more preferably at least 90% similarity (more preferably at least 90% identity) to the enzymes of SEQ ID NO:27 and still more preferably at least 95% similarity (still more preferably at least 95% identity) to the enzymes of SEQ ID NO:27. Also described are portions of such enzymes with such portion of the enzyme generally containing at least 30 amino acids and at least 50 amino acids.

As known in the art "similarity" between two enzymes is determined by comparing the amino acid sequence and its conserved amino acid substitutes of one enzyme to the sequence of a second enzyme.

A variant, i.e. a "fragment", "analog" or "derivative" polypeptide, and reference polypeptide may differ in amino acid sequence by one or more substitutions, additions, deletions, fusions and truncations, which may be present in any combination.

Among preferred variants are those that vary from a reference by conservative amino acid substitutions. Such substitutions are those that substitute a given amino acid in a polypeptide by another amino acid of like characteristics. Typically seen as conservative substitutions are the replacements, one for another, among the aliphatic amino acids Ala, Val, Leu and Ile; interchange of the hydroxyl residues Ser and Thr, exchange of the acidic residues Asp and Glu, substitution between the amide residues Asn and Gin, exchange of the basic residues Lye and Arg and replacements among the aromatic residues Phe, Tyr.

Most highly preferred are variants which retain the same biological function and activity as the reference polypeptide from which it varies.

Fragments or portions of the enzymes of the present invention may be employed for producing the corresponding full-length enzyme by peptide synthesis; therefore, the fragments may be employed as intermediates for producing the full-length enzymes. Fragments or portions of the polynucleotides of the present invention may be used to synthesize full-length polynucleotides of the present invention.

The present invention also relates to vectors which include polynucleotides of the present invention, host cells which are genetically engineered with vectors of the invention and the production of enzymes of the invention by recombinant techniques.

Host cells are genetically engineered (transduced or transformed or transfected) with the vectors of this invention which may be, for example, a cloning vector or an expression vector. The vector may be, for example, in the form of a plasmid, a viral particle, a phage, etc. The engineered host cells can be cultured in conventional nutrient media modified as appropriate for activating promoters, selecting transformants or amplifying the genes of the present invention. The culture conditions, such as temperature, pH and the like, are those previously used with the host cell selected for expression, and will be apparent to the ordinarily skilled artisan.

The polynucleotides of the present invention may be employed for producing enzymes by recombinant techniques. Thus, for example, the polynucleotide may be included in any one of a variety of expression vectors for expressing an enzyme. Such vectors include chromosomal, nonchromosomal and synthetic DNA sequences, e.g., derivatives of SV40; bacterial plasmids; phage DNA; baculovirus; yeast plasmids; vectors derived from combinations of plasmids and phage DNA, viral DNA such as vaccinia, adenovirus, fowl pox virus, and pseudorabies. However, any other vector may be used as long as it in replicable and viable in the hoot.

The appropriate DNA sequence may be inserted into the vector by a variety of procedures. In general, the DNA sequence is inserted into an appropriate restriction endonuclease site(s) by procedures known in the art. Such procedures and others are deemed to be within the scope of those skilled in the art.

The DNA sequence in the expression vector is operatively linked to an appropriate expression control sequence(s) (promoter) to direct mRNA synthesis. As representative examples of such promoters, there may be mentioned: LTR or SV40 promoter, the E. coli. lac or trp, the phage lambda P_{L} promoter and other promoters known to control expression of genes in prokaryotic or eukaryotic cells or their viruses. The expression vector also contains a ribosome binding site for translation initiation and a transcription terminator. The vector may also include appropriate sequences for amplifying expression.

In addition, the expression vectors preferably contain one or more selectable marker genes to provide a phenotypic trait for selection of transformed host cells such as dihydrofolate reductase or neomycin resistance for eukaryotic cell culture, or such- as tetracycline or ampicillin resistance in E. coli.

The vector containing the appropriate DNA sequence as hereinabove described, as well an an appropriate promoter or control sequence, may be employed to transform an appropriate host to permit the host to express the protein.

As representative examples of appropriate hosts, there may be mentioned: bacterial cells, such as E. coli, Streptomyces, Bacillus subtilis; fungal cells, such as yeast; insect cells such as Drosophila S2 and Spodoptera Sf9; animal cells such as CHO, COS or Bowes melanoma; adenoviruses; plant cells, etc. The selection of an appropriate host is deemed to be within the scope of those skilled in the art from the teachings herein.

More particularly, the present invention also includes recombinant constructs comprising one or more of the sequences as broadly described above. The constructs comprise a vector, such as a plasmid or viral vector, into which a sequence of the invention has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available. The following vectors are provided by way of example; Bacterial: pQE70, pQE60, pQB-9 (Qiagen), pD10, psiX174, pBluescript II KS, pNH8A, pNH16a, pNH18A, pNH46A (Stratagene); ptrc99a, pKK223-3, pKK233-3, pDR540, pRIT5 (Pharmacia); Eukaryotic: pSV2CAT, pOG44, pXTl, pSG (Stratagene) pSVK3, pBPV, pMSG, pSVL (Pharmacia) . However, any other plasmid or vector may be used as long as they are replicable and viable in the host.

Promoter regions can be selected from any desired gene using CAT (chloramphenicol transferase) vectors or other vectors with selectable markers. Two appropriate vectors are pKK232-8 and pCM7. Particular named bacterial promoters include lacI, lacZ, T3, T7, gpt, lambda P_{R}, P_{L} and trp. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art.

In a further embodiment, the present invention relates to host cells containing the above-described constructs. The host cell can be a higher eukaryotic cell, such as a mammalian cell, or a lower eukaryotic cell, such as a yeast cell, or the host cell can be a prokaryotic cell, such as a bacterial cell. Introduction of the construct into the host cell can be effected by calcium phosphate transfection, DEAE-Dextran mediated transfection, or electroporation (Davis, L., Dibner, M., Battey, I., Basic Methods in Molecular Biology, (1986)).

The constructs in host cells can be used in a conventional manner to produce the gene product encoded by the recombinant sequence. Alternatively, the enzymes of the invention can be synthetically produced by conventional peptide synthesizers.

Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook, et al., Molecular Cloning: A Laboratory Manual, Second Edition, Cold Spring Harbor, N.Y., (1989), the disclosure of which is hereby incorporated by reference.

Transcription of the DNA encoding the enzymes of the present invention by higher eukaryotes is increased by inserting an enhancer sequence into the vector. Enhancers are cis-acting elements of DNA, usually about from 10 to 300 bp that act on a promoter to increase its transcription. Examples include the SV40 enhancer on the late side of the replication origin bp 100 to 270, a cytomegalovirus early promoter enhancer, the polyoma enhancer on the late side of the replication origin, and adenovirus enhancers.

Generally, recombinant expression vectors will include origins of replication and selectable markers permitting transformation of the host cell, e.g., the ampicillin resistance gene of E. coli and S. cerevisiae TRP1 gene, and a promoter derived from a highly-expressed gene to direct transcription of a downstream structural sequence. Such promoters can be derived from operons encoding glycolytic enzymes such as 3-phosphoglycerate kinase (PGK). α-factor, acid phosphatase, or heat shock proteins, among others. The heterologous structural sequence is assembled in appropriate phase with translation initiation and termination sequences, and preferably, a leader sequence capable of directing secretion of translated enzyme. Optionally, the heterologous sequence can encode a fusion enzyme including an N-terminal identification peptide imparting desired characteristics, e.g., stabilization or simplified purification of expressed recombinant product.

Useful expression vectors for bacterial use are constructed by inserting a structural DNA sequence encoding a desired protein together with suitable translation initiation and termination signals in operable reading phase with a functional promoter. The vector will comprise one or more phenotypic selectable markers and an origin of replication to ensure maintenance of the vector and to, if desirable, provide amplification within the host. Suitable prokaryotic hosts for transformation include E. coli, Bacillus subtilis, Salmonella typhimurium and various species within the genera Pseudomonas, Streptomyces, and Staphylococcus, although others may also be employed as a matter of choice.

As a representative but nonlimiting example, useful expression vectors for bacterial use can comprise a selectable marker and bacterial origin of replication derived from commercially available plasmids comprising genetic elements of the well, known cloning vector pBR322 (ATCC 37017). Such commercial vectors include, for example, pKK223-3 (Pharmacia Fine Chemicals, Uppsala, Sweden) and GEM1 (Promega Biotec, Madison, WI, USA). These pBR322 "backbone" sections are combined with an appropriate promoter and the structural sequence to be expressed.

Following transformation of a suitable host strain and growth of the host strain to an appropriate cell density, the selected promoter is induced by appropriate means (e.g., temperature shift or chemical induction) and cells are cultured for an additional period.

Cells are typically harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents, such methods are well known to those skilled in the art.

Various mammalian cell culture systems can also be employed to express recombinant protein. Examples of mammalian expression systems include the COS-7 lines of monkey kidney fibroblasts, described by Gluzman, Cell, 23:175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice, and polyadenylation sites may be used to provide the required nontranscribed genetic elements.

The enzyme can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Protein refolding steps can be used, as necessary, in completing configuration of the mature protein. Finally, high performance liquid chromatography (HPLC) can be employed for final purification steps.

The enzymes of the present invention may be a naturally purified product, or a product of chemical synthetic procedures, or produced by recombinant techniques from a prokaryotic or eukaryotic host (for example, by bacterial, yeast, higher plant, insect and mammalian cells in culture). Depending upon the host employed in a recombinant production procedure, the enzymes of the present invention may be glycosylated or may be non-glycosylated. Enzymes of the invention may or may not also include an initial methionine amino acid residue.

β-galactosidase hydrolyzes lactose to galactose and glucose. Accordingly, the OC1/4V, 9N2-31B/G, AEDII12RA-18B/G and F1-12G enzymes may be employed in the food processing industry for the production of low lactose content milk and for the production of galactose or glucose from lactose contained in whey obtained in a large amount as a by-product in the production of cheese. Generally, it is desired that enzymes used in food processing, such as the aforementioned β-galactosidases, be stable at elevated temperatures to help prevent microbial contamination.

These enzymes may also be employed in the pharmaceutical industry. The enzymes are used to treat intolerance to lactose. In this case, a thermostable enzyme is desired, as well. Thermostable β-galactosidases also have uses in diagnostic applications, where they are employed as reporter molecules.

Glucosidases act on soluble cellooligosaccharides from the non-reducing end to give glucose as the sole product. Glucanases (endo- and exo-) act in the depolymerization of cellulose, generating more non-reducing ends (endo-glucanases, for instance, act on internal linkages yielding cellobiose, glucose and cellooligosaccharides as products). β-glucasidases are used in applications where glucose is the desired product. Accordingly, M11TL, F1-12G, GC74-22G and MSB8-6G (and OCl/4V, VC1-7GI, 9N2-31B/G and AEDII12RA18B/G) may be employed in a wide variety of industrial applications, including in corn wet milling for the separation of starch and gluten, in the fruit industry for clarification and equipment maintenance, in baking for viscosity reduction, in the textile industry for the processing of blue jeans, and in the detergent industry as an additive. For these and other applications, thermostable enzymes are desirable

Antibodies generated against the enzymes corresponding to a sequence of the present invention can be obtained by direct injection of the enzymes into an animal or by administering the enzymes to an animal, preferably a nonhuman. The antibody so obtained will then bind the enzymes itself. In this manner, even a sequence encoding only a fragment of the enzymes can be used to generate antibodies binding the whole native enzymes. Such antibodies can then be used to isolate the enzyme from cells expressing that enzyme.

For preparation of monoclonal antibodies, any technique which provides antibodies produced by continuous cell line cultures can be used. Examples include the hybridoma technique (Kohler and Milstein, 1975, Nature, 256:495-497), the trioma technique, the human B-cell hybridoma technique (Kozbor et al., 1983, Immunology Today 4:72), and the EBV-hybridoma technique to produce human monoclonal antibodies (Cole, et al., 1985, in Monoclonal Antibodies and Cancer Therapy, Alan R. Liss, Inc., pp. 77-96).

Techniques described for the production of single chain antibodies (U.S. Patent 4,946,778) can be adapted to produce single chain antibodies to immunogenic enzyme products of this invention. Also, transgenic mice may be used to express humanized antibodies to immunogenic enzyme products of this invention.

Antibodies generated against the enzyme of the present invention may be used in screening for similar enzymes from other organisms and samples. Such screening techniques are known in the art, for example, one such screening assay is described in "Methods for Measuring Cellulase Activities", Methods in enzymology, Vol 160, pp. 87-116, which is hereby incorporated by reference in its entirety.

The present invention will be further described with reference to the following examples; however, it is to be understood that the present invention is not limited to such examples. All parts or amounts, unless otherwise specified, are by weight.

In order to facilitate understanding of the following examples certain frequently occurring methods and/or terms will be described.

"Plasmids" are designated by a lower case p preceded and/or followed by capital letters and/or numbers. The starting plasmids herein are either commercially available, publicly available on an unrestricted basin, or can be constructed from available plasmids in accord with published procedures. In addition, equivalent plasmids to those described are known in the art and will be apparent to the ordinarily skilled artisan.

"Digestion" of DNA refers to catalytic cleavage of the DNA with a restriction enzyme that acts only at certain sequences in the CNA. The various restriction enzymes used herein are commercially available and their reaction conditions, cofactors and other requirements were used as would be known to the ordinarily skilled artisan. For analytical purposes, typically 1 µg of plasmid or DNA fragment is used with about 2 units of enzyme in about 20 µl of buffer solution. For the purpose of isolating DNA fragments for plasmid construction, typically 5 to 50 µg of DNA are digested with 20 to 250 units of enzyme in a larger volume. Appropriate buffers and substrate amounts for particular restriction enzymes are specified by the manufacturer. Incubation times of about 1 hour at 37°C are ordinarily used, but may vary in accordance with the supplier's instructions. After digestion the reaction is electrophoresed directly on a polyacrylamide gel to isolate the desired fragment.

Size separation of the cleaved fragments is performed using a percent polyacrylamide gel described by Goeddel, D. et al.. Nucleic Acids Res., 8:4057 (1980).

"Oligonucleotides" refers to either a single stranded polydeoxynucleotide or two complementary polydeoxynucleotide strands which may be chemically synthesized. Such synthetic oligonucleotides have no 5' phosphate and thus will not ligate to another oligonucleotide without adding a phosphate with an ATP in the presence of a kinase. A synthetic oligonucleotide will ligate to a fragment that has not been dephosphorylated.

"Ligation" refers to the process of forming phosphodiester bonds between two double stranded nucleic acid fragments (Maniatis, T., et al., Id., p. 146). Unless otherwise provided, ligation may be accomplished using known buffers and conditions with 10 units of T4 DNA ligase ("ligase") per 0.5 µg of approximately equimolar amounts of the DMA fragments to be ligated.

Unless otherwise stated, transformation was performed as described in, the method of Graham, P. and Van der Eb, A. , Virology, 52:456-457 (1973).

### Example 1

### Bacterial Expression and Purification of Glycosidase Enzymes

DNA encoding the enzymes described herein, SEQ ID NOS:1 through 14 were initially amplified from a pBluescript vector containing the DBA by the PCR technique using the primers noted herein. The amplified sequences were then inserted into the respective PQE vector listed beneath the primer sequences, and the enzyme was expressed according to the protocols set forth herein. The 5' and 3' primer sequences for the respective genes are as follows:
*Thermococcus* AEDII12RA -18B/G
   5' CCGAGAATTCATTAAAGAGGAGAAATTAACTATGGTGAATGCTATGATTGTC (SEQ ID NO:29)
   3' CGGAAGATCTTCATAGCTCCGGAAGCCCATA (SEQ ID NO:30)
   Vector: pQE12; and contains the following restriction enzyme sites 5' EcoRI and 3' Blg II.
OC1/4V-33B/G
   5' CCGAGAATTCATTTTAACTATGATAAC3AAGGTCCGATTTTCC (SEQ ID NO:31)
   3' CGGAAGATCTTTAAGATTTTAGRAATTCCTT (SEQ ID NO:32)
   Vector: pQE12; and contains the following restriction enzyme sites 5' BcoR2 'aDd 3' Bgl II.
*Thermococcus* 9N2 - 31B/G
   5' CCGAGAATTCATTAAAGAGGAGAAATTAACTATCTACCAGAAGGCTTTCTC (SSQ ID NO:33)
   3' CGGAGGTACCTCACCCAAGTCCGAACCTTCTC (SBQ ID NO:34)
   Vector: pQE30; and contains the following restriction enzyme sites 5' EcoRI and 3' KpnI.
*Staphylothermus marinus* F1 - 12G
   5' CCGAGAATTCATTAAAGAGGAGAAATTAACTATGATAAGGTTTCCTGATTAT (SBQ ID NO:35)
   3' CGGAAGATCTTTATTCGAGGTTCTTCTTTAATCC (SEQ ID NO:36)
   Vector: pQE12; and contains the following restriction enzyme sites 5' EcoRI and 3' Bgl II.
*Thermococcus chitonophagus* GC74 - 220
   5' CCGAGAATTCATTCATTAAAGAGGAGATAACTATGCTTCCAGGAGAACTTTCTC (SEQ ID NO:37)
   3' CGGAGGATCCCTACCCCTCCTCTAAGATCTC (SEQ ID NO: 38)
   Vector: pQE12; and contains the following restriction enzyme sites 5' EeoRI and 3' BamHI.
M11TL
   5' AATAATCTAGAGCATGCAATTCCCCAAAGACTTCATGATAG (SEQ ID NO: 3 9)
   3' AATAAAAGL'ITACTGGATCAGTGTAAC3ATQCT (SEQ ID NO: 4 0)
   Vector: pQE70; and contains the following restriction enzyme sites 5' SphI and 3' Hind III.
*Thermotoga maritima* MSB8 -6G
   5' CCGACAATTGATTAAAGAGGAGAAATTAACTATGGAAAGGATCGATGAAATT (SEQ ID NO:41)
   3' CGGAGGTACCTCATGGTTTGAATCTCTTCTC (SEQ ID NO: 42)
   Vector: pQE12; and contains the following restriction enzyme sites 5' EcoRI and 3' KpnI.
*Pyrococcus furiosus* VC1 - 7G1
   5' CCGACAATTGATTARAGAGGAGAALTTAACTATGTTCCCTGAAAAGTTCCTT (SEQ ID NO:43)
   3' CQGAGGTACCTCATCCCCTCAGCAATTOCTC (SEQ ID NO:44)
   Vector: pQE12; and contains the following restriction enzyme sites 5' EcoRI and 3' Kpn I.
*Bankia gouldi* endoglucanase (37GP1)
   5' AATAAGGRTCCGTTTAGCGACGCTCGC (SEQ ID NO:45)
   3' AATAAAAGCTTCCGGGTTGTACAGCGGTAATAGGC (SEQ ID NO:46)
   Vector: pQE52; and contains the following restriction enzyme sites 5' Bam HI and 3' Hind III.
*Thermotoga maritima* α-galactosidase (6GC2)
   5' TTTATTGAATTCATTAAAGGGAAATTAACTATGATCATCGTGGAAATTCGAAAG (SEQ ID NO:47)
   **3'** TCTATAAAGCTTTCATTCTCTCACCCTCTTOGTAGAAG (SEQ ID NO:48)
   Vector: pQET; and contains the following restriction enzyme sites 5' EcoRI and 3' Hind III.
*Thermotoga maritima* β-mannanase (6GP2)
   5' TTTATTCAATTGATTAAGATTAAAGGAGAAATTAACTAGGGATTGGTGGCGACGAC (SEQ ID NO:49)
   3' TTTATTAAGCTTACTTTCATATTCACATACCTCCC (SEQ ID NO:50)
   Vector: pQEt; and contains the following restriction enzyme sites 5' Hind III and 3' EcoRI.
AEPII la β-mannanaae (63GB1)
   5' TTTATTGAAATTCATTAAAGAGGAGAAATTAACTAACTATGCTACCAGAAGTTCCTATGGGC (SEQ ID NO:51)
   3' TTTATTAAGCTTCTCATAACGGCTATGATGGTTATTTC (SEQ ID NO:52)
   Vector: pQEt; and contains the following restriction enzyme sites 5' Kind III and 3' B-coRI.
*OC1*/*4V* endoglucanase (33GP1)
   5' AAAAAACAATTGAATTCATTAAAGAGGAGAAATTAACTATAGAAACACTTCAGATATGTTCTT (SEQ ID NO:53)
   3' TTTTTCGGATCCAATTCTTCATTTACTCTCTTTGCCTG (SBQ ID NO:54)
   Vector: pQEt; and contains the following restriction enzyme sites 5' BamHI and 3' EcoRI.
*Thermotoga maritima* pullalanase (6GP3)
   5' TTTTGGAATTCATTAAAGAGGAGAAATTAACTAACTATGGAACTCTGATCATAGAAGGTTAC (SEQ ID NO: 55)
   3' ATAAGAAGCTTTTCACTCTCTCTCTCTACAGAACGTACGC (SEQ ID NO:56)
   Vector: pQEt; and contains the following restriction enzyme sites 5' BcoRI and 3' Hind III.

The restriction enzyme sites indicated correspond to the restriction enzyme sites on the bacterial expression vector indicated for the respective gene (Qiagen, Inc. Chatsworth, CA). The pQE vector encodes antibiotic resistance (Amp'), a bacterial origin of replication (ori), an IPTG-regulatable promoter operator (P/O), a ribosome binding site (RBS), a 6-His tag and restriction enzyme sites.

The pQE vector was digested with the restriction enzymes indicated. The amplified sequences were ligated into the respective pQE vector and inserted in frame with the sequence encoding for the RBS. The ligation mixture was then used to transform the E. coli strain M15/pREP4 (Qiagen, Inc.) by electroporation. M15/pRBP4 contains multiple copies of the plasmid pREP4, which expresses the lad repressor and also confers kanamycin resistance (Kan'). Transformants were identified by their ability to grow on LB plates and ampicillin/kanamycin resistant colonies were selected. Plasmid DNA was isolated and confirmed by restriction analysis. Clones containing the desired constructs were grown overnight (O/N) in liquid culture in LB media supplemented with both Amp (100 ug/ml) and Kan (25 µg/ml). The O/N culture was used to inoculate a large culture at a ratio of 1:100 to 1:250. The cells were grown to an optical density 600 (O.D.⁶⁰⁰) of between 0.4 and 0.6. IPTG ("Isopropyl-B-D-thiogalacto pyranoside") was then added to a final concentration of 1 mM. IPTG induces by inactivating the lacI repressor, clearing the P/O leading to increased gene expression. Cells were grown an extra 3 to 4 hours. Cells were then harvested by centrifugation.

The primer sequences set out above may also be employed to isolate the target gene from the deposited material by hybridization techniques described above.

### Example 2

### Isolation of A Selected Clone From the Deposited genomic clones

A clone is isolated directly by screening the deposited material using the oligonucleotide primers set forth in Example 1 for the particular gene desired to be isolated. The specific oligonucleotides are synthesized using an Applied Biosystems DNA synthesizer. The oligonucleotides, are labeled with ³²P- -ATP using T4 polynucleotide kinase and purified according to a standard protocol (Maniatis et al., Molecular Cloning: A Laboratory Manual, Cold Spring Harbor Press, Cold Spring, NY, 1982). The deposited clones in the pBluescript vectors may be employed to transform bacterial hosts which are then plated on 1.5% agar plates to the density of 20,000-50,000 pfu/150 mm plate. These plates are screened using Nylon membranes according to the standard screening protocol (Stratagene, 1993). Specifically, the Nylon membrane with denatured and fixed DNA is prehybridized in 6 x SSC, 20 mM NaH₂PO₄, 0.4%SDS, 5 x Denhardt's 500 µg/ml denatured, sonicated salmon sperm DNA: and 6 x SSC, 0.1% SDS. After one hour of prehybridization, the membrane is hybridized with hybridization buffer 6xSSC, 20 mM NaH₂PO₄, 0.4%SDS, 500 ug/ml denatured, sonicated salmon sperm DNA with 1x10⁶ cpm/ml ³²P-probe overnight at 42°C. The membrane is washed at 45-50°C with-washing buffer 6 x SSC, 0.1% SDS for 20-30 minutes dried and exposed to Kodak X-ray film overnight. Positive clones are isolated and purified by secondary and tertiary screening. The purified clone is sequenced to verify its identity to the primer sequence.

Once the clone is isolated, the two oligonucleotide primers corresponding to the gene of interest are used to amplify the gene from the deposited material. A polymerase chain reaction is carried out in 25 µl of reaction mixture with 0.5 ug of the DNA of the gene of interest. The reaction mixture is 1.5-5 mM MgCl₂ , 0.01% (w/v) gelatin, 20 µM each of dATP, dCTP, DGTP, dTTP, 25 pmol of each primer and 0.25 Unit of Taq polymerase. Thirty five cycles of PCR (denaturation at 94°C for 1 min; annealing at 55°C for 1 min; elongation at 72°C for 1 min) are performed with the Perkin-Elmer Cetus automated thermal cycler. The amplified product is analyzed by agarose gel electrophoresis and the DNA band with expected molecular weight is excised and purified. The PCR product is verified to be the gene of interest by subcloning and sequencing the DNA product. The ends of the newly purified genes are nucleotide sequenced to identify full length sequences. Complete sequencing of full length genes is then performed by Exonuclease III digestion or primer walking.

### Example 3

### Screening for Galactosidase Activity

Screening procedures for α-galactosidase protein activity may be assayed for as follows:
Substrate plates were provided by a standard plating procedure. Dilute XL1-Blue MRF *E coli* host of (Stratagene Cloning Systems, La Jolla, CA) to O.D.₆₀₀ = 1.0 with NZY media. In 15 ml tubes, inoculate 200 µl diluted host cells with phage. Mix gently and incubate tubes at 37°C for 15 min. Add approximately 3.5 ml LB top agarose (0.7%) containing 1mM IPTG to each tube and pour onto all NYZ plate surface. Allow to cool and incubate at 37°C overnight. The assay plates are obtained as substrate p-Nitrophenyl α-galactosidase (Sigma) (200 mg/100 ml) (100 mM NaCl, 100 mM Potassium-Phosphate) 1% (w/v) agarose. The plaques are overlayed with nitrocellulose and incubated at 4°C for 30 minutes whereupon the nitrocellulose is removed and overlayed onto the substrate plates. The substrate plates are then incubated at 70°C for 20 minutes.

### Example 4

### Screening of Clones for Mannanase Activity

A solid phase screening assay was utilized as a primary screening method to test clones for β-mannanase activity.

A culture solution of the Y1090-*E. coli* host strain (Stratagene Cloning Systems, La Jolla, CA) was diluted to O.D.₆₀₀=1.0 with NZY media. The amplified library from *Thermotoga maritima* lambda gtl1 library was diluted in SM (phage dilution buffer) : 5 x 10⁷ pfu/µl diluted 1:1000 then 1:100 to 5 x 10² pfu/µl. Then 8 µl of phage dilution (5 x 10² pfu/µl) was plated in 200 µl host cells. They were then incubated in 15 ml tubes at 37°C for 15 minutes.

Approximately 4 ml of molten, LB top agarose (0.7%) at approximately 52°C was added to each tube and the mixture was poured onto the surface of LB agar plates. The agar plates were then incubated at 37 °C for five hours. The plates were replicated and induced with 10 mM IPTG-soaked Duralon-UV™ nylon membranes (Stratagene Cloning Systems, La Jolla, CA) overnight. The nylon membranes and plates were marked with a needle to keep their orientation and the nylon membranes were then removed and stored at 4 °C.

An Azo-galactomannan overlay was applied to the LB plates containing the lambda plaques. The overlay contains 1% agarose, 50 mM potassium-phosphate buffer pH 7, 0.4% Azocarob-galactomannan. (Megazyme, Australia). The plates were incubated at 72 °C. The Azocarob-galactomannan treated plates were observed after 4 hours then returned to incubation overnight. Putative positives were identified by clearing zones on the Azocarob-galactomannan plates. Two positive clones were observed.

The nylon membranes referred to above, which correspond to the positive clones were retrieved, oriented over the plate and the portions matching the locations of the clearing zones for positive clones wre cut out. Phage was eluted from the membrane cut-out portions by soaking the individual portions in 500 µl SM (phage dilution buffer) and 25 µl CHCl₃.

### Example 5

### Screening of Clones for Mannosidase Activity

A solid phase screening assay was utilized as a primary screening method to test clones for β-mannosidase activity.

A culture solution of the Y1090-*E. coli* host strain (Stratagene Cloning Systems, La Jolla, CA) was diluted to O.D.₆₀₀=1.0 with NZY media. The amplified library from AEPII 1a lambda gtl1 library was diluted in SM (phage dilution buffer): 5 x 10⁷ pfu/µl diluted 1:1000 then 1:100 to 5 x 10² pfu/µl. Then 8 µl of phage dilution (5 x 10² pfu/µl) was plated in 200 µl host cells. They were then incubated in 15 ml tubes at 37°C for 15 minutes.

Approximately 4 ml of molten, LB top agarose (0.7%) at approximately 52 °C was added to each tube and the mixture was poured onto the surface of LB agar plates. The agar plates were then incubated at 37 °C for five hours. The plates were replicated and induced with 10 mM IPTG-soaked Duralon-UV™ nylon membranes (Stratagene Cloning Systems, La Jolla, CA) overnight. The nylon membranes and plates were marked with a needle to keep their orientation and the nylon membranes were then removed and stored at 4 °C.

A p-nitrophenyl-β-D-manno-pyranoside overlay was applied to the LB plates containing the lambda plaques. The overlay contains 1% agarose, 50 mM potassium-phosphate buffer pH 7, 0.4% p-nitrophenyl-β-D-mano-pyranoside. (Megazyme, Australia). The plates were incubated at 72 °C**.** The p-nitrophenyl-β-D-manno-pyranoside treated plates were observed after 4 hours then returned to incubation overnight. Putative positives were identified by clearing zones on the p-nitrophenyl-β-D-manno-pyranoside plates. Two positive clones were observed.

The nylon membranes referred to above, which correspond to the positive clones were retrieved, oriented over the plate and the portions matching the locations of the clearing zones for positive clones wre cut out. Phage was eluted from the membrane cut-out portions by soaking the individual portions in 500 µl SM (phage dilution buffer), and 25 µl **CHCl₃.**

### Example 6

### Screening for Pullulanase Activity

Screening procedures for pullulanase protein activity may be assayed for as follows:
Substrate plates were provided by a standard plating procedure. Host cells are diluted to O.D.₆₀₀= 1.0 with NZY or appropriate media. In 15 ml tubes, inoculate 200 µl diluted host cells with phage. Mix gently and incubate tubes at 37 °C for 15 min. Add approximately 3.5 ml LB top agarose (0.7%) is added to each tube and the mixture is plated, allowed to cool, and incubated at 37°C for about 28 hours. Overlays of 4.5 mis of the following substrate are poured:

| 100 ml total volume | |
|---|---|
| 0.5g | Red Pullulan Red (Megazyme, Australia) |
| 1.0g | Agarose |
| 5ml | Buffer (Tris-HCL pH 7.2 @ 75°C) |
| 2ml | 5M NaCl |
| 5ml | CaCl₂ (1.00mM) |
| 85ml | dH₂O |

Plates are cooled at room temperature, and thenm incubated at 75°C for 2 hours. Positives are observed as showing substrate degradation.

### Example 7

### Screening for Endoglucanase Activity

Screening procedures for endoglucanase protein activity may be assayed for as follows:
1. The gene library is plated onto 6 LB/GelRite/0.1% CMC/NZY agar plates (-4, 800 plaque forming units/plate) in E.coli host with LB agarose as top agarose. The plates are incubated at 37°C overnight.
2. Plates are chilled at 4°C for one hour.
3. The plates are overlayed with Duralon membranes (Stratagene) at room temperature for one hour and the membranes are oriented and lifted off the plates and stored at 4°C.
4. The top agarose layer is removed and plates are incubated at 37°C for -3 hours.
**5.** The plate surface is rinsed with NaCl.
6. The plate is stained with 0.1% Congo Red for 15 minutes.
7. The plate is destained with 1M NaCl.
8. The putative positives identified on plate are isolated from the Duralon membrane (positives are identified by clearing zones around clones). The phage is eluted from the membrane by incubating in 500µl SM + 25µl CECl₃, to elute.
9. Insert DNA is subcloned into any appropriate cloning vector and subclones are reassayed for CMCase activity using the following protocol:
   i) Spin 1ml orvernight miniprep of clone at maximum speed for 3 minutes.
   ii) Decant the supernatant and use it to fill "swells" that have been made in an LB/GelRite/0.1% CMC plate.
   iii) Incubate at 37°C for 2 hours.
   iv) Stain with 0.1% Congo Red for 15 minutes.
   v) Destain with 1M NaCl for 15 minutes.
   vi) Identify positives by clearing zone around clone.

## Claims

1. A polynucleotide selected from the group consisting of:
(a) a polynucleotide comprising a nucleic acid sequence consisting of the nucleic acid sequence shown in SEQ ID NO:13;
(b) a polynucleotide which encodes an enzyme comprising an amino acid sequence shown in SEQ ID NO: 27;
(c) a polynucleotide encoding an enzyme encoded by the DNA contained in ATCC Deposit No. 97379, wherein said enzyme has glycosidase activity;
(d) a polynucleotide having at least 70%, 80%. 90%, 95% or 97% identity to a polynucleotide of (a), (b) or (c), or a polynucleotide that hybridizes under stringent conditions to SEQ ID NO:13. which encodes a polypeptide having glycosidase activity;
(e) a polynucleotide of (c) or (d), wherein the glycosidase activity is more specifically β-glucosidase, endoglucanase, α-galactosidase, β-mannanase or a β-mannosidase activity; and
(f) a polynucleotide which is complementary to a polynucleotide of (a), (b), (c), (d) or (e).

2. The polynucleotide of claim 1, wherein the polynucleotide is DNA or RNA.

3. The polynucleotide of claim 1, wherein the polynucleotide is thermostable.

4. The polynucleotide of claim 1, wherein the polynucleotide is stable under alkaline pH.

5. A vector comprising the polynucleotide of claim 1 or 2.

6. A host cell comprising the vector of claim 5.

7. A process for producing a polypeptide comprising expressing from the host cell of claim 6 a polypeptide encoded by said polynucleotide of claim 1, part (a), (b), (c) and (d).

8. A process for producing a cell comprising transforming or transfecting the cell with the vector of claim 5 such that the cell expresses the polypeptide encoded by the polynucleotide contained in the vector.

9. An enzyme having a glycosidase activity comprising a member selected from the group consisting of:
(a) an enzyme encoded by the polynucleotide of claim 1, parts (a) to (d);
(b) an enzyme comprising an amino acid sequence which is at least 70%, 90% or 95% identical to the amino acid sequence set forth in SEQ ID NO: 27, or an enzyme comprising an amino acid sequence that varies by at least one conservative substitution of the amino acid sequence set forth in SEQ ID NO: 27; and
(c) an enzyme obtainable by the process of claim 7.

10. The enzyme of claim 9, wherein the glycosidase activity is more specifically β-glucosidase, endoglucanase, α-galactosidase, β-mannanase or a β-mannosidase activity.

11. The enzyme of claim 9, wherein the enzyme is thermostable.

12. The enzyme of claim 9, wherein the enzyme is stable under alkaline pH.

13. A pharmaceutical composition comprising the polynucleotide of claim 1 and/or the enzyme of claim 9 and optionally a pharmaceutical acceptable carrier and/or diluent.

14. A diagnostic composition comprising the polynucleotide of claim 1 and/or the enzyme of claim 9.

15. A drilling or well stimulation composition comprising the polynucleotide of claim 1 and/or the enzyme of claim 9.

16. A method for generating glucose from soluble cellooligosaccharides comprising administering an effective amount of an enzyme having the amino acid sequence set forth in SEQ ID NO: 27 or as set forth in claim 9.

17. Use of the polynucleotide of claim 1 and/or the enzyme of claim 9 in corn wet milling for the separation of starch and gluten, in the fruit industry for clarification and equipment maintenance, in the beet sugar industry, in baking for viscosity reduction, in converting plant biomass into fuels and chemicals, in fracturing during oil or gas recovery, in waste treatment, in animal feed, in the textile industry for the processing of blue jeans, in drilling and well stimulation and/or in the detergent industry as an additive.

18. Use of the polynucleotide of claim 1 and/or the enzyme of claim 9 in the preparation of a composition for the hydrolysis of cellooligosaccharides to glucose.

19. Use of the polynucleotide of claim 1 and/or the enzyme of claim 9 in depolymerization of cellulose.

20. Use of the polynucleotide of claim 1 and/or the enzyme of claim 9 in hydrolysis of a polysaccharide or oligosaccharide.

21. The use of claim 20, wherein the polysaccharide or the oligosaccharide comprises a raffinose, a stachyose or a verbascose.

22. Use of the polynucleotide of claim 1 and/or the enzyme of claim 9 in catalysis of the hydrolysis of mannan or glucan.

23. Use of the polynucleotide of claim 1 and/or the enzyme of claim 9 in the preparation of a composition for the hydrolysis of lactose.

24. Use of the polynucleotide of claim 1 and/or the enzyme of claim 9 in the preparation of a medicament.

25. The use according to claim 24, wherein said medicament is for the treatment of lactose intolerance.

26. The use according to claim 24, wherein said medicament is for the treatment of excessive gas.

27. Use of a therapeutically effective amount of the polynucleotide of claim 1 and/or the enzyme of claim 9 in the preparation of a medicament for the treatment of lactose intolerance in a patient in need of such therapy.

28. A method for making low lactose content milk comprising contacting a composition comprising a milk with an enzyme as set forth in claim 9, thereby making a low lactose content milk.

29. Use of the polynucleotide of claim 1 and/or the enzyme of claim 9 as a reporter molecule.

30. A method for hydrolyzing a guar gum comprising contacting a composition comprising a guar gum with an enzyme as set forth in claim 9, thereby hydrolyzing the guar gum.

31. A method for hydrolyzing starch comprising contacting a composition comprising a starch with an enzyme as set forth in claim 9.

32. A method for making glucose or maltose from a starch comprising contacting a composition comprising a starch with an enzyme as set forth in claim 9 under conditions wherein the enzyme hydrolyzes the starch.

33. A method for breaking down raffinose, stachyose and/or verbascose comprising contacting a composition comprising a raffinose, a stachyose and/or a verbascose with an enzyme as set forth in claim 9, thereby breaking down the raffinose, stachyose and/or verbascose.

34. method for separating starch and gluten in a corn wet milling process comprising contacting a composition comprising corn with the enzyme of claim 9, wherein the enzyme separates starch and gluten.

35. A method for separating starch and gluten comprising contacting a composition comprising a starch and a gluten with an enzyme as set forth in claim 9 under conditions wherein the enzyme can hydrolyze a glycosidic linkage.

36. A method for generating a glucose, a cellobiose or a cellooligosaccharide comprising contacting a composition comprising cellulose with the enzyme of claim 9, wherein the enzyme generates a glucose, a cellobiose or a cellooligosaccharide.

37. A method for making a sweetener comprising contacting a composition comprising a starch with an enzyme as set forth in claim 9 under conditions wherein the enzyme hydrolyzes the starch.

38. A method for treating a textile comprising contacting the textile with an enzyme as set forth in claim 9 under conditions wherein the enzyme can hydrolyze a glycosidic linkage.

39. A method for processing blue jeans comprising contacting a composition comprising a textile comprising blue jeans with the enzyme as set forth in claim 9, thereby effecting processing blue jeans.

40. A method for treating an animal feed comprising contacting the animal feed with an enzyme as set forth in claim 9 under conditions wherein the enzyme can hydrolyze a glycosidic linkage.

41. A method for making a fuel comprising contacting a plant biomass with an enzyme as set forth in claim 9 under conditions wherein the enzyme can hydrolyze a glycosidic linkage.

42. A method for conversion of plant biomass into a fuel or a chemical comprising contacting a composition comprising a plant biomass with the enzyme of claim 9, thereby converting the plant biomass into a fuel or a chemical.

43. A method for waste treatment comprising contacting a composition comprising a waste product with an enzyme as set forth in claim 9, thereby treating the waste product.

44. A method for treating a baked product comprising contacting the baked product with an enzyme as set forth in claim 9 under conditions wherein the enzyme can hydrolyze a glycosidic linkage.

45. A method for viscosity reduction in a baking process comprising contacting a composition comprising a baking product with an enzyme as set forth in claim 9, thereby effecting viscosity reduction in the baking process.

46. A method for drilling or well stimulation comprising administering the enzyme as set forth in claim 9 during the drilling or well operation, thereby stimulating the drilling or well operation.

47. A detergent comprising an enzyme as set forth in claim 9.

48. A textile comprising an enzyme as set forth in claim 9.

49. A feed comprising an enzyme as set forth in claim 9.

50. A plant biomass Comprising an enzyme as set forth in claim 9.

51. A baked product comprising an enzyme as set forth in claim 9.

52. A composition comprising a whey and an enzyme as set forth in claim 9.

53. A composition comprising a milk or a cheese and an enzyme as set forth in claim 9.

54. A composition comprising a fruit and an enzyme as set forth in claim 9.

55. A composition comprising a fabric and an enzyme as set forth in claim 9.

56. A composition comprising a starch and an enzyme as set forth in claim 9.

57. A composition comprising a juice or a beer and an enzyme as set forth in claim 9.

58. A plant biomass conversion composition comprising an enzyme as set forth in claim 9.

## Patentansprüche

1. Polynucleotid ausgewählt aus der Gruppe bestehend aus:
a) einem Polynucleotid, das eine Nucleinsäure umfasst, die aus der in SEQ ID NO: 13 gezeigten Nucleinsäuresequenz besteht;
b) einem Polynucleotid, das ein Enzym codiert, das eine in SEQ ID NO: 27 gezeigte Aminosäuresequenz umfasst;
c) einem Polynucleotid, das ein Enzym codiert, das durch die in ATCC-Hinterlegungsnummer 97379 enthaltene DNA codiert wird, wobei das Enzym Glycosidaseaktivität aufweist;
d) einem Polynucleotid mit mindestens 70 %, 80 %, 90 %, 95 % oder 97 % Identität zu einem Polynucleotid von a), b) oder c), oder einem Polynucleotid, das unter stringenten Bedingungen mit SEQ ID NO: 13 hybridisiert, welches ein Polypeptid mit Glycosidaseaktivität codiert;
e) einem Polynucleotid von c) oder d), wobei die Glycosidaseaktivität genauer β-Glucosidase-, Endoglucanase-, a-Galactosidase-, β-Mannanase- oder β-Mannosidaseaktivität ist; und
f) einem Polynucleotid, das zu einem Polynucleotid von a), b), c), d) oder e) komplementär ist.

2. Polynucleotid nach Anspruch 1, wobei das Polynucleotid DNA oder RNA ist.

3. Polynucleotid nach Anspruch 1, wobei das Polynucleotid hitzestabil ist.

4. Polynucleotid nach Anspruch 1, wobei das Polynucleotid bei einem alkalischen pH-Wert stabil ist.

5. Vektor, der das Polynucleotid nach Anspruch 1 oder 2 umfasst.

6. Wirtszelle, die den Vektor nach Anspruch 5 umfasst.

7. Verfahren zur Herstellung eines Polypeptids, umfassend das Exprimieren eines Polypeptids, das durch das Polynucleotid nach Anspruch 1, Teil a), b), c) und d) codiert wird, in der Wirtszelle nach Anspruch 6.

8. Verfahren zur Herstellung einer Zelle, welches das Transformieren oder Transfizieren der Zelle mit dem Vektor nach Anspruch 5 umfasst, so dass die Zelle das Polypeptid exprimiert, das durch das im Vektor enthaltene Polynucleotid codiert wird.

9. Enzym mit Glycosidaseactivität, umfassend ein Mitglied ausgewählt aus der Gruppe bestehend aus:
a) einem Enzym, das durch das Polynucleotid nach Anspruch 1, Teile a) bis d) codiert wird;
b) einem Enzym, umfassend eine Aminosäuresequenz, die mindestens 70%, 90% oder 95% Identität zu der in SEQ ID NO: 27 angegebenen Aminosäuresequenz hat, oder einem Enzym, das eine Aminosäuresequenz umfasst, welche sich durch mindestens eine konservative Substitution von der in SEQ ID NO: 27 gezeigten Aminosäuresequenz unterscheidet;
c) einem Enzym, das durch das Verfahren nach Anspruch 7 erhältlich ist.

10. Enzym nach Anspruch 9, wobei die Glycosidaseaktivität genauer β-Glucosidase-, Endoglucanase-, α-Galactosidase-, β-Mannanase- oder β-Mannosidaseaktivität ist.

11. Enzym nach Anspruch 9, wobei das Enzym hitzestabil ist.

12. Enzym nach Anspruch 9, wobei das Enzym bei einem alkalischen pH-Wert stabil ist.

13. Arzneimittel, umfassend das Polynucleotid nach Anspruch 1 und/oder das Enzym nach Anspruch 9 und gegebenenfalls einen pharmazeutisch verträglichen Träger und/oder Verdünnungsmittel.

14. Diagnostische Zusammensetzung, umfassend das Polynucleotid nach Anspruch 1 und/oder das Enzym nach Anspruch 9.

15. Zusammensetzung zur Förderung bei Bohr- oder Schachtarbeiten, umfassend das Polynucleotid nach Anspruch 1 und/oder das Enzym nach Anspruch 9.

16. Verfahren zur Erzeugung von Glucose aus löslichen Cellooligosacchariden, umfassend das Verabreichen einer wirksamen Menge eines Enzyms mit der Aminosäuresequenz wie in SEQ ID NO: 27 angegeben oder wie in Anspruch 9 beschrieben.

17. Verwendung des Polynucleotids nach Anspruch 1 und/oder des Enzyms nach Anspruch 9 beim Nassmahlen von Getreide zur Trennung von Stärke und Gluten, in der Früchteindustrie zur Klärung und Wartung der Geräte, in der Rübenzuckerindustrie, beim Backen zur Reduzierung der Viskosität, bei der Umwandlung pflanzlicher Biomasse in Brennstoffe und Chemikalien, beim Fraktionieren während der Öl- oder Gasgewinnung, bei der Abfallbehandlung, in Futtermittel, in der Textilindustrie zur Verarbeitung von Bluejeans, zur Förderung bei Bohr- und Schachtarbeiten, und/oder in der Detergensindustrie als ein Additiv.

18. Verwendung des Polynucleotids nach Anspruch 1 und/oder des Enzyms nach Anspruch 9 für die Herstellung einer Zusammensetzung zur Hydrolyse von Cellooligosacchariden zu Glucose.

19. Verwendung des Polynucleotids nach Anspruch 1 und/oder des Enzyms nach Anspruch 9 bei der Depolymerisation von Cellulose.

20. Verwendung des Polynucleotids nach Anspruch 1 und/oder des Enzyms nach Anspruch 9 zur Herstellung einer Zusammensetzung für die Hydrolyse eines Polysaccharids oder Oligosaccharids.

21. Verwendung nach Anspruch 20, wobei das Polysaccharid oder Oligosaccharid eine Raffinose, Stachyose oder Verbascose umfasst.

22. Verwendung des Polynucleotids nach Anspruch 1 und/oder des Enzyms nach Anspruch 9 in der Katalyse der Hydrolyse von Mannan oder Glucan.

23. Verwendung des Polynucleotids nach Anspruch 1 und/oder des Enzyms nach Anspruch 9 zur Herstellung einer Zusammensetzung für die Hydrolyse von Lactose.

24. Verwendung des Polynucleotids nach Anspruch 1 und/oder des Enzyms nach Anspruch 9 für die Herstellung eines Arzneimittels.

25. Verwendung nach Anspruch 24, wobei das Arzneimittel zur Behandlung von Lactose-Intoleranz ist.

26. Verwendung nach Anspruch 24, wobei das Arzneimittel zur Behandlung von überschüssigem Gas ist.

27. Verwendung einer therapeutisch wirksamen Menge des Polynucleotids nach Anspruch 1 und/oder des Enzyms nach Anspruch 9 für die Herstellung eines Arzneimittels zur Behandlung von Lactose-Intoleranz bei einem Patienten, der eine solche Therapie benötigt.

28. Verfahren zur Herstellung einer lactosearmen Milch, umfassend das Inkontaktbringen einer Milch umfassenden Zusammensetzung mit einem Enzym nach Anspruch 9, wodurch eine lactosearme Milch hergestellt wird.

29. Verwendung des Polynucleotids nach Anspruch 1 und/oder des Enzyms nach Anspruch 9 als Reportermolekül.

30. Verfahren zur Hydrolyse von Guargummi, umfassend das Inkontaktbringen einer Guargummi umfassenden Zusammensetzung mit einem Enzym nach Anspruch 9, wodurch das Guargummi hydrolysiert wird.

31. Verfahren zum Hydrolysieren von Stärke, umfassend das Inkontaktbringen einer Stärke umfassenden Zusammensetzung mit einem Enzym nach Anspruch 9.

32. Verfahren zur Herstellung von Glucose oder Maltose aus Stärke, umfassend das Inkontaktbringen einer Stärke enthaltenden Zusammensetzung mit einem Enzym nach Anspruch 9 unter Bedingungen, bei denen das Enzym die Stärke hydrolysiert.

33. Verfahren zum Abbau von Raffinose, Stachyose und/oder Verbascose, umfassend das Inkontaktbringen einer Raffinose, Stachyose und/oder Verbascose enthaltenden Zusammensetzung mit einem Enzym nach Anspruch 9 unter Bedingungen, bei denen die Raffinose, Stachyose und/oder Verbascose abgebaut wird.

34. Verfahren zur Trennung von Stärke und Gluten bei einem Nassmahlverfahren von Getreide, umfassend das Inkontaktbringen einer Getreide enthaltenden Zusammensetzung mit dem Enzym nach Anspruch 9, wobei das Enzym Stärke und Gluten trennt.

35. Verfahren zur Trennung von Stärke und Gluten, umfassend das Inkontaktbringen einer Stärke und Gluten enthaltenden Zusammensetzung mit einem Enzym nach Anspruch 9 unter Bedingungen, bei denen das Enzym eine glycosidische Bindung hydrolysieren kann.

36. Verfahren zur Erzeugung einer Glucose, einer Cellobiose oder eines Cellooligosaccharids, umfassend das Inkontaktbringen einer Cellulose enthaltenden Zusammensetzung mit dem Enzym nach Anspruch 9, wobei das Enzym eine Glucose, eine Cellobiose oder ein Cellooligosaccharid erzeugt.

37. Verfahren zur Herstellung eines Süßstoffs, umfassend das Inkontaktbringen einer Stärke enthaltenden Zusammensetzung mit einem Enzym nach Anspruch 9 unter Bedingungen, bei denen das Enzym die Stärke hydrolysiert.

38. Verfahren zur Behandlung einer Textilie, umfassend das Inkontaktbringen der Textilie mit einem Enzym nach Anspruch 9 unter Bedingungen, bei denen das Enzym eine glycosidische Bindung hydrolysieren kann.

39. Verfahren zur Verarbeitung von Bluejeans, umfassend das Inkontaktbringen einer Zusammensetzung, die eine Bluejeans umfassende Textilie enthält, mit dem Enzym nach Anspruch 9, wodurch die Verarbeitung von Bluejeans bewirkt wird.

40. Verfahren zur Behandlung von Tierfutter, umfassend das Inkontaktbringen des Tierfutters mit einem Enzym nach Anspruch 9 unter Bedingungen, bei denen das Enzym eine glycosidische Bindung hydrolysieren kann.

41. Verfahren zur Herstellung eines Brennstoffs, umfassend das Inkontaktbringen einer Pflanzenbiomasse mit einem Enzym nach Anspruch 9 unter Bedingungen, bei denen das Enzym eine glycosidische Bindung hydrolysieren kann.

42. Verfahren zur Umwandlung von Pflanzenbiomasse in einen Brennstoff oder eine Chemikalie, umfassend das Inkontaktbringen einer eine Pflanzenbiomasse umfassenden Zusammensetzung mit dem Enzym nach Anspruch 9, wodurch die Pflanzenbiomasse in einen Brennstoff oder eine Chemikalie umgewandelt wird.

43. Verfahren zur Behandlung von Abfall, umfassend das Inkontaktbringen einer ein Abfallprodukt umfassenden Zusammensetzung mit einem Enzym nach Anspruch 9, wodurch das Abfallprodukt behandelt wird.

44. Verfahren zur Behandlung eines Backerzeugnisses, umfassend das Inkontaktbringen des Backerzeugnisses mit einem Enzym nach Anspruch 9 unter Bedingungen, bei denen das Enzym eine glycosidische Bindung hydrolysieren kann.

45. Verfahren zur Reduzierung der Viskosität in einem Backverfahren, umfassend das Inkontaktbringen einer ein Backerzeugnis enthaltenden Zusammensetzung mit einem Enzym nach Anspruch 9, wodurch die Reduzierung der Viskosität im Backverfahren bewirkt wird.

46. Verfahren zur Förderung bei Bohr- oder Schachtarbeiten, umfassend das Zuführen des Enzyms nach Anspruch 9 während der Bohr- oder Schachtarbeiten, wodurch die Bohr- oder Schachtarbeiten gefördert werden.

47. Detergens, umfassend ein Enzym nach Anspruch 9.

48. Textilie, umfassend ein Enzym nach Anspruch 9.

49. Futtermittel, umfassend ein Enzym nach Anspruch 9.

50. Pflanzenbiomasse, umfassend ein Enzym nach Anspruch 9.

51. Backerzeugnis, umfassend ein Enzym nach Anspruch 9.

52. Zusammensetzung, umfassend Molke und ein Enzym nach Anspruch 9.

53. Zusammensetzung, umfassend eine Milch oder einen Käse und ein Enzym nach Anspruch 9.

54. Zusammensetzung, umfassend eine Frucht und ein Enzym nach Anspruch 9.

55. Zusammensetzung, umfassend ein Gewebe und ein Enzym nach Anspruch 9.

56. Zusammensetzung, umfassend eine Stärke und ein Enzym nach Anspruch 9.

57. Zusammensetzung, umfassend einen Saft oder ein Bier und ein Enzym nach Anspruch 9.

58. Zusammensetzung zur Umwandlung von Pflanzenbiomasse, umfassend ein Enzym nach Anspruch 9.

## Revendications

1. Polynucléotide choisi dans le groupe constitué de :
(a) un polynucléotide comprenant une séquence d'acide nucléique consistant en la séquence d'acide nucléique présentée dans SEQ ID NO : 13 ;
(b) un polynucléotide qui code une enzyme comprenant une séquence d'acide aminé présentée dans SEQ ID NO : 27 ;
(c) un polynucléotide qui code une enzyme codée par l'ADN contenu dans le dépôt ATCC No. 97379, ladite enzyme ayant une activité glucosidase ;
(d) un polynucléotide ayant au moins 70%, 80%, 90%, 95% ou 97% d'identité avec un polynucléotide selon (a), (b) ou (c), ou un polynucléotide qui hybride dans des conditions stringentes à SEQ ID NO : 13, qui code un polypeptide ayant une activité glycosidase ;
(e) un polynucléotide selon (c) ou (d), dans lequel l'activité glucosidase est plus spécifiquement une activité β-glucosidase, endoglucanase, α-galactosidase, β-mannanase ou β-mannosidase ; et
(f) un polynucléotide complémentaire d'un polynucléotide selon (a), (b), (c) (d) ou (e).

2. Polynucléotide selon la revendication 1, ledit polynucléotide étant un ADN ou un ARN.

3. Polynucléotide selon la revendication 1, dans lequel le polynucléotide est thermostable.

4. Polynucléotide selon la revendication 1, dans lequel le polynucléotide est stable à pH alcalin.

5. Vecteur comprenant le polynucléotide selon la revendication 1 ou 2.

6. Cellule hôte comprenant le vecteur selon la revendication 5.

7. Procédé pour produire un polypeptide, comprenant l'expression à partir de la cellule hôte selon la revendication 6 d'un polypeptide codé par ledit polynucléotide selon la revendication 1, partie (a), (b), (c) et (d).

8. Procédé pour produire une cellule, comprenant la transformation ou la transfection de la cellule avec le vecteur selon la revendication 5 de telle sorte que la cellule exprime le polypeptide codé par le polynucléotide contenu dans le vecteur.

9. Enzyme ayant une activité glycosidase, comprenant un membre choisi dans le groupe constitué de :
(a) une enzyme codée par le polynucléotide de la revendication 1, parties (a) à (d) ;
(b) une enzyme comprenant une séquence d'acide aminé qui est au moins 70%, 90% or 95% identique à la séquence d'acides aminés présentée dans SEQ ID NO : 27, ou une enzyme comprenant une séquence d'acides aminés qui varie par au moins une substitution conservatrice de la séquence d'acides aminés présentée dans SEQ ID NO : 27 ; et
(c) une enzyme susceptible d'être obtenue par le procédé de la revendication 7.

10. Enzyme selon le revendication 9, dans laquelle l'activité glycosidase est plus spécifiquement une activité β-glucosidase, endoglucanase, α-galactosidase, β-mannanase ou β-mannosidase.

11. Enzyme selon la revendication 9, dans laquelle l'enzyme est thermostable.

12. Enzyme selon la revendication 9, dans laquelle l'enzyme est stable à pH alcalin.

13. Composition pharmaceutique comprenant le polynucléotide selon la revendication 1 et/ou l'enzyme selon la revendication 9 et, éventuellement, un support et/ou diluant acceptable sur le plan pharmaceutique.

14. Composition diagnostique comprenant le polynucléotide selon la revendication 1 et/ou l'enzyme selon la revendication 9.

15. Composition de stimulation de forage et de puits comprenant le polynucléotide selon la revendication 1 et/ou l'enzyme selon la revendication 9.

16. Méthode pour générer du glucose à partir de cellooligosaccharides solubles comprenant l'administration d'une quantité efficace d'une enzyme ayant la séquence d'acides aminés présentée dans SEQ ID NO : 27 ou telle que présentée dans la revendication 9.

17. Utilisation du polynucléotide selon la revendication 1 et/ou de l'enzyme selon la revendication 9 dans le concassage à mouture humide du grain pour la séparation de l'amidon et du gluten, dans l'industrie du fruit pour la clarification et pour la maintenance des équipements, dans l'industrie du sucre de betterave, en cuisson pour réduire la viscosité, dans la conversion de la biomasse végétale en carburants et en produits chimiques, dans la fracturation lors de la récupération du pétrole ou du gaz, dans le traitement des déchets, dans l'alimentation animale, dans l'industrie textile pour le traitement de blue jeans, dans la stimulation de forage et de puits et/ou dans l'industrie des détergents en tant qu'additif.

18. Utilisation du polynucléotide selon la revendication 1 et/ou de l'enzyme selon la revendication 9 dans la préparation d'une composition pour l'hydrolyse des cellooligosaccharides en glucose.

19. Utilisation du polynucléotide selon la revendication 1 et/ou de l'enzyme selon la revendication 9 dans la dépolymérisation de la cellulose.

20. Utilisation du polynucléotide selon la revendication 1 et/ou de l'enzyme selon la revendication 9 dans l'hydrolyse d'un polysaccharide ou d'un oligosaccharide.

21. Utilisation selon la revendication 20, dans laquelle le polysaccharide ou l'oligosaccharide comprend une raffinose, une stachyose ou une verbascose.

22. Utilisation du polynucléotide selon la revendication 1 et/ou de l'enzyme selon la revendication 9 dans la catalyse de l'hydrolyse du mannane ou du glucane.

23. Utilisation du polynucléotide selon la revendication 1 et/ou de l'enzyme selon la revendication 9 dans la préparation d'une composition pour l'hydrolyse du lactose.

24. Utilisation du polynucléotide selon la revendication 1 et/ou de l'enzyme selon la revendication 9 dans la préparation d'un médicament.

25. Utilisation selon la revendication 24, dans laquelle ledit médicament sert au traitement de l'intolérance au lactose.

26. Utilisation selon la revendication 24, dans laquelle ledit médicament sert au traitement des flatulences excessives.

27. Utilisation d'une quantité efficace sur le plan thérapeutique du polynucléotide selon la revendication 1 et/ou de l'enzyme selon la revendication 9 dans la préparation d'un médicament pour le traitement de l'intolérance au lactose chez un patient ayant besoin de ce type de traitement.

28. Procédé de fabrication d'un lait à faible teneur en lactose, comprenant une étape consistant à mettre en contact une composition comprenant un lait avec une enzyme selon la revendication 9, produisant ainsi un lait à faible teneur en lactose.

29. Utilisation du polynucléotide selon la revendication 1 et/ou de l'enzyme selon la revendication 9 en tant que molécule d'exploration (ou molécule « reporter »).

30. Procédé d'hydrolyse d'une gomme de guar comprenant une étape consistant à mettre en contact une composition contenant une gomme de guar avec une enzyme selon la revendication 9, hydrolysant ainsi la gomme de guar.

31. Procédé pour hydrolyser l'amidon, comprenant la mise en contact d'une composition comprenant un amidon avec une enzyme telle que décrite dans la revendication 9.

32. Procédé pour fabriquer du glucose ou du maltose à partir d'un amidon, comprenant la mise en contact d'une composition comprenant un amidon avec une enzyme telle que décrite dans la revendication 9, dans des conditions dans lesquelles l'enzyme hydrolyse l'amidon.

33. Procédé de fractionnement de la raffinose, de la stachyose et/ou de la verbascose comprenant une étape consistant à mettre en contact une composition comprenant une raffinose, une stachyose et/ou une verbascose avec une enzyme selon la revendication 9, fractionnant ainsi la raffinose, la stachyose et/ou la verbascose.

34. Procédé de séparation de l'amidon et du gluten dans un procédé de concassage à mouture humide du grain comprenant une étape consistant à mettre en contact une composition comprenant du grain avec une enzyme selon la revendication 9, dans lequel l'enzyme sépare l'amidon et le gluten.

35. Procédé de séparation de l'amidon et du gluten comprenant une étape consistant à mettre en contact une composition comprenant un amidon et un gluten avec une enzyme selon la revendication 9, dans des conditions dans lesquelles l'enzyme peut hydrolyser une liaison glycosidique.

36. Procédé de génération d'un glucose, d'une cellobiose ou d'un cellooligosaccharide comprenant une étape consistant à mettre en contact une composition comprenant de la cellulose avec l'enzyme selon la revendication 9, dans lequel l'enzyme génère un glucose, une cellobiose ou un cellooligosaccharide.

37. Procédé pour fabriquer un édulcorant, comprenant la mise en contact d'une composition comprenant un amidon avec une enzyme telle que décrite dans la revendication 9, dans des conditions dans lesquelles l'enzyme hydrolyse l'amidon.

38. Procédé pour traiter un textile comprenant la mise en contact du textile avec une enzyme telle que décrite dans la revendication 9 dans des conditions dans lesquelles l'enzyme peut hydrolyser une liaison glycosidique.

39. Procédé pour traiter un blue jean comprenant une étape consistant à mettre en contact une composition comprenant un textile comprenant un blue jean avec l'enzyme selon la revendication 9, effectuant ainsi le traitement du blue jean.

40. Procédé pour traiter un aliment pour animaux comprenant la mise en contact de l'aliment pour animaux avec une enzyme telle que décrite dans la revendication 9 dans des conditions dans lesquelles l'enzyme peut hydrolyser une liaison glycosidique.

41. Procédé pour faire un carburant comprenant la mise en contact d'une biomasse de plante avec une enzyme telle que décrite dans la revendication 9 dans des conditions dans lesquelles l'enzyme peut hydrolyser une liaison glycosidique.

42. Procédé de conversion de la biomasse végétale en carburant ou en produit chimique, comprenant l'étape consistant à mettre en contact une composition comprenant une biomasse végétale avec l'enzyme selon la revendication 9, convertissant ainsi la biomasse végétale en carburant ou en produit chimique.

43. Procédé de traitement des déchets comprenant l'étape consistant à mettre en contact une composition comprenant un produit résiduel avec l'enzyme selon la revendication 9, traitant ainsi le produit résiduel.

44. Procédé pour traiter un produit de boulangerie ou pâtisserie, comprenant la mise en contact du produit de boulangerie ou pâtisserie avec une enzyme telle que décrite dans la revendication 9 dans des conditions dans lesquelles l'enzyme peut hydrolyser une liaison glycosidique.

45. Procédé de réduction de la viscosité dans un procédé de cuisson comprenant l'étape consistant à mettre en contact une composition comprenant un produit de boulangerie ou de pâtisserie avec l'enzyme selon la revendication 9, effectuant ainsi la réduction de la viscosité dans le processus de cuisson.

46. Procédé de stimulation de forage ou de puits, comprenant l'étape consistant à administrer l'enzyme selon la revendication 9 lors de l'opération du trou de forage ou du puits, stimulant ainsi l'opération du trou de forage ou du puits.

47. Détergent comprenant une enzyme telle que décrite dans la revendication 9.

48. Textile comprenant une enzyme telle que décrite dans la revendication 9.

49. Aliment comprenant une enzyme telle que décrite dans la revendication 9.

50. Biomasse végétale comprenant une enzyme telle que décrite dans la revendication 9.

51. Produit de boulangerie ou pâtisserie comprenant une enzyme telle que décrite dans la revendication 9.

52. Composition comprenant un lactosérum et une enzyme selon la revendication 9.

53. Composition comprenant un lait ou un fromage et une enzyme selon la revendication 9.

54. Composition comprenant un fruit et une enzyme selon la revendication 9.

55. Composition comprenant un tissu et une enzyme selon la revendication 9.

56. Composition comprenant un amidon et une enzyme selon la revendication 9.

57. Composition comprenant un jus ou une bière et une enzyme selon la revendication 9.

58. Composition de conversion de la biomasse végétale comprenant une enzyme selon la revendication 9.


### SEQUENCE LISTING

<110> Diversa Corporation
<120> Glycosidase Enzymes
<130> C 2072 EP/1 S3
<140>
   <141>
<160> 56
<170> PatentIn Ver. 2.1
<210> 1
   <211> 1446
   <212> DNA
   <213> Desulfurococcus sp.
<220>
   <221> CDS
   <222> (1)..(1443)
<400> 1
   atg aaa ttc ccc aaa gac ttc atg ata ggc tac tca tct tca ccg ttt    48
   Met Lys Phe Pro Lys Asp Phe Met Ile Gly Tyr Ser Ser Ser Pro Phe
    1               5                  10                  15
   caa ttt gaa gct ggt att ccc ggg tcc gag gat ccg aat agt gat tgg    96
   Gln Phe Glu Ala Gly Ile Pro Gly Ser Glu Asp Pro Asn Ser Asp Trp
               20                  25                  30
   tgg gta tgg gtg cat gat ccg gag aac aca gca gct gga cta gtc agc   144
   Trp Val Trp Val His Asp Pro Glu Asn Thr Ala Ala Gly Leu Val Ser
           35                  40                  45
   ggc gat ttt ccc gag aac ggc cca ggt tac tgg aat tta aac caa aat   192
   Gly Asp Phe Pro Glu Asn Gly Pro Gly Tyr Trp Asn Leu Asn Gln Asn
       50                  55                  60
   gac cac gac ctg gct gag aag ctg ggg gtt aac act att aga gta ggc   240
   Asp His Asp Leu Ala Glu Lys Leu Gly Val Asn Thr Ile Arg Val Gly
   65                  70                  75                  80
   gtt gag tgg agt agg att ttt cca aag cca act ttc aat gtt aaa gtc   288
   Val Glu Trp Ser Arg Ile Phe Pro Lys Pro Thr Phe Asn Val Lys Val
                   85                  90                  95
   cct gta gag aga gat gag aac ggc agc att gtt cac gta gat gtc gat   336
   Pro Val Glu Arg Asp Glu Asn Gly Ser Ile Val His Val Asp Val Asp
               100                 105                 110
   gat aaa gcg gtt gaa aga ctt gat gaa tta gcc aac aag gag gcc gta   384
   Asp Lys Ala Val Glu Arg Leu Asp Glu Leu Ala Asn Lys Glu Ala Val
           115                 120                 125
   aac cat tac gta gaa atg tat aaa gac tgg gtt gaa aga ggt aga aaa   432
   Asn His Tyr Val Glu Met Tyr Lys Asp Trp Val Glu Arg Gly Arg Lys
       130                 135                 140
   ctt ata ctc aat tta tac cat tgg ccc ctg cct ctc tgg ctt cac aac    480
   Leu Ile Leu Asn Leu Tyr His Trp Pro Leu Pro Leu Trp Leu His Asn
   145                 150                 155                 160
   cca atc atg gtg aga aga atg ggc ccg gac aga gcg ccc tca ggc tgg    528
   Pro Ile Met Val Arg Arg Met Gly Pro Asp Arg Ala Pro Ser Gly Trp
                   165                 170                 175
   ctt aac gag gag tcc gtg gtg gag ttt gcc aaa tac gcc gca tac att    576
   Leu Asn Glu Glu Ser Val Val Glu Phe Ala Lys Tyr Ala Ala Tyr Ile
               180                 185                 190
   gct tgg aaa atg ggc gag cta cct gtt atg tgg agc acc atg aac gaa    624
   Ala Trp Lys Met Gly Glu Leu Pro Val Met Trp Ser Thr Met Asn Glu
           195                 200                 205
   ccc aac gtc gtt tat gag caa gga tac atg ttc gtt aaa ggg ggt ttc    672
   Pro Asn Val Val Tyr Glu Gln Gly Tyr Met Phe Val Lys Gly Gly Phe
       210                 215                 220
   cca ccc ggc tac ttg agt ttg gaa gct gct gat aag gcc agg aga aat    720
   Pro Pro Gly Tyr Leu Ser Leu Glu Ala Ala Asp Lys Ala Arg Arg Asn
   225                 230                 235                 240
   atg atc cag gct cat gca cgg gcc tat gac aat att aaa cgc ttc agt    768
   Met Ile Gln Ala His Ala Arg Ala Tyr Asp Asn Ile Lys Arg Phe Ser
                   245                 250                 255
   aag aaa cct gtt gga cta ata tac gct ttc caa tgg ttc gaa cta tta    816
   Lys Lys Pro Val Gly Leu Ile Tyr Ala Phe Gln Trp Phe Glu Leu Leu
               260                 265                 270
   gag ggt cca gca gaa gta ttt gat aag ttt aag agc tct aag tta tac    864
   Glu Gly Pro Ala Glu Val Phe Asp Lys Phe Lys Ser Ser Lys Leu Tyr
           275                 280                 285
   tat ttc aca gac ata gta tcg aag ggt agt tca atc atc aat gtt gaa    912
   Tyr Phe Thr Asp Ile Val Ser Lys Gly Ser Ser Ile Ile Asn Val Glu
       290                 295                 300
   tac agg aga gat ctt gcc aat agg cta gac tgg ttg ggc gtt aac tac    960
   Tyr Arg Arg Asp Leu Ala Asn Arg Leu Asp Trp Leu Gly Val Asn Tyr
   305                 310                 315                 320
   tat agc cgt tta gtc tac aaa atc gtc gat gac aaa cct ata atc ctg    1008
   Tyr Ser Arg Leu Val Tyr Lys Ile Val Asp Asp Lys Pro Ile Ile Leu
                   325                 330                 335
   cac ggg tat gga ttc ctt tgt aca cct ggg ggg atc agc ccg gct gaa    1056
   His Gly Tyr Gly Phe Leu Cys Thr Pro Gly Gly Ile Ser Pro Ala Glu
               340                 345                 350
   aat cct tgt agc gat ttt ggg tgg gag gtg tat cct gaa gga ctc tac    1104
   Asn Pro Cys Ser Asp Phe Gly Trp Glu Val Tyr Pro Glu Gly Leu Tyr
           355                 360                 365
   cta ctt cta aaa gaa ctt tac aac cga tac ggg gta gac ttg atc gtg    1152
   Leu Leu Leu Lys Glu Leu Tyr Asn Arg Tyr Gly Val Asp Leu Ile Val
       370                 375                 380
   acc gag aac ggt gtt tca gac agc agg gat gcg ttg aga ccg gca tac     1200
   Thr Glu Asn Gly Val Ser Asp Ser Arg Asp Ala Leu Arg Pro Ala Tyr
   385                 390                 395                 400
   ctg gtc tcg cat gtt tac agc gta tgg aaa gcc gct aac gag ggc att     1248
   Leu Val Ser His Val Tyr Ser Val Trp Lys Ala Ala Asn Glu Gly Ile
                   405                 410                 415
   ccc gtc aaa ggc tac ctc cac tgg agc ttg aca gac aat tac gag tgg     1296
   Pro Val Lys Gly Tyr Leu His Trp Ser Leu Thr Asp Asn Tyr Glu Trp
               420                 425                 430
   gcc cag ggc ttc agg cag aaa ttc ggt tta gtc atg gtt gac ttc aaa     1344
   Ala Gln Gly Phe Arg Gln Lys Phe Gly Leu Val Met Val Asp Phe Lys
           435                 440                 445
   act aag aaa agg tat ctc cgc cca agc gcc cta gtg ttc cgg gag atc     1392
   Thr Lys Lys Arg Tyr Leu Arg Pro Ser Ala Leu Val Phe Arg Glu Ile
       450                 455                 460
   gca acg cat aac gga ata ccg gat gag cta cag cat ctt aca ctg atc     1440
   Ala Thr His Asn Gly Ile Pro Asp Glu Leu Gln His Leu Thr Leu Ile
   465                 470                 475                 480
   cag taa                                                             1446
   Gln
<210> 2
   <211> 1317
   <212> DNA
   <213> Thermotoga sp.
<220>
   <221> CDS
   <222> (1).. (1314)
<400> 2
   atg ata aga agg tcc gat ttt cca aaa gat ttt atc ttc gga acg gct     48
   Met Ile Arg Arg Ser Asp Phe Pro Lys Asp Phe Ile Phe Gly Thr Ala
    1               5                  10                  15
   acg gca gca tac cag att gaa ggt gca gca aac gaa gat ggc aga ggg     96
   Thr Ala Ala Tyr Gln Ile Glu Gly Ala Ala Asn Glu Asp Gly Arg Gly
               20                  25                  30
   cca tca att tgg gat gtc ttt tca cac acg cct ggc aaa acc ctg aac    144
   Pro Ser Ile Trp Asp Val Phe Ser His Thr Pro Gly Lys Thr Leu Asn
           35                  40                  45
   ggt gac aca gga gac gtt gcg tgt gac cat tat cac cga tac aag gaa    192
   Gly Asp Thr Gly Asp Val Ala Cys Asp His Tyr His Arg Tyr Lys Glu
       50                  55                  60
   gat atc cag ctg atg aaa gaa ata ggg tta gac gct tac agg ttc tct    240
   Asp Ile Gln Leu Met Lys Glu Ile Gly Leu Asp Ala Tyr Arg Phe Ser
   65                  70                  75                  80
   atc tcc tgg ccc aga att atg cca gat ggg aag aac atc aac caa aag    288
   Ile Ser Trp Pro Arg Ile Met Pro Asp Gly Lys Asn Ile Asn Gln Lys
                   85                  90                  95
   ggt gtg gat ttc tac aac aga ctc gtt gat gag ctt ttg aag aat gat    336
   Gly Val Asp Phe Tyr Asn Arg Leu Val Asp Glu Leu Leu Lys Asn Asp
               100                 105                 110
   atc ata cca ttc gta aca ctc tat cac tgg gac tta ccc tac gca ctt    384
   Ile Ile Pro Phe Val Thr Leu Tyr His Trp Asp Leu Pro Tyr Ala Leu
           115                 120                 125
   tat gaa aaa ggt gga tgg ctt aac cca gat ata gcg ctc tat ttc aga    432
   Tyr Glu Lys Gly Gly Trp Leu Asn Pro Asp Ile Ala Leu Tyr Phe Arg
       130                 135                 140
   gca tac gca acg ttt atg ttc aac gaa ctc ggt gat cgt gtg aaa cat    480
   Ala Tyr Ala Thr Phe Met Phe Asn Glu Leu Gly Asp Arg Val Lys His
   145                 150                 155                 160
   tgg att aca ctg aac gaa cca tgg tgt tct tct ttc tcg ggt tat tac    528
   Trp Ile Thr Leu Asn Glu Pro Trp Cys Ser Ser Phe Ser Gly Tyr Tyr
                   165                 170                 175
   acg gga gag cat gcc ccg ggt cat caa aat tta caa gaa gcg ata atc    576
   Thr Gly Glu His Ala Pro Gly His Gln Asn Leu Gln Glu Ala Ile Ile
               180                 185                 190
   gcg gcg cac aac ctg ttg agg gaa cat gga cat gcc gtc cag gcg tcc    624
   Ala Ala His Asn Leu Leu Arg Glu His Gly His Ala Val Gln Ala Ser
           195                 200                 205
   aga gaa gaa gta aaa gat ggg gaa gtt ggc tta acc aac gtt gtg atg    672
   Arg Glu Glu Val Lys Asp Gly Glu Val Gly Leu Thr Asn Val Val Met
       210                 215                 220
   aaa ata gaa ccg ggc gat gca aaa ccc gaa agt ttc ttg gtc gca agt    720
   Lys Ile Glu Pro Gly Asp Ala Lys Pro Glu Ser Phe Leu Val Ala Ser
   225                 230                 235                 240
   ctt gtt gat aag ttc gtt aat gca tgg tcc cat gac cct gtt gtt ttc    768
   Leu Val Asp Lys Phe Val Asn Ala Trp Ser His Asp Pro Val Val Phe
                   245                 250                 255
   gga aaa tat ccc gaa gaa gca gtt gca ctt tat acg gaa aaa ggg ttg    816
   Gly Lys Tyr Pro Glu Glu Ala Val Ala Leu Tyr Thr Glu Lys Gly Leu
               260                 265                 270
   caa gtt ctc gat agc gat atg aat att att tcg act cct ata gac ttc    864
   Gln Val Leu Asp Ser Asp Met Asn Ile Ile Ser Thr Pro Ile Asp Phe
           275                 280                 285
   ttt ggt gtg aat tat tac aca aga aca ctt gtt gtt ttt gat atg aac    912
   Phe Gly Val Asn Tyr Tyr Thr Arg Thr Leu Val Val Phe Asp Met Asn
       290                 295                 300
   aat cct ctt gga ttt tcg tat gtt cag gga gac ctt ccc aaa acg gag    960
   Asn Pro Leu Gly Phe Ser Tyr Val Gln Gly Asp Leu Pro Lys Thr Glu
   305                 310                 315                 320
   atg gga tgg gaa atc tac ccg cag gga tta ttt gat atg ctg gtc tat    1008
   Met Gly Trp Glu Ile Tyr Pro Gln Gly Leu Phe Asp Met Leu Val Tyr
                   325                 330                 335
   ctg aag gaa aga tat aaa cta cca ctt tat atc aca gag aac ggg atg    1056
   Leu Lys Glu Arg Tyr Lys Leu Pro Leu Tyr Ile Thr Glu Asn Gly Met
               340                 345                 350
   gct gga cct gat aaa ttg gaa aac gga aga gtt cat gat aat tac cga    1104
   Ala Gly Pro Asp Lys Leu Glu Asn Gly Arg Val His Asp Asn Tyr Arg
           355                 360                 365
   att gaa tat ttg gaa aag cac ttt gaa aaa gca ctt gaa gca atc aat    1152
   Ile Glu Tyr Leu Glu Lys His Phe Glu Lys Ala Leu Glu Ala Ile Asn
       370                 375                 380
   gca gat gtt gat ttg aaa ggt tac ttc att tgg tct ttg atg gat aac    1200
   Ala Asp Val Asp Leu Lys Gly Tyr Phe Ile Trp Ser Leu Met Asp Asn
   385                 390                 395                 400
   ttc gaa tgg gcg tgc gga tac tcc aaa cgt ttc ggt ata atc tac gta    1248
   Phe Glu Trp Ala Cys Gly Tyr Ser Lys Arg Phe Gly Ile Ile Tyr Val
                   405                 410                 415
   gat tac aat acc cca aaa agg ata ttg aaa gat tca gcg atg tgg ttg    1296
   Asp Tyr Asn Thr Pro Lys Arg Ile Leu Lys Asp Ser Ala Met Trp Leu
               420                 425                 430
   aag gaa ttt cta aaa tct taa                                        1317
   Lys Glu Phe Leu Lys Ser
           435
<210> 3
   <211> 1266
   <212> DNA
   <213> Staphylothermus marinus
<220>
   <221> CDS
   <222> (1)..(1263) .
<400> 3
   atg ata agg ttt cct gat tat ttc ttg ttt gga aca gct aca tca tcg      48
   Met Ile Arg Phe Pro Asp Tyr Phe Leu Phe Gly Thr Ala Thr Ser Ser
    1               5                  10                  15
   cac cag atc gag ggt aat aac ata ttt aat gat tgg tgg gag tgg gag      96
   His Gln Ile Glu Gly Asn Asn Ile Phe Asn Asp Trp Trp Glu Trp Glu
               20                  25                  30
   act aaa ggc agg att aag gtg aga tcg ggt aag gca tgt aat cat tgg     144
   Thr Lys Gly Arg Ile Lys Val Arg Ser Gly Lys Ala Cys Asn His Trp
           35                  40                  45
   gaa ctc tat aaa gaa gac ata gag ctt atg gct gag ctg gga tat aat     192
   Glu Leu Tyr Lys Glu Asp Ile Glu Leu Met Ala Glu Leu Gly Tyr Asn
       50                  55                  60
   gct tat agg ttc tcc ata gag tgg agt aga ata ttt ccc aga aaa gat     240
   Ala Tyr Arg Phe Ser Ile Glu Trp Ser Arg Ile Phe Pro Arg Lys Asp
   65                  70                  75                  80
   cat ata gat tat gag tcg ctt aat aag tat aag gaa ata gtt aat cta     288
   His Ile Asp Tyr Glu Ser Leu Asn Lys Tyr Lys Glu Ile Val Asn Leu
                   85                  90                  95
   ctt aga aaa tac ggg ata gaa cct gta atc act ctt cac cac ttc aca     336
   Leu Arg Lys Tyr Gly Ile Glu Pro Val Ile Thr Leu His His Phe Thr
               100                 105                 110
   aac ccg caa tgg ttt atg aaa att ggt gga tgg act agg gaa gag aac     384
   Asn Pro Gln Trp Phe Met Lys Ile Gly Gly Trp Thr Arg Glu Glu Asn
           115                 120                 125
   ata aaa tat ttt ata aaa tat gta gaa ctt ata gct tcc gag ata aaa     432
   Ile Lys Tyr Phe Ile Lys Tyr Val Glu Leu Ile Ala Ser Glu Ile Lys
       130                 135                 140
   gac gtg aaa ata tgg atc act att aat gaa cca ata ata tat gtt tta     480
   Asp Val Lys Ile Trp Ile Thr Ile Asn Glu Pro Ile Ile Tyr Val Leu
   145                 150                 155                 160
   caa gga tat att tcc ggc gaa tgg cca cct gga att aaa aat tta aaa     528
   Gln Gly Tyr Ile Ser Gly Glu Trp Pro Pro Gly Ile Lys Asn Leu Lys
                   165                 170                 175
   ata gct gat caa gta act aag aat ctt tta aaa gca cat aat gaa gcc     576
   Ile Ala Asp Gln Val Thr Lys Asn Leu Leu Lys Ala His Asn Glu Ala
               180                 185                 190
   tat aat ata ctt cat aaa cac ggt att gta ggc ata gct aaa aac atg     624
   Tyr Asn Ile Leu His Lys His Gly Ile Val Gly Ile Ala Lys Asn Met
           195                 200                 205
   ata gca ttt aaa cca gga tct aat aga gga aaa gac att aat att tat     672
   Ile Ala Phe Lys Pro Gly Ser Asn Arg Gly Lys Asp Ile Asn Ile Tyr
       210                 215                 220
   cat aaa gtc gat aaa gca ttc aac tgg gga ttt ctc aac gga ata tta     720
   His Lys Val Asp Lys Ala Phe Asn Trp Gly Phe Leu Asn Gly Ile Leu
   225                 230                 235                 240
   agg gga gaa cta gaa act ctc cgt gga aaa tac cga gtt gag ccc gga     768
   Arg Gly Glu Leu Glu Thr Leu Arg Gly Lys Tyr Arg Val Glu Pro Gly
                   245                 250                 255
   aat att gat ttc ata ggc ata aac tat tat tca tca tat att gta aaa     816
   Asn Ile Asp Phe Ile Gly Ile Asn Tyr Tyr Ser Ser Tyr Ile Val Lys
               260                 265                 270
   tat act tgg aat cct ttt aaa cta cat att aaa gtc gaa cca tta gat     864
   Tyr Thr Trp Asn Pro Phe Lys Leu His Ile Lys Val Glu Pro Leu Asp
           275                 280                 285
   aca ggt cta tgg aca act atg ggt tac tgc ata tat cct aga gga ata     912
   Thr Gly Leu Trp Thr Thr Met Gly Tyr Cys Ile Tyr Pro Arg Gly Ile
       290                 295                 300
   tat gaa gtt gta atg aaa act cat gag aaa tac ggc aaa gaa ata atc     960
   Tyr Glu Val Val Met Lys Thr His Glu Lys Tyr Gly Lys Glu Ile Ile
   305                 310                 315                 320
   att aca gag aac ggt gtt gca gta gaa aat gat gaa tta agg att tta    1008
   Ile Thr Glu Asn Gly Val Ala Val Glu Asn Asp Glu Leu Arg Ile Leu
                   325                 330                 335
   tcc att atc agg cac tta caa tac tta tat aaa gcc atg aat gaa gga    1056
   Ser Ile Ile Arg His Leu Gln Tyr Leu Tyr Lys Ala Met Asn Glu Gly
               340                 345                 350
   gca aag gtg aaa gga tat ttc tac tgg agc ttc atg gat aat ttt gag    1104
   Ala Lys Val Lys Gly Tyr Phe Tyr Trp Ser Phe Met Asp Asn Phe Glu
           355                 360                 365
   tgg gat aaa gga ttt aac caa agg ttc gga cta gta gaa gtt gat tat    1152
   Trp Asp Lys Gly Phe Asn Gln Arg Phe Gly Leu Val Glu Val Asp Tyr
       370                 375                 380
   aag act ttt gag aga aaa cct aga aaa agc gca tat gta tat agt caa    1200
   Lys Thr Phe Glu Arg Lys Pro Arg Lys Ser Ala Tyr Val Tyr Ser Gln
   385                 390                 395                 400
   ata gca cgt acc aag act ata agt gat gaa tac cta gaa aaa tat gga    1248
   Ile Ala Arg Thr Lys Thr Ile Ser Asp Glu Tyr Leu Glu Lys Tyr Gly
                   405                 410                 415
   tta aag aac ctc gaa taa                                            1266
   Leu Lys Asn Leu Glu
               420
<210> 4
   <211> 1530
   <212> DNA
   <213> Thermococcus sp.
<220>
   <221> CDS
   <222> (1)..(1527)
<400> 4
   atg cta cca gaa ggc ttt ctc tgg ggc gtg tcc cag tcc ggc ttt cag    48
   Met Leu Pro Glu Gly Phe Leu Trp Gly Val Ser Gln Ser Gly Phe Gln
    1               5                  10                  15
   ttc gag atg ggc gac aag ctc agg agg aac att gat ccg aac aca gac    96
   Phe Glu Met Gly Asp Lys Leu Arg Arg Asn Ile Asp Pro Asn Thr Asp
               20                  25                  30
   tgg tgg aag tgg gtc agg gat ccc ttc aac ata aag agg gaa ctc gtc   144
   Trp Trp Lys Trp Val Arg Asp Pro Phe Asn Ile Lys Arg Glu Leu Val
           35                  40                  45
   agc ggc gac ctg ccc gag gag ggg ata aac aac tac gaa ctt tac gag   192
   Ser Gly Asp Leu Pro Glu Glu Gly Ile Asn Asn Tyr Glu Leu Tyr Glu
       50                  55                  60
   aag gat cac cgc ctc gcc aga gac ctc ggt ctg aac gtt tac agg att   240
   Lys Asp His Arg Leu Ala Arg Asp Leu Gly Leu Asn Val Tyr Arg Ile
   65                  70                  75                  80
   gga ata gag tgg agc agg atc ttt ccc tgg cca acg tgg ttt gtg gag      288
   Gly Ile Glu Trp Ser Arg Ile Phe Pro Trp Pro Thr Trp Phe Val Glu
                   85                  90                  95
   gtt gac gtt gag cgg gac agc tac gga ctc gtg aag gac gtc aaa atc      336
   Val Asp Val Glu Arg Asp Ser Tyr Gly Leu Val Lys Asp Val Lys Ile
               100                 105                 110
   gat aaa gac acg ctc gaa gag ctc gac gag ata gcg aat cat cag gag      384
   Asp Lys Asp Thr Leu Glu Glu Leu Asp Glu Ile Ala Asn His Gln Glu
           115                 120                 125
   ata gcc tac tac cgc cgc gtt ata gag cac ctc agg gag ctg ggc ttc      432
   Ile Ala Tyr Tyr Arg Arg Val Ile Glu His Leu Arg Glu Leu Gly Phe
       130                 135                 140
   aag gtc atc gtg aac ctc aac cac ttc acg ctc ccc ctc tgg ctt cac      480
   Lys Val Ile Val Asn Leu Asn His Phe Thr Leu Pro Leu Trp Leu His
   145                 150                 155                 160
   gat ccg ata atc gcg agg gag aag gcc ctc acc aac ggt agg att ggc      528
   Asp Pro Ile Ile Ala Arg Glu Lys Ala Leu Thr Asn Gly Arg Ile Gly
                   165                 170                 175
   atc gcg aac gca ctc ggg gac ctc gtt gat atg tgg agc acc ttc aac      576
   Ile Ala Asn Ala Leu Gly Asp Leu Val Asp Met Trp Ser Thr Phe Asn
               180                 185                 190
   gag ccg atg gtc gtt gtg gag ctc ggt tac ctc gcg ccc tac tcc ggc      624
   Glu Pro Met Val Val Val Glu Leu Gly Tyr Leu Ala Pro Tyr Ser Gly
           195                 200                 205
   ttt ccg ccg ggg gtt atg aac ccc gag gcg gca aag ctg gca atc ctc      672
   Phe Pro Pro Gly Val Met Asn Pro Glu Ala Ala Lys Leu Ala Ile Leu
       210                 215                 220
   tgg gtc ggg cag gag agc gtg gtg gag ttc gcc aag tac gcg gcg tac      720
   Trp Val Gly Gln Glu Ser Val Val Glu Phe Ala Lys Tyr Ala Ala Tyr
   225                 230                 235                 240
   aac atg ata aac gcc cac gca ctg gcc tac aag atg ata aag aag ttc      768
   Asn Met Ile Asn Ala His Ala Leu Ala Tyr Lys Met Ile Lys Lys Phe
                   245                 250                 255
   gac agg gta aag gcc gat aag gat tcc cgc tcc gag gcc gag gtc ggg      816
   Asp Arg Val Lys Ala Asp Lys Asp Ser Arg Ser Glu Ala Glu Val Gly
               260                 265                 270
   ata atc tac aac aac ata ggc gtt gcc tat cca tac gac tcc aac gac      864
   Ile Ile Tyr Asn Asn Ile Gly Val Ala Tyr Pro Tyr Asp Ser Asn Asp
           275                 280                 285
   cca aag gac gtg aaa gct gca gaa aac gac aac tac ttc cac agc ggg      912
   Pro Lys Asp Val Lys Ala Ala Glu Asn Asp Asn Tyr Phe His Ser Gly
       290                 295                 300
   ctc ttc ttc gac gca atc cac aag ggc aag ctc aac atc gag ttc gac      960
   Leu Phe Phe Asp Ala Ile His Lys Gly Lys Leu Asn Ile Glu Phe Asp
   305                 310                 315                 320
   ggt gag acc ttc gtc aaa gtt cgg cat ctc agg ggg aac gac tgg ata     1008
   Gly Glu Thr Phe Val Lys Val Arg His Leu Arg Gly Asn Asp Trp Ile
                   325                 330                 335
   ggc gtt aac tac tac acg aga gaa gtc gtc agg tat tcg gag ccc aag     1056
   Gly Val Asn Tyr Tyr Thr Arg Glu Val Val Arg Tyr Ser Glu Pro Lys
               340                 345                 350
   ttc ccg agc ata ccc ctg ata tcc ttc cgg gga gtt cac aac tac ggc     1104
   Phe Pro Ser Ile Pro Leu Ile Ser Phe Arg Gly Val His Asn Tyr Gly
           355                 360                 365
   tac gcc tgc agg ccc ggg agt tct tcc gcc gac gga agg ccc gta agc     1152
   Tyr Ala Cys Arg Pro Gly Ser Ser Ser Ala Asp Gly Arg Pro Val Ser
       370                 375                 380
   gac atc ggc tgg gag atc tat ccg gag ggg atc tac gac tcg ata aga     1200
   Asp Ile Gly Trp Glu Ile Tyr Pro Glu Gly Ile Tyr Asp Ser Ile Arg
   385                 390                 395                 400
   gag gcc aac aaa tac ggg gtc ccg gtt tac gtc acc gaa aac gga ata     1248
   Glu Ala Asn Lys Tyr Gly Val Pro Val Tyr Val Thr Glu Asn Gly Ile
                   405                 410                 415
   gcc gat tca act gac acc ctg cgg ccg tac tac ctc gcg agc cat gta     1296
   Ala Asp Ser Thr Asp Thr Leu Arg Pro Tyr Tyr Leu Ala Ser His Val
               420                 425                 430
   gcg aag att gag gag gcg tac gag gcg ggt tac gac gtc agg ggc tac     1344
   Ala Lys Ile Glu Glu Ala Tyr Glu Ala Gly Tyr Asp Val Arg Gly Tyr
           435                 440                 445
   ctc tac tgg gcg ctg acc gac aac tac gag tgg gcc ctc ggt ttc agg     1392
   Leu Tyr Trp Ala Leu Thr Asp Asn Tyr Glu Trp Ala Leu Gly Phe Arg
       450                 455                 460
   atg agg ttc ggc ctc tat aaa gtg gat ctc ata acc aag gag aga aca     1440
   Met Arg Phe Gly Leu Tyr Lys Val Asp Leu Ile Thr Lys Glu Arg Thr
   465                 470                 475                 480
   ccg cgg gag gaa agc gta aag gtt tat agg ggc atc gtg gag aac aac     1488
   Pro Arg Glu Glu Ser Val Lys Val Tyr Arg Gly Ile Val Glu Asn Asn
                   485                 490                 495
   gga gtg agc aag gaa atc cgg gag aag ttc gga ctt ggg tga             1530
   Gly Val Ser Lys Glu Ile Arg Glu Lys Phe Gly Leu Gly
               500                 505
<210> 5
   <211> 2166
   <212> DNA
   <213> Thermotoga maritima
<220>
   <221> CDS
   <222> (1)..(2163)
<400> 5
   atg gaa agg atc gat gaa att ctc tct cag tta act aca gag gaa aag    48
   Met Glu Arg Ile Asp Glu Ile Leu Ser Gln Leu Thr Thr Glu Glu Lys
    1               5                  10                  15
   gtg aag ctc gtt gtg ggg gtt ggt ctt cca gga ctt ttt ggg aac cca    96
   Val Lys Leu Val Val Gly Val Gly Leu Pro Gly Leu Phe Gly Asn Pro
               20                  25                  30
   cat tcc aga gtg gcg ggt gcg gct gga gaa aca cat ccc gtt cca aga   144
   His Ser Arg Val Ala Gly Ala Ala Gly Glu Thr His Pro Val Pro Arg
           35                  40                  45
   ctt gga att cct gcg ttt gtc ctg gca gat ggt ccc gca gga ctc aga   192
   Leu Gly Ile Pro Ala Phe Val Leu Ala Asp Gly Pro Ala Gly Leu Arg
       50                  55                  60
   ata aat ccc aca agg gaa aac gat gaa aac act tac tac acg acg gca   240
   Ile Asn Pro Thr Arg Glu Asn Asp Glu Asn Thr Tyr Tyr Thr Thr Ala
   65                  70                  75                  80
   ttt ccc gtt gaa atc atg ctc gct tct acc tgg aac aga gac ctt ctg   288
   Phe Pro Val Glu Ile Met Leu Ala Ser Thr Trp Asn Arg Asp Leu Leu
                   85                  90                  95
   gaa gaa gtg gga aaa gcc atg gga gaa gaa gtt agg gaa tac ggt gtc   336
   Glu Glu Val Gly Lys Ala Met Gly Glu Glu Val Arg Glu Tyr Gly Val
               100                 105                 110
   gat gtg ctt ctt gca cct gcg atg aac att cac aga aac cct ctt tgt   384
   Asp Val Leu Leu Ala Pro Ala Met Asn Ile His Arg Asn Pro Leu Cys
           115                 120                 125
   gga agg aat ttc gag tac tac tca gaa gat cct gtc ctt tcc ggt gaa   432
   Gly Arg Asn Phe Glu Tyr Tyr Ser Glu Asp Pro Val Leu Ser Gly Glu
       130                 135                 140
   atg gct tca gcc ttt gtc aag gga gtt caa tct caa ggg gtg gga gcc   480
   Met Ala Ser Ala Phe Val Lys Gly Val Gln Ser Gln Gly Val Gly Ala
   145                 150                 155                 160
   tgc ata aaa cac ttt gtc gcg aac aac cag gaa acg aac agg atg gta   528
   Cys Ile Lys His Phe Val Ala Asn Asn Gln Glu Thr Asn Arg Met Val
                   165                 170                 175
   gtg gac acg atc gtg tcc gag cga gcc ctc aga gaa ata tat ctg aaa   576
   Val Asp Thr Ile Val Ser Glu Arg Ala Leu Arg Glu Ile Tyr Leu Lys
               180                 185                 190
   ggt ttt gaa att gct gtc aag aaa gca aga ccc tgg acc gtg atg agc   624
   Gly Phe Glu Ile Ala Val Lys Lys Ala Arg Pro Trp Thr Val Met Ser
           195                 200                 205
   gct tac aac aaa ctg aat gga aaa tac tgt tca cag aac gaa tgg ctt   672
   Ala Tyr Asn Lys Leu Asn Gly Lys Tyr Cys Ser Gln Asn Glu Trp Leu
       210                 215                 220
   ttg aag aag gtt ctc agg gaa gaa tgg gga ttt ggc ggt ttc gtg atg   720
   Leu Lys Lys Val Leu Arg Glu Glu Trp Gly Phe Gly Gly Phe Val Met
   225                 230                 235                 240
   agc gac tgg tac gcg gga gac aac cct gta gaa cag ctc aag gcc gga    768
   Ser Asp Trp Tyr Ala Gly Asp Asn Pro Val Glu Gln Leu Lys Ala Gly
                   245                 250                 255
   aac gat atg atc atg cct ggg aaa gcg tat cag gtg aac aca gaa aga    816
   Asn Asp Met Ile Met Pro Gly Lys Ala Tyr Gln Val Asn Thr Glu Arg
               260                 265                 270
   aga gat gaa ata gaa gaa atc atg gag gcg ttg aag gag gga aaa ttg    864
   Arg Asp Glu Ile Glu Glu Ile Met Glu Ala Leu Lys Glu Gly Lys Leu
           275                 280                 285
   agt gag gag gtt ctc gat gag tgt gtg aga aac att ctc aaa gtt ctt    912
   Ser Glu Glu Val Leu Asp Glu Cys Val Arg Asn Ile Leu Lys Val Leu
       290                 295                 300
   gtg aac gcg cct tcc ttc aaa ggg tac agg tac tca aac aag ccg gat    960
   Val Asn Ala Pro Ser Phe Lys Gly Tyr Arg Tyr Ser Asn Lys Pro Asp
   305                 310                 315                 320
   ctc gaa tct cac gcg gaa gtc gcc tac gaa gca ggt gcg gag ggt gtt    1008
   Leu Glu Ser His Ala Glu Val Ala Tyr Glu Ala Gly Ala Glu Gly Val
                   325                 330                 335
   gtc ctt ctt gag aac aac ggt gtt ctt ccg ttc gat gaa aat acc cat    1056
   Val Leu Leu Glu Asn Asn Gly Val Leu Pro Phe Asp Glu Asn Thr His
               340                 345                 350
   gtc gcc gtc ttt ggc acc ggt caa atc gaa aca ata aag gga gga acg    1104
   Val Ala Val Phe Gly Thr Gly Gln Ile Glu Thr Ile Lys Gly Gly Thr
           355                 360                 365
   gga agt gga gac acc cat ccg aga tac acg atc tct atc ctt gaa ggc    1152
   Gly Ser Gly Asp Thr His Pro Arg Tyr Thr Ile Ser Ile Leu Glu Gly
       370                 375                 380
   ata aaa gaa aga aac atg aag ttc gac gaa gaa ctc gct tcc act tat    1200
   Ile Lys Glu Arg Asn Met Lys Phe Asp Glu Glu Leu Ala Ser Thr Tyr
   385                 390                 395                 400
   gag gag tac ata aaa aag atg aga gaa aca gag gaa tat aaa ccc aga    1248
   Glu Glu Tyr Ile Lys Lys Met Arg Glu Thr Glu Glu Tyr Lys Pro Arg
                   405                 410                 415
   acc gac tct tgg gga acg gtc ata aaa ccg aaa ctc cca gag aat ttc    1296
   Thr Asp Ser Trp Gly Thr Val Ile Lys Pro Lys Leu Pro Glu Asn Phe
               420                 425                 430
   ctc tca gaa aaa gag ata aag aaa cct cca aag aaa aac gat gtt gca    1344
   Leu Ser Glu Lys Glu Ile Lys Lys Pro Pro Lys Lys Asn Asp Val Ala
           435                 440                 445
   gtt gtt gtg atc agt agg atc tcc ggt gag gga tac gac aga aag ccg    1392
   Val Val Val Ile Ser Arg Ile Ser Gly Glu Gly Tyr Asp Arg Lys Pro
       450                 455                 460
   gtg aaa ggt gac ttc tac ctc tcc gat gac gag ctg gaa ctc ata aaa    1440
   Val Lys Gly Asp Phe Tyr Leu Ser Asp Asp Glu Leu Glu Leu Ile Lys
   465                 470                 475                 480
   acc gtc tcg aaa gaa ttc cac gat cag ggt aag aaa gtt gtg gtt ctt    1488
   Thr Val Ser Lys Glu Phe His Asp Gln Gly Lys Lys Val Val Val Leu
                   485                 490                 495
   ctg aac atc gga agt ccc atc gaa gtc gca agc tgg aga gac ctt gtg    1536
   Leu Asn Ile Gly Ser Pro Ile Glu Val Ala Ser Trp Arg Asp Leu Val
               500                 505                 510
   gat gga att ctt ctc gtc tgg cag gcg gga cag gag atg gga aga ata    1584
   Asp Gly Ile Leu Leu Val Trp Gln Ala Gly Gln Glu Met Gly Arg Ile
           515                 520                 525
   gtg gcc gat gtt ctt gtg gga aag att aat ccc tcc gga aaa ctt cca    1632
   Val Ala Asp Val Leu Val Gly Lys Ile Asn Pro Ser Gly Lys Leu Pro
       530                 535                 540
   acg acc ttc ccg aag gat tac tcg gac gtt cca tcc tgg acg ttc cca    1680
   Thr Thr Phe Pro Lys Asp Tyr Ser Asp Val Pro Ser Trp Thr Phe Pro
   545                 550                 555                 560
   gga gag cca aag gac aat ccg caa aga gtg gtg tac gag gaa gac atc    1728
   Gly Glu Pro Lys Asp Asn Pro Gln Arg Val Val Tyr Glu Glu Asp Ile
                   565                 570                 575
   tac gtg gga tac agg tac tac gac acc ttc ggt gtg gaa cct gcc tac    1776
   Tyr Val Gly Tyr Arg Tyr Tyr Asp Thr Phe Gly Val Glu Pro Ala Tyr
               580                 585                 590
   gaa ttc ggc tac ggc ctc tct tac aca aag ttt gaa tac aaa gat tta    1824
   Glu Phe Gly Tyr Gly Leu Ser Tyr Thr Lys Phe Glu Tyr Lys Asp Leu
           595                 600                 605
   aaa atc gct atc gac ggt gag acg ctc aga gtg tcg tac acg atc aca    1872
   Lys Ile Ala Ile Asp Gly Glu Thr Leu Arg Val Ser Tyr Thr Ile Thr
       610                 615                 620
   aac act ggg gac aga gct gga aag gaa gtc tca cag gtc tac atc aaa    1920
   Asn Thr Gly Asp Arg Ala Gly Lys Glu Val Ser Gln Val Tyr Ile Lys
   625                 630                 635                 640
   gct cca aaa gga aaa ata gac aaa ccc ttc cag gag ctg aaa gcg ttt    1968
   Ala Pro Lys Gly Lys Ile Asp Lys Pro Phe Gln Glu Leu Lys Ala Phe
                   645                 650                 655
   cac aaa aca aaa ctt ttg aac ccg ggt gaa tca gaa gaa atc tcc ttg    2016
   His Lys Thr Lys Leu Leu Asn Pro Gly Glu Ser Glu Glu Ile Ser Leu
               660                 665                 670
   gaa att cct ctc aga gat ctt gcg agt ttc gat ggg aaa gaa tgg gtt    2064
   Glu Ile Pro Leu Arg Asp Leu Ala Ser Phe Asp Gly Lys Glu Trp Val
           675                 680                 685
   gtc gag tca gga gaa tac gag gtc agg gtc ggt gca tct tcg agg gat    2112
   Val Glu Ser Gly Glu Tyr Glu Val Arg Val Gly Ala Ser Ser Arg Asp
       690                 695                 700
   ata agg ttg aga gat att ttt ctg gtt gag gga gag aag aga ttc aaa    2160
   Ile Arg Leu Arg Asp Ile Phe Leu Val Glu Gly Glu Lys Arg Phe Lys
   705                 710                 715                 720
   cca tga 2166
   Pro
<210> 6
   <211> 1365
   <212> DNA
   <213> Thermococcus alcaliphilus
<220>
   <221> CDS
   <222>. (1)..(1362)
<400> 6.
   atg atc cac tgc ccg gtt aaa ggg att ata tct gag gct cgc ggc ata     48
   Met Ile His Cys Pro Val Lys Gly Ile Ile Ser Glu Ala Arg Gly Ile
    1               5                  10                  15
   acc atc aca ata gat tta agt ttt caa ggc caa ata aat aat ttg gtg     96
   Thr Ile Thr Ile Asp Leu Ser Phe Gln Gly Gln Ile Asn Asn Leu Val
               20                  25                  30
   aat gct atg att gtc ttt ccg gag ttc ttc ctc ttt gga acc gcc aca    144
   Asn Ala Met Ile Val Phe Pro Glu Phe Phe Leu Phe Gly Thr Ala Thr
           35                  40                  45
   tct tct cat cag atc gag gga gat aat aaa tgg aac gac tgg tgg tat    192
   Ser Ser His Gln Ile Glu Gly Asp Asn Lys Trp Asn Asp Trp Trp Tyr
       50                  55                  60
   tat gag gag ata ggt aag ctc ccc tac aaa tcc ggt aaa gcc tgc aat    240
   Tyr Glu Glu Ile Gly Lys Leu Pro Tyr Lys Ser Gly Lys Ala Cys Asn
   65                  70                  75                  80
   cac tgg gag ctt tac agg gaa gat ata gag cta atg gca cag ctc ggc    288
   His Trp Glu Leu Tyr Arg Glu Asp Ile Glu Leu Met Ala Gln Leu Gly
                   85                  90                  95
   tac aat gcc tac cgc ttt tcg ata gag tgg agc cgt ctc ttc ccg gaa    336
   Tyr Asn Ala Tyr Arg Phe Ser Ile Glu Trp Ser Arg Leu Phe Pro Glu
               100                 105                 110
   gag ggc aaa ttc aat gaa gaa gcc ttc aac cgc tac cgt gaa ata att    384
   Glu Gly Lys Phe Asn Glu Glu Ala Phe Asn Arg Tyr Arg Glu Ile Ile
           115                 120                 125
   gaa atc ctc ctt gag aag ggg att act cca aac gtt aca ctg cac cac    432
   Glu Ile Leu Leu Glu Lys Gly Ile Thr Pro Asn Val Thr Leu His His
       130                 135                 140
   ttc aca tca ccg ctg tgg ttc atg cgg aag gga ggc ttt ttg aag gaa    480
   Phe Thr Ser Pro Leu Trp Phe Met Arg Lys Gly Gly Phe Leu Lys Glu
   145                 150                 155                 160
   gaa aac ctc aag tac tgg gag cag tac gtt gat aaa gcc gcg gag ctc    528
   Glu Asn Leu Lys Tyr Trp Glu Gln Tyr Val Asp Lys Ala Ala Glu Leu
                   165                 170                 175
   ctc aag gga gtc aag ctt gta gct aca ttc aac gag ccg atg gtc tat    576
   Leu Lys Gly Val Lys Leu Val Ala Thr Phe Asn Glu Pro Met Val Tyr
                   180                 185                 190
   gtt atg atg ggc tac ctc aca gcc tac tgg ccg ccc ttc atc aag agt      624
   Val Met Met Gly Tyr Leu Thr Ala Tyr Trp Pro Pro Phe Ile Lys Ser
           195                 200                 205
   ccc ttt aaa gcc ttt aaa gtt gcc gca aac ctc ctt aag gcc cat gca      672
   Pro Phe Lys Ala Phe Lys Val Ala Ala Asn Leu Leu Lys Ala His Ala
       210                 215                 220
   atg gca tat gat atc ctc cat ggt aac ttt gat gtg ggg ata gtt aaa      720
   Met Ala Tyr Asp Ile Leu His Gly Asn Phe Asp Val Gly Ile Val Lys
   225                 230                 235                 240
   aac atc ccc ata atg ctc cct gca agc aac aga gag aaa gac gta gaa      768
   Asn Ile Pro Ile Met Leu Pro Ala Ser Asn Arg Glu Lys Asp Val Glu
                   245                 250                 255
   gct gcc caa aag gcg gat aac ctc ttt aac tgg aac ttc ctt gat gca      816
   Ala Ala Gln Lys Ala Asp Asn Leu Phe Asn Trp Asn Phe Leu Asp Ala
               260                 265                 270
   ata tgg agc gga aaa tat aaa gga gct ttt gga act tac aaa act cca      864
   Ile Trp Ser Gly Lys Tyr Lys Gly Ala Phe Gly Thr Tyr Lys Thr Pro
           275                 280                 285
   gaa agc gat gca gac ttc ata ggg ata aac tac tac aca gcc agc gag      912
   Glu Ser Asp Ala Asp Phe Ile Gly Ile Asn Tyr Tyr Thr Ala Ser Glu
       290                 295                 300
   gta agg cat agc tgg aat ccg cta aag ttt ttc ttc gat gcc aag ctt      960
   Val Arg His Ser Trp Asn Pro Leu Lys Phe Phe Phe Asp Ala Lys Leu
   305                 310                 315                 320
   gca gac tta agc gag aga aaa aca gat atg ggt tgg agt gtc tat cca     1008
   Ala Asp Leu Ser Glu Arg Lys Thr Asp Met Gly Trp Ser Val Tyr Pro
                   325                 330                 335
   aag ggc ata tac gaa gct ata gca aag gtt tca cac tac gga aag cca     1056
   Lys Gly Ile Tyr Glu Ala Ile Ala Lys Val Ser His Tyr Gly Lys Pro
               340                 345                 350
   atg tac atc acg gaa aac ggg ata gct acc tta gac gat gag tgg agg     1104
   Met Tyr Ile Thr Glu Asn Gly Ile Ala Thr Leu Asp Asp Glu Trp Arg
           355                 360                 365
   ata gag ttt atc atc cag cac ctc cag tac gtt cac aaa gcc tta aac     1152
   Ile Glu Phe Ile Ile Gln His Leu Gln Tyr Val His Lys Ala Leu Asn
       370                 375                 380
   gat ggc ttt gac ttg aga ggc tac ttc tat tgg tct ttt atg gat aac     1200
   Asp Gly Phe Asp Leu Arg Gly Tyr Phe Tyr Trp Ser Phe Met Asp Asn
   385                 390                 395                 400
   ttc gag tgg gct gag ggt ttt aga cca cgc ttt ggg ctg gtc gag gtg     1248
   Phe Glu Trp Ala Glu Gly Phe Arg Pro Arg Phe Gly Leu Val Glu Val
                   405                 410                 415
   gac tac acg acc ttc aag agg aga ccg aga aag agt gct tac ata tat     1296
   Asp Tyr Thr Thr Phe Lys Arg Arg Pro Arg Lys Ser Ala Tyr Ile Tyr
               420                 425                 430
   gga gaa att gca agg gaa aag aaa ata aaa gac gaa ctg ctg gca aag      1344
   Gly Glu Ile Ala Arg Glu Lys Lys Ile Lys Asp Glu Leu Leu Ala Lys
           435                 440                 445
   tat ggg ctt ccg gag cta tga                                          1365
   Tyr Gly Leu Pro Glu Leu
       450
<210> 7
   <211> 1536
   <212> DNA
   <213> Thermococcus chitonophagus
<220>
   <221> CDS
   <222> (1) .. (1533)
<400> 7
   atg ctt cca gag aac ttt ctc tgg gga gtt tca cag tcc gga ttc cag     48
   Met Leu Pro Glu Asn Phe Leu Trp Gly Val Ser Gln Ser Gly Phe Gln
     1               5                  10                  15
   ttt gaa atg ggg gac aga ctg agg agg cac att gat cca aac aca gat     96
   Phe Glu Met Gly Asp Arg Leu Arg Arg His Ile Asp Pro Asn Thr Asp
                20                  25                  30
   tgg tgg tac tgg gta aga gat gaa tat aat atc aaa aaa gga cta gta    144
   Trp Trp Tyr Trp Val Arg Asp Glu Tyr Asn Ile Lys Lys Gly Leu Val
            35                  40                  45
   agt ggg gat ctt ccc gaa gac ggt ata aat tca tat gaa tta tat gag    192
   Ser Gly Asp Leu Pro Glu Asp Gly Ile Asn Ser Tyr Glu Leu Tyr Glu
        50                  55                  60
   aga gac caa gaa att gca aag gat tta ggg ctc aac aca tat agg atc    240
   Arg Asp Gln Glu Ile Ala Lys Asp Leu Gly Leu Asn Thr Tyr Arg Ile
    65                  70                  75                  80
   gga att gaa tgg agc aga gta ttt cca tgg cca acg act ttt gtc gac    288
   Gly Ile Glu Trp Ser Arg Val Phe Pro Trp Pro Thr Thr Phe Val Asp
                    85                  90                  95
   gtg gag tat gaa att gat gag tct tac ggg ttg gta aag gat gtg aag    336
   Val Glu Tyr Glu Ile Asp Glu Ser Tyr Gly Leu Val Lys Asp Val Lys
               100                 105                 110
   att tct aaa gac gca tta gaa aaa ctt gat gaa atc gct aac caa agg    384
   Ile Ser Lys Asp Ala Leu Glu Lys Leu Asp Glu Ile Ala Asn Gln Arg
           115                 120                 125
   gaa ata ata tat tat agg aac cta ata aat tcc cta aga aag agg ggt    432
   Glu Ile Ile Tyr Tyr Arg Asn Leu Ile Asn Ser Leu Arg Lys Arg Gly
       130                 135                 140
   ttt aag gta ata cta aac cta aat cat ttt acc ctc cca ata tgg ctt    480
   Phe Lys Val Ile Leu Asn Leu Asn His Phe Thr Leu Pro Ile Trp Leu
   145                 150                 155                 160
   cat gat cct atc gaa tct aga gaa aaa gcc ctg acc aat aag aga aac    528
   His Asp Pro Ile Glu Ser Arg Glu Lys Ala Leu Thr Asn Lys Arg Asn
                   165                 170                 175
   gga tgg gta agc gaa agg agt gtt ata gag ttt gca aaa ttt gcc gcg    576
   Gly Trp Val Ser Glu Arg Ser Val Ile Glu Phe Ala Lys Phe Ala Ala
               180                 185                 190
   tat tta gca tat aaa ttc gga gac ata gta gac atg tgg agc aca ttt    624
   Tyr Leu Ala Tyr Lys Phe Gly Asp Ile Val Asp Met Trp Ser Thr Phe
           195                 200                 205
   aat gaa cct atg gtg gtc gcc gag ttg ggg tat tta gcc cca tac tca    672
   Asn Glu Pro Met Val Val Ala Glu Leu Gly Tyr Leu Ala Pro Tyr Ser
       210                 215                 220
   gga ttc ccc ccg gga gtc atg aat cca gaa gca gca aag tta gtt atg    720
   Gly Phe Pro Pro Gly Val Met Asn Pro Glu Ala Ala Lys Leu Val Met
   225                 230                 235                 240
   cta cat atg ata aac gcc cat gct tta gca tat agg atg ata aag aaa    768
   Leu His Met Ile Asn Ala His Ala Leu Ala Tyr Arg Met Ile Lys Lys
                   245                 250                 255
   ttt gac aga aaa aaa gct gat cca gaa tca aaa gaa cca gct gaa ata    816
   Phe Asp Arg Lys Lys Ala Asp Pro Glu Ser Lys Glu Pro Ala Glu Ile
               260                 265                 270
   gga att ata tac aat aac atc ggc gtc aca tat ccg ttt aat ccg aaa    864
   Gly Ile Ile Tyr Asn Asn Ile Gly Val Thr Tyr Pro Phe Asn Pro Lys
           275                 280                 285
   gac tca aag gat cta caa gca tcc gat aat gcc aat ttc ttc cac agt    912
   Asp Ser Lys Asp Leu Gln Ala Ser Asp Asn Ala Asn Phe Phe His Ser
       290                 295                 300
   agg cta ttc tta acg gct atc cac agg gga aaa tta aat atc gaa ttt    960
   Arg Leu Phe Leu Thr Ala Ile His Arg Gly Lys Leu Asn Ile Glu Phe
   305                 310                 315                 320
   gac gga gag aca ttt gtt tac ctt cca tat tta aag ggc aat gat tgg    1008
   Asp Gly Glu Thr Phe Val Tyr Leu Pro Tyr Leu Lys Gly Asn Asp Trp
                   325                 330                 335
   ctg gga gtg aat tat tat aca aga gaa gtc gtt aaa tac caa gat ccc    1056
   Leu Gly Val Asn Tyr Tyr Thr Arg Glu Val Val Lys Tyr Gln Asp Pro
               340                 345                 350
   atg ttt cca agt atc cct ctc ata agc ttc aag ggc gtt cca gat tat    1104
   Met Phe Pro Ser Ile Pro Leu Ile Ser Phe Lys Gly Val Pro Asp Tyr
           355                 360                 365
   gga tac gga tgt aga cca gga acg acg tca aag gac ggt aat cct gtt    1152
   Gly Tyr Gly Cys Arg Pro Gly Thr Thr Ser Lys Asp Gly Asn Pro Val
       370                 375                 380
   agt gac att gga tgg gag gta tat ccc aaa ggc atg tac gac tct ata    1200
   Ser Asp Ile Gly Trp Glu Val Tyr Pro Lys Gly Met Tyr Asp Ser Ile
   385                 390                 395                 400
   gta gct gcc aat gaa tat gga gtt cct gta tac gta aca gaa aac gga     1248
   Val Ala Ala Asn Glu Tyr Gly Val Pro Val Tyr Val Thr Glu Asn Gly
                   405                 410                 415
   ata gca gat tca aaa gat gta tta agg ccc tat tac atc gca tct cac     1296
   Ile Ala Asp Ser Lys Asp Val Leu Arg Pro Tyr Tyr Ile Ala Ser His
                   420                 425                 430
   att gaa gcc atg gaa gag gct tac gaa aat ggt tat gac gtg aga gga     1344
   Ile Glu Ala Met Glu Glu Ala Tyr Glu Asn Gly Tyr Asp Val Arg Gly
               435                 440                 445
   tac tta cac tgg gca tta acc gat aat tac gaa tgg gcc tta ggg ttc     1392
   Tyr Leu His Trp Ala Leu Thr Asp Asn Tyr Glu Trp Ala Leu Gly Phe
       450                 455                 460
   aga atg agg ttt ggc ttg tac gaa gta aac ttg ata acc aaa gag aga     1440
   Arg Met Arg Phe Gly Leu Tyr Glu Val Asn Leu Ile Thr Lys Glu Arg
   465                 470                 475                 480
   aaa ccc agg aaa aag agt gta aga gta ttc aga gag ata gtt att aat     1488
   Lys Pro Arg Lys Lys Ser Val Arg Val Phe Arg Glu Ile Val Ile Asn
                   485                 490                 495
   aat ggg cta aca agc aac atc agg aaa gag atc tta gag gag ggg tag     1536
   Asn Gly Leu Thr Ser Asn Ile Arg Lys Glu Ile Leu Glu Glu Gly
               500                 505                 510
<210> 8
   <211> 1533
   <212> DNA
   <213> Pyrococcus furiosus
<220>
   <221> CDS
   <222> (1)..(1530)
<400> 8
   atg ttc cct gaa aag ttc ctt tgg ggt gtg gca caa tcg ggt ttt cag     48
   Met Phe Pro Glu Lys Phe Leu Trp Gly Val Ala Gln Ser Gly Phe Gln
   1               5                   10                  15
   ttt gaa atg ggg gat aaa ctc agg agg aat att gac act aac act gat     96
   Phe Glu Met Gly Asp Lys Leu Arg Arg Asn Ile Asp Thr Asn Thr Asp
               20                  25                  30
   tgg tgg cac tgg gta agg gat aag aca aat ata gag aaa ggc ctc gtt     144
   Trp Trp His Trp Val Arg Asp Lys Thr Asn Ile Glu Lys Gly Leu Val
           35                  40                  45
   agt gga gat ctt ccc gag gag ggg att aac aat tac gag ctt tat gag     192
   Ser Gly Asp Leu Pro Glu Glu Gly Ile Asn Asn Tyr Glu Leu Tyr Glu
       50                  55                  60
   aag gac cat gag att gca aga aag ctg ggt ctt aat gct tac aga ata     240
   Lys Asp His Glu Ile Ala Arg Lys Leu Gly Leu Asn Ala Tyr Arg Ile
   65                  70                  75                  80
   ggc ata gag tgg agc aga ata ttc cca tgg cca acg aca ttt att gat     288
   Gly Ile Glu Trp Ser Arg Ile Phe Pro Trp Pro Thr Thr Phe Ile Asp
                   85                  90                  95
   gtt gat tat agc tat aat gaa tca tat aac ctt ata gaa gat gta aag     336
   Val Asp Tyr Ser Tyr Asn Glu Ser Tyr Asn Leu Ile Glu Asp Val Lys
               100                 105                 110
   atc acc aag gac act ttg gag gag tta gat gag atc gcc aac aag agg     384
   Ile Thr Lys Asp Thr Leu Glu Glu Leu Asp Glu Ile Ala Asn Lys Arg
           115                 120                 125
   gag gtg gcc tac tat agg tca gtc ata aac agc ctg agg agc aag ggg     432
   Glu Val Ala Tyr Tyr Arg Ser Val Ile Asn Ser Leu Arg Ser Lys Gly
       130                 135                 140
   ttt aag gtt ata gtt aat cta aat cac ttc acc ctt cca tat tgg ttg     480
   Phe Lys Val Ile Val Asn Leu Asn His Phe Thr Leu Pro Tyr Trp Leu
   145                 150                 155                 160
   cat gat ccc att gag gct agg gag agg gcg tta act aat aag agg aac     528
   His Asp Pro Ile Glu Ala Arg Glu Arg Ala Leu Thr Asn Lys Arg Asn
                   165                 170                 175
   ggc tgg gtt aac cca aga aca gtt ata gag ttt gca aag tat gcc gct     576
   Gly Trp Val Asn Pro Arg Thr Val Ile Glu Phe Ala Lys Tyr Ala Ala
               180                 185                 190
   tac ata gcc tat aag ttt gga gat ata gtg gat atg tgg agc acg ttt     624
   Tyr Ile Ala Tyr Lys Phe Gly Asp Ile Val Asp Met Trp Ser Thr Phe
           195                 200                 205
   aat gag cct atg gtg gtt gtt gag ctt ggc tac cta gcc ccc tac tct     672
   Asn Glu Pro Met Val Val Val Glu Leu Gly Tyr Leu Ala Pro Tyr Ser
       210                 215                 220
   ggc ttc cct cca ggg gtt cta aat cca gag gcc gca aag ctg gcg ata     720
   Gly Phe Pro Pro Gly Val Leu Asn Pro Glu Ala Ala Lys Leu Ala Ile
   225                 230                 235                 240
   ctt cac atg ata aat gca cat gct tta gct tat agg cag ata aag aag     768
   Leu His Met Ile Asn Ala His Ala Leu Ala Tyr Arg Gln Ile Lys Lys
                   245                 250                 255
   ttt gac act gag aaa gct gat aag gat tct aaa gag cct gca gaa gtt     816
   Phe Asp Thr Glu Lys Ala Asp Lys Asp Ser Lys Glu Pro Ala Glu Val
               260                 265                 270
   ggt ata att tac aac aac att gga gtt gct tat ccc aag gat ccg aac     864
   Gly Ile Ile Tyr Asn Asn Ile Gly Val Ala Tyr Pro Lys Asp Pro Asn
           275                 280                 285
   gat tcc aag gat gtt aag gca gca gaa aac gac aac ttc ttc cac tca     912
   Asp Ser Lys Asp Val Lys Ala Ala Glu Asn Asp Asn Phe Phe His Ser
       290                 295                 300
   ggg ctg ttc ttc gag gcc ata cac aaa gga aaa ctt aat ata gag ttt     960
   Gly Leu Phe Phe Glu Ala Ile His Lys Gly Lys Leu Asn Ile Glu Phe
   305                 310                 315                 320
   gac ggt gaa acg ttt ata gat gcc ccc tat cta aag ggc aat gac tgg    1008
   Asp Gly Glu Thr Phe Ile Asp Ala Pro Tyr Leu Lys Gly Asn Asp Trp
                   325                 330                 335
   ata ggg gtt aat tac tac aca agg gaa gta gtt acg tat cag gaa cca    1056
   Ile Gly Val Asn Tyr Tyr Thr Arg Glu Val Val Thr Tyr Gln Glu Pro
               340                 345                 350
   atg ttt cct tca atc ccg ctg atc acc ttt aag gga gtt caa gga tat    1104
   Met Phe Pro Ser Ile Pro Leu Ile Thr Phe Lys Gly Val Gln Gly Tyr
           355                 360                 365
   ggc tat gcc tgc aga cct gga act ctg tca aag gat gac aga ccc gtc    1152
   Gly Tyr Ala Cys Arg Pro Gly Thr Leu Ser Lys Asp Asp Arg Pro Val
       370                 375                 380
   agc gac ata gga tgg gaa ctc tat cca gag ggg atg tac gat tca ata    1200
   Ser Asp Ile Gly Trp Glu Leu Tyr Pro Glu Gly Met Tyr Asp Ser Ile
   385                 390                 395                 400
   gtt gaa gct cac aag tac ggc gtt cca gtt tac gtg acg gag aac gga    1248
   Val Glu Ala His Lys Tyr Gly Val Pro Val Tyr Val Thr Glu Asn Gly
                   405                 410                 415
   ata gcg gat tca aag gac atc cta aga cct tac tac ata gcg agc cac    1296
   Ile Ala Asp Ser Lys Asp Ile Leu Arg Pro Tyr Tyr Ile Ala Ser His
               420                 425                 430
   ata aag atg ata gag aag gcc ttt gag gat ggg tat gaa gtt aag ggc    1344
   Ile Lys Met Ile Glu Lys Ala Phe Glu Asp Gly Tyr Glu Val Lys Gly
           435                 440                 445
   tac ttc cac tgg gca tta act gac aac ttc gag tgg gct ctc ggg ttt    1392
   Tyr Phe His Trp Ala Leu Thr Asp Asn Phe Glu Trp Ala Leu Gly Phe
       450                 455                 460
   aga atg cgc ttt ggc ctc tac gaa gtc aac cta att aca aag gag aga    1440
   Arg Met Arg Phe Gly Leu Tyr Glu Val Asn Leu Ile Thr Lys Glu Arg
   465                 470                 475                 480
   att ccc agg gag aag agc gtg tcg ata ttc aga gag ata gta gcc aat    1488
   Ile Pro Arg Glu Lys Ser Val Ser Ile Phe Arg Glu Ile Val Ala Asn
                   485                 490                 495
   aat ggt gtt acg aaa aag att gaa gag gaa ttg ctg agg gga tga        1533
   Asn Gly Val Thr Lys Lys Ile Glu Glu Glu Leu Leu Arg Gly
               500                 505                 510
<210> 9
   <211> 1614
   <212> DNA
   <213> Bankia gouldi
<220>
   <221> CDS
   <222> (1)..(1611)
<400> 9
   atg aga ata cgt tta gcg acg ctc gcg ctc tgc gca gcg ctg agc cca 48
   Met Arg Ile Arg Leu Ala Thr Leu Ala Leu Cys Ala Ala Leu Ser Pro
    1               5                  10                  15
   gtc acc ttt gca gat aat gta acc gta caa atc gac gcc gac ggc ggt    96
   Val Thr Phe Ala Asp Asn Val Thr Val Gln Ile Asp Ala Asp Gly Gly
               20                  25                  30
   aaa aaa ctc atc agc cga gcc ctt tac ggc atg aat aac tcc aac gca   144
   Lys Lys Leu Ile Ser Arg Ala Leu Tyr Gly Met Asn Asn Ser Asn Ala
           35                  40                  45
   gaa agc ctt acc gat act gac tgg cag cgt ttt cgc gat gca ggt gtg   192
   Glu Ser Leu Thr Asp Thr Asp Trp Gln Arg Phe Arg Asp Ala Gly Val
       50                  55                  60
   cgc atg ctg cgg gaa aat ggc ggc aac aac agc acc aaa tat aac tgg   240
   Arg Met Leu Arg Glu Asn Gly Gly Asn Asn Ser Thr Lys Tyr Asn Trp
   65                  70                  75                  80
   caa ctg cac ctg agc agt cat ccg gat tgg tac aac aat gtc tac gcc   288
   Gln Leu His Leu Ser Ser His Pro Asp Trp Tyr Asn Asn Val Tyr Ala
                   85                  90                  95
   ggc aac aac aac tgg gac aac cgg gta gcc ctg att cag gaa aac ctg   336
   Gly Asn Asn Asn Trp Asp Asn Arg Val Ala Leu Ile Gln Glu Asn Leu
               100                 105                 110
   ccc ggc gcc gac acc atg tgg gca ttc cag ctc atc ggt aag gtc gcg   384
   Pro Gly Ala Asp Thr Met Trp Ala Phe Gln Leu Ile Gly Lys Val Ala
           115                 120                 125
   gcg act tct gcc tac aac ttt aac gat tgg gaa ttc aac cag tcg caa   432
   Ala Thr Ser Ala Tyr Asn Phe Asn Asp Trp Glu Phe Asn Gln Ser Gln
       130                 135                 140
   tgg tgg acc ggc gtc gct cag aat ctc gct ggc ggc ggt gaa ccc aat   480
   Trp Trp Thr Gly Val Ala Gln Asn Leu Ala Gly Gly Gly Glu Pro Asn
   145                 150                 155                 160
   ctg gac ggc ggc ggc gaa gcg ctg gtt gaa gga gac ccc aat ctc tac   528
   Leu Asp Gly Gly Gly Glu Ala Leu Val Glu Gly Asp Pro Asn Leu Tyr
                   165                 170                 175
   ctc atg gat tgg tcg cca gcc gac act gtg ggt att ctc gac cac tgg   576
   Leu Met Asp Trp Ser Pro Ala Asp Thr Val Gly Ile Leu Asp His Trp
               180                 185                 190
   ttt ggc gta aac ggg ctg ggc gtg cgg cgt ggc aaa gcc aaa tac tgg   624
   Phe Gly Val Asn Gly Leu Gly Val Arg Arg Gly Lys Ala Lys Tyr Trp
           195                 200                 205
   agt atg gat aac gag ccc ggc atc tgg gtt ggc acc cac gac gat gta   672
   Ser Met Asp Asn Glu Pro Gly Ile Trp Val Gly Thr His Asp Asp Val
       210                 215                 220
   gtg aaa gaa caa acg ccg gta gaa gat ttc ctg cac acc tat ttc gaa   720
   Val Lys Glu Gln Thr Pro Val Glu Asp Phe Leu His Thr Tyr Phe Glu
   225                 230                 235                 240
   acc gcc aaa aaa gcc cgc gcc aaa ttt ccc ggt att aaa atc acc ggt   768
   Thr Ala Lys Lys Ala Arg Ala Lys Phe Pro Gly Ile Lys Ile Thr Gly
                   245                 250                 255
   ccg gtg ccc gct aat gag tgg cag tgg tat gcc tgg ggc ggt ttc tcg      816
   Pro Val Pro Ala Asn Glu Trp Gln Trp Tyr Ala Trp Gly Gly Phe Ser
               260                 265                 270
   gta ccc cag gaa caa ggg ttt atg agc tgg atg gag tat ttc atc aag      864
   Val Pro Gln Glu Gln Gly Phe Met Ser Trp Met Glu Tyr Phe Ile Lys
           275                 280                 285
   cgg gtg tct gaa gag caa cgc gca agt ggt gtt cgc ctc ctc gat gta      912
   Arg Val Ser Glu Glu Gln Arg Ala Ser Gly Val Arg Leu Leu Asp Val
       290                 295                 300
   ctc gat ctg cac tac tac ccc ggc gct tac aat gcg gaa gat atc gtg      960
   Leu Asp Leu His Tyr Tyr Pro Gly Ala Tyr Asn Ala Glu Asp Ile Val
   305                 310                 315                 320
   caa tta cat cgc acg ttc ttc gac cgc gac ttt gtt tca ctg gat gcc     1008
   Gln Leu His Arg Thr Phe Phe Asp Arg Asp Phe Val Ser Leu Asp Ala
                   325                 330                 335
   aac ggg gtg aaa atg gta gaa ggt ggc tgg gat gac agc atc aac aag     1056
   Asn Gly Val Lys Met Val Glu Gly Gly Trp Asp Asp Ser Ile Asn Lys
               340                 345                 350
   gaa tat att ttc ggg cga gtg aac gat tgg ctc gag gaa tat atg ggg     1104
   Glu Tyr Ile Phe Gly Arg Val Asn Asp Trp Leu Glu Glu Tyr Met Gly
           355                 360                 365
   cca gac cat ggt gta acc ctg ggc tta acc gaa atg tgc gtg cgc aat     1152
   Pro Asp His Gly Val Thr Leu Gly Leu Thr Glu Met Cys Val Arg Asn
       370                 375                 380
   gtg aat ccg atg act acc gcc atc tgg tat gcc tcc atg ctc ggc acc     1200
   Val Asn Pro Met Thr Thr Ala Ile Trp Tyr Ala Ser Met Leu Gly Thr
   385                 390                 395                 400
   ttc gcg gat aac ggc gtc gaa ata ttc acc cca tgg tgc tgg aac acc     1248
   Phe Ala Asp Asn Gly Val Glu Ile Phe Thr Pro Trp Cys Trp Asn Thr
                   405                 410                 415
   gga atg tgg gaa aca ctc cac ctc ttc agc cgc tac aac aaa cct tat     1296
   Gly Met Trp Glu Thr Leu His Leu Phe Ser Arg Tyr Asn Lys Pro Tyr
               420                 425                 430
   cgg gtc gcc tcc agc tcc agt ctt gaa gag ttt gtc agc gcc tac agc     1344
   Arg Val Ala Ser Ser Ser Ser Leu Glu Glu Phe Val Ser Ala Tyr Ser
           435                 440                 445
   tcc att aac gaa gca gaa gac gcc atg acg gta ctt ctg gtg aat cgt     1392
   Ser Ile Asn Glu Ala Glu Asp Ala Met Thr Val Leu Leu Val Asn Arg
       450                 455                 460
   tcc act agc gag acc cac acc gcc act gtc gct atc gac gat ttc cca     1440
   Ser Thr Ser Glu Thr His Thr Ala Thr Val Ala Ile Asp Asp Phe Pro
   465                 470                 475                 480
   ctg gat ggc ccc tac cgc acc ctg cgc tta cac aac ctg ccg ggg gag     1488
   Leu Asp Gly Pro Tyr Arg Thr Leu Arg Leu His Asn Leu Pro Gly Glu
                   485                 490                 495
   gaa acc ttc gta tct cac cga gac aac gcc ctg gaa aaa ggt aca gtg    1536
   Glu Thr Phe Val Ser His Arg Asp Asn Ala Leu Glu Lys Gly Thr Val
               500                 505                 510
   cgc gcc agc gac aat acg gta aca ctg gag ttg ccc cct ctg tcc gtt    1584
   Arg Ala Ser Asp Asn Thr Val Thr Leu Glu Leu Pro Pro Leu Ser Val
           515                 520                 525
   act gca ata ttg ctc aag gcc cgg ccc taa                            1614
   Thr Ala Ile Leu Leu Lys Ala Arg Pro
       530                 535
<210> 10
   <211> 1668
   <212> DNA
   <213> Thermotoga maritima
<220>
   <221> CDS
   <222> (1)..(1665)
<400> 10
   gtg atc tgt gtg gaa ata ttc gga aag acc ttc aga gag gga aga ttc    48
   Val Ile Cys Val Glu Ile Phe Gly Lys Thr Phe Arg Glu Gly Arg Phe
     1               5                  10                  15
   gtt ctc aaa gag aaa aac ttc aca gtt gag ttc gcg gtg gag aag ata    96
   Val Leu Lys Glu Lys Asn Phe Thr Val Glu Phe Ala Val Glu Lys Ile
                20                  25                  30
   cac ctt ggc tgg aag atc tcc ggc agg gtg aag gga agt ccg gga agg   144
   His Leu Gly Trp Lys Ile Ser Gly Arg Val Lys Gly Ser Pro Gly Arg
            35                  40                  45
   ctt gag gtt ctt cga acg aaa gca ccg gaa aag gta ctt gtg aac aac   192
   Leu Glu Val Leu Arg Thr Lys Ala Pro Glu Lys Val Leu Val Asn Asn
        50                  55                  60
   tgg cag tcc tgg gga ccg tgc agg gtg gtc gat gcc ttt tct ttc aaa   240
   Trp Gln Ser Trp Gly Pro Cys Arg Val Val Asp Ala Phe Ser Phe Lys
    65                  70                  75                  80
   cca cct gaa ata gat ccg aac tgg aga tac acc gct tcg gtg gtg ccc   288
   Pro Pro Glu Ile Asp Pro Asn Trp Arg Tyr Thr Ala Ser Val Val Pro
                    85                  90                  95
   gat gta ctt gaa agg aac ctc cag agc gac tat ttc gtg gct gaa gaa   336
   Asp Val Leu Glu Arg Asn Leu Gln Ser Asp Tyr Phe Val Ala Glu Glu
               100                 105                 110
   gga aaa gtg tac ggt ttt ctg agt tcg aaa atc gca cat cct ttc ttc   384
   Gly Lys Val Tyr Gly Phe Leu Ser Ser Lys Ile Ala His Pro Phe Phe
           115                 120                 125
   gct gtg gaa gat ggg gaa ctt gtg gca tac ctc gaa tat ttc gat gtc   432
   Ala Val Glu Asp Gly Glu Leu Val Ala Tyr Leu Glu Tyr Phe Asp Val
           130                 135                 140
   gag ttc gac gac ttt gtt cct ctt gaa cct ctc gtt gta ctc gag gat    480
   Glu Phe Asp Asp Phe Val Pro Leu Glu Pro Leu Val Val Leu Glu Asp
   145                 150                 155                 160
   ccc aac aca ccc ctt ctt ctg gag aaa tac gcg gaa ctc gtc gga atg    528
   Pro Asn Thr Pro Leu Leu Leu Glu Lys Tyr Ala Glu Leu Val Gly Met
                   165                 170                 175
   gaa aac aac gcg aga gtt cca aaa cac aca ccc act gga tgg tgc agc    576
   Glu Asn Asn Ala Arg Val Pro Lys His Thr Pro Thr Gly Trp Cys Ser
               180                 185                 190
   tgg tac cat tac ttc ctt gat ctc acc tgg gaa gag acc ctc aag aac    624
   Trp Tyr His Tyr Phe Leu Asp Leu Thr Trp Glu Glu Thr Leu Lys Asn
           195                 200                 205
   ctg aag ctc gcg aag aat ttc ccg ttc gag gtc ttc cag ata gac gac    672
   Leu Lys Leu Ala Lys Asn Phe Pro Phe Glu Val Phe Gln Ile Asp Asp
       210                 215                 220
   gcc tac gaa aag gac ata ggt gac tgg ctc gtg aca aga gga gac ttt    720
   Ala Tyr Glu Lys Asp Ile Gly Asp Trp Leu Val Thr Arg Gly Asp Phe
   225                 230                 235                 240
   cca tcg gtg gaa gag atg gca aaa gtt ata gcg gaa aac ggt ttc atc    768
   Pro Ser Val Glu Glu Met Ala Lys Val Ile Ala Glu Asn Gly Phe Ile
                   245                 250                 255
   ccg ggc ata tgg acc gcc ccg ttc agt gtt tct gaa acc tcg gat gta    816
   Pro Gly Ile Trp Thr Ala Pro Phe Ser Val Ser Glu Thr Ser Asp Val
               260                 265                 270
   ttc aac gaa cat ccg gac tgg gta gtg aag gaa aac gga gag ccg aag    864
   Phe Asn Glu His Pro Asp Trp Val Val Lys Glu Asn Gly Glu Pro Lys
           275                 280                 285
   atg gct tac aga aac tgg aac aaa aag ata tac gcc ctc gat ctt tcg    912
   Met Ala Tyr Arg Asn Trp Asn Lys Lys Ile Tyr Ala Leu Asp Leu Ser
       290                 295                 300
   aaa gat gag gtt ctg aac tgg ctt ttc gat ctc ttc tca tct ctg aga    960
   Lys Asp Glu Val Leu Asn Trp Leu Phe Asp Leu Phe Ser Ser Leu Arg
   305                 310                 315                 320
   aag atg ggc tac agg tac ttc aag atc gac ttt ctc ttc gcg ggt gcc   1008
   Lys Met Gly Tyr Arg Tyr Phe Lys Ile Asp Phe Leu Phe Ala Gly Ala
                   325                 330                 335
   gtt cca gga gaa aga aaa aag aac ata aca cca att cag gcg ttc aga   1056
   Val Pro Gly Glu Arg Lys Lys Asn Ile Thr Pro Ile Gln Ala Phe Arg
               340                 345                 350
   aaa ggg att gag acg atc aga aaa gcg gtg gga gaa gat tct ttc atc   1104
   Lys Gly Ile Glu Thr Ile Arg Lys Ala Val Gly Glu Asp Ser Phe Ile
           355                 360                 365
   ctc gga tgc ggc tct ccc ctt ctt ccc gca gtg gga tgc gtc gac ggg   1152
   Leu Gly Cys Gly Ser Pro Leu Leu Pro Ala Val Gly Cys Val Asp Gly
   370 375 380
   atg agg ata gga cct gac act gcg ccg ttc tgg gga gaa cat ata gaa 1200
   Met Arg Ile Gly Pro Asp Thr Ala Pro Phe Trp Gly Glu His Ile Glu
   385 390 395 400
   gac aac gga gct ccc gct gca aga tgg gcg ctg aga aac gcc ata acg 1248
   Asp Asn Gly Ala Pro Ala Ala Arg Trp Ala Leu Arg Asn Ala Ile Thr
   405 410 415
   agg tac ttc atg cac gac agg ttc tgg ctg aac gac ccc gac tgt ctg 1296
   Arg Tyr Phe Met His Asp Arg Phe Trp Leu Asn Asp Pro Asp Cys Leu
   420 425 430
   ata ctg aga gag gag aaa acg gat ctc aca cag aag gaa aag gag ctc 1344
   Ile Leu Arg Glu Glu Lys Thr Asp Leu Thr Gln Lys Glu Lys Glu Leu
   435 440 445
   tac tcg tac acg tgt gga gtg ctc gac aac atg atc ata gaa agc gat 1392
   Tyr Ser Tyr Thr Cys Gly Val Leu Asp Asn Met Ile Ile Glu Ser Asp
   450 455 460
   gat ctc tcg ctc gtc aga gat cat gga aaa aag gtt ctg aaa gaa acg 1440
   Asp Leu Ser Leu Val Arg Asp His Gly Lys Lys Val Leu Lys Glu Thr
   465 470 475 480
   ctc gaa ctc ctc ggt gga aga cca cgg gtt caa aac atc atg tcg gag 1488
   Leu Glu Leu Leu Gly Gly Arg Pro Arg Val Gln Asn Ile Met Ser Glu
   485 490 495
   gat ctg aga tac gag atc gtc tcg tct ggc act ctc tca gga aac gtc 1536
   Asp Leu Arg Tyr Glu Ile Val Ser Ser Gly Thr Leu Ser Gly Asn Val
   500 505 510
   aag atc gtg gtc gat ctg aac agc aga gag tac cac ctg gaa aaa gaa 1584
   Lys Ile Val Val Asp Leu Asn Ser Arg Glu Tyr His Leu Glu Lys Glu
   515 520 525
   gga aag tcc tcc ctg aaa aaa aga gtc gtc aaa aga gaa gac gga aga 1632
   Gly Lys Ser Ser Leu Lys Lys Arg Val Val Lys Arg Glu Asp Gly Arg
   530 535 540
   aac ttc tac ttc tac gaa gag ggt gag aga gaa tga 1668
   Asn Phe Tyr Phe Tyr Glu Glu Gly Glu Arg Glu
   545 550 555
<210> 11
   <211> 2043
   <212> DNA
   <213> Thermotoga maritima
<220>
   <221> CDS
   <222> (1)..(2090)
<400> 11
   atg ggg att ggt ggc gac gac tcc tgg agc ccg tca gta tcg gcg gaa    48
   Met Gly Ile Gly Gly Asp Asp Ser Trp Ser Pro Ser Val Ser Ala Glu
    1              5                  10                  15
   ttc ctt tta ttg atc gtt gag ctc tct ttc gtt ctc ttt gca agt gac      96
   Phe Leu Leu Leu Ile Val Glu Leu Ser Phe Val Leu Phe Ala Ser Asp
               20                  25                  30
   gag ttc gtg aaa gtg gaa aac gga aaa ttc gct ctg aac gga aaa gaa     144
   Glu Phe Val Lys Val Glu Asn Gly Lys Phe Ala Leu Asn Gly Lys Glu
           35                  40                  45
   ttc aga ttc att gga agc aac aac tac tac atg cac tac aag agc aac     192
   Phe Arg Phe Ile Gly Ser Asn Asn Tyr Tyr Met His Tyr Lys Ser Asn
       50                  55                  60
   gga atg ata gac agt gtt ctg gag agt gcc aga gac atg ggt ata aag     240
   Gly Met Ile Asp Ser Val Leu Glu Ser Ala Arg Asp Met Gly Ile Lys
   65                  70                  75                  80
   gtc ctc aga atc tgg ggt ttc ctc gac ggg gag agt tac tgc aga gac     288
   Val Leu Arg Ile Trp Gly Phe Leu Asp Gly Glu Ser Tyr Cys Arg Asp
                   85                  90                  95
   aag aac acc tac atg cat cct gag ccc ggt gtt ttc ggg gtg cca gaa     336
   Lys Asn Thr Tyr Met His Pro Glu Pro Gly Val Phe Gly Val Pro Glu
               100                 105                 110
   gga ata tcg aac gcc cag agc ggt ttc gaa aga ctc gac tac aca gtt     384
   Gly Ile Ser Asn Ala Gln Ser Gly Phe Glu Arg Leu Asp Tyr Thr Val
           115                 120                 125
   gcg aaa gcg aaa gaa ctc ggt ata aaa ctt gtc att gtt ctt gtg aac     432
   Ala Lys Ala Lys Glu Leu Gly Ile Lys Leu Val Ile Val Leu Val Asn
       130                 135                 140
   aac tgg gac gac ttc ggt gga atg aac cag tac gtg agg tgg ttt gga     480
   Asn Trp Asp Asp Phe Gly Gly Met Asn Gln Tyr Val Arg Trp Phe Gly
   145                 150                 155                 160
   gga acc cat cac gac gat ttc tac aga gat gag aag atc aaa gaa gag     528
   Gly Thr His His Asp Asp Phe Tyr Arg Asp Glu Lys Ile Lys Glu Glu
                   165                 170                 175
   tac aaa aag tac gtc tcc ttt ctc gta aac cat gtc aat acc tac acg     576
   Tyr Lys Lys Tyr Val Ser Phe Leu Val Asn His Val Asn Thr Tyr Thr
               180                 185                 190
   gga gtt cct tac agg gaa gag ccc acc atc atg gcc tgg gag ctt gca     624
   Gly Val Pro Tyr Arg Glu Glu Pro Thr Ile Met Ala Trp Glu Leu Ala
           195                 200                 205
   aac gaa ccg cgc tgt gag acg gac aaa tcg ggg aac acg ctc gtt gag     672
   Asn Glu Pro Arg Cys Glu Thr Asp Lys Ser Gly Asn Thr Leu Val Glu
       210                 215                 220
   tgg gtg aag gag atg agc tcc tac ata aag agt ctg gat ccc aac cac     720
   Trp Val Lys Glu Met Ser Ser Tyr Ile Lys Ser Leu Asp Pro Asn His
   225                 230                 235                 240
   ctc gtg gct gtg ggg gac gaa gga ttc ttc agc aac tac gaa gga ttc     768
   Leu Val Ala Val Gly Asp Glu Gly Phe Phe Ser Asn Tyr Glu Gly Phe
                   245                 250                 255
   aaa cct tac ggt gga gaa gcc gag tgg gcc tac aac ggc tgg tcc ggt     816
   Lys Pro Tyr Gly Gly Glu Ala Glu Trp Ala Tyr Asn Gly Trp Ser Gly
                   260                 265                 270
   gtt gac tgg aag aag ctc ctt tcg ata gag acg gtg gac ttc ggc acg     864
   Val Asp Trp Lys Lys Leu Leu Ser Ile Glu Thr Val Asp Phe Gly Thr
               275                 280                 285
   ttc cac ctc tat ccg tcc cac tgg ggt gtc agt cca gag aac tat gcc     912
   Phe His Leu Tyr Pro Ser His Trp Gly Val Ser Pro Glu Asn Tyr Ala
       290                 295                 300
   cag tgg gga gcg aag tgg ata gaa gac cac ata aag atc gca aaa gag     960
   Gln Trp Gly Ala Lys Trp Ile Glu Asp His Ile Lys Ile Ala Lys Glu
   305                 310                 315                 320
   atc gga aaa ccc gtt gtt ctg gaa gaa tat gga att cca aag agt gcg    1008
   Ile Gly Lys Pro Val Val Leu Glu Glu Tyr Gly Ile Pro Lys Ser Ala
                   325                 330                 335
   cca gtt aac aga acg gcc atc tac aga ctc tgg aac gat ctg gtc tac    1056
   Pro Val Asn Arg Thr Ala Ile Tyr Arg Leu Trp Asn Asp Leu Val Tyr
               340                 345                 350
   gat ctc ggt gga gat gga gcg atg ttc tgg atg ctc gcg gga atc ggg    1104
   Asp Leu Gly Gly Asp Gly Ala Met Phe Trp Met Leu Ala Gly Ile Gly
           355                 360                 365
   gaa ggt tcg gac aga gac gag aga ggg tac tat ccg gac tac gac ggt    1152
   Glu Gly Ser Asp Arg Asp Glu Arg Gly Tyr Tyr Pro Asp Tyr Asp Gly
       370                 375                 380
   ttc aga ata gtg aac gac gac agt cca gaa gcg gaa ctg ata aga gaa    1200
   Phe Arg Ile Val Asn Asp Asp Ser Pro Glu Ala Glu Leu Ile Arg Glu
   385                 390                 395                 400
   tac gcg aag ctg ttc aac aca ggt gaa gac ata aga gaa gac acc tgc    1248
   Tyr Ala Lys Leu Phe Asn Thr Gly Glu Asp Ile Arg Glu Asp Thr Cys
                   405                 410                 415
   tct ttc atc ctt cca aaa gac ggc atg gag atc aaa aag acc gtg gaa    1296
   Ser Phe Ile Leu Pro Lys Asp Gly Met Glu Ile Lys Lys Thr Val Glu
               420                 425                 430
   gtg agg gct ggt gtt ttc gac tac agc aac acg ttt gaa aag ttg tct    1344
   Val Arg Ala Gly Val Phe Asp Tyr Ser Asn Thr Phe Glu Lys Leu Ser
           435                 440                 445
   gtc aaa gtc gaa gat ctg gtt ttt gaa aat gag ata gag cat ctc gga    1392
   Val Lys Val Glu Asp Leu Val Phe Glu Asn Glu Ile Glu His Leu Gly
       450                 455                 460
   tac gga att tac ggc ttt gat ctc gac aca acc cgg atc ccg gat gga    1440
   Tyr Gly Ile Tyr Gly Phe Asp Leu Asp Thr Thr Arg Ile Pro Asp Gly
   465                 470                 475                 480
   gaa cat gaa atg ttc ctt gaa ggc cac ttt cag gga aaa acg gtg aaa    1488
   Glu His Glu Met Phe Leu Glu Gly His Phe Gln Gly Lys Thr Val Lys
                   485                 490                 495
   gac tct atc aaa gcg aaa gtg gtg aac gaa gca cgg tac gtg ctc gca     1536
   Asp Ser Ile Lys Ala Lys Val Val Asn Glu Ala Arg Tyr Val Leu Ala
               500                 505                 510
   gag gaa gtt gat ttt tcc tct cca gaa gag gtg aaa aac tgg tgg aac     1584
   Glu Glu Val Asp Phe Ser Ser Pro Glu Glu Val Lys Asn Trp Trp Asn
           515                 520                 525
   agc gga acc tgg cag gca gag ttc ggg tca cct gac att gaa tgg aac     1632
   Ser Gly Thr Trp Gln Ala Glu Phe Gly Ser Pro Asp Ile Glu Trp Asn
       530                 535                 540
   ggt gag gtg gga aat gga gca ctg cag ctg aac gtg aaa ctg ccc gga     1680
   Gly Glu Val Gly Asn Gly Ala Leu Gln Leu Asn Val Lys Leu Pro Gly
   545                 550                 555                 560
   aag agc gac tgg gaa gaa gtg aga gta gca agg aag ttc gaa aga ctc     1728
   Lys Ser Asp Trp Glu Glu Val Arg Val Ala Arg Lys Phe Glu Arg Leu
                   565                 570                 575
   tca gaa tgt gag atc ctc gag tac gac atc tac att cca aac gtc gag     1776
   Ser Glu Cys Glu Ile Leu Glu Tyr Asp Ile Tyr Ile Pro Asn Val Glu
               580                 585                 590
   gga ctc aag gga agg ttg agg ccg tac gcg gtt ctg aac ccc ggc tgg     1824
   Gly Leu Lys Gly Arg Leu Arg Pro Tyr Ala Val Leu Asn Pro Gly Trp
           595                 600                 605
   gtg aag ata ggc ctc gac atg aac aac gcg aac gtg gaa agt gcg gag     1872
   Val Lys Ile Gly Leu Asp Met Asn Asn Ala Asn Val Glu Ser Ala Glu
       610                 615                 620
   atc atc act ttc ggc gga aaa gag tac aga aga ttc cat gta aga att     1920
   Ile Ile Thr Phe Gly Gly Lys Glu Tyr Arg Arg Phe His Val Arg Ile
   625                 630                 635                 640
   gag ttc gac aga aca gcg ggg gtg aaa gaa ctt cac ata gga gtt gtc     1968
   Glu Phe Asp Arg Thr Ala Gly Val Lys Glu Leu His Ile Gly Val Val
                   645                 650                 655
   ggt gat cat ctg agg tac gat gga ccg att ttc atc gat aat gtg aga     2016
   Gly Asp His Leu Arg Tyr Asp Gly Pro Ile Phe Ile Asp Asn Val Arg
               660                 665                 670
   ctt tat aaa aga aca gga ggt atg tga                                 2043
   Leu Tyr Lys Arg Thr Gly Gly Met
           675                 680
<210> 12
   <211> 1539.
   <212> DNA
   <213> *Thermococcus chitonophagus*
<220>
   <221> CDS
   <222> (1)..(1536)
<400> 12
   atg cta cca gaa gag ttc cta tgg ggc gtt ggg cag tca ggc ttt cag   48
   Met Leu Pro Glu Glu Phe Leu Trp Gly Val Gly Gln Ser Gly Phe Gln
    1               5                  10                  15
   ttc gaa atg ggc gac aag ctc agg agg cac atc gat cca aat acc gac   96
   Phe Glu Met Gly Asp Lys Leu Arg Arg His Ile Asp Pro Asn Thr Asp
               20                  25                  30
   tgg tgg aag tgg gtt cgc gat cct ttc aac ata aaa aag gag ctt gtg   144
   Trp Trp Lys Trp Val Arg Asp Pro Phe Asn Ile Lys Lys Glu Leu Val
           35                  40                  45
   agt ggg gac ctt ccc gag gac ggc atc aac aac tac gaa ctt ttt gaa   192
   Ser Gly Asp Leu Pro Glu Asp Gly Ile Asn Asn Tyr Glu Leu Phe Glu
       50                  55                  60
   aac gat cac aag ctc gct aaa ggc ctt gga ctc aac gca tac agg att   240
   Asn Asp His Lys Leu Ala Lys Gly Leu Gly Leu Asn Ala Tyr Arg Ile
   65                  70                  75                  80
   gga ata gag tgg agc aga atc ttt ccc tgg ccg acg tgg acg gtc gat   288
   Gly Ile Glu Trp Ser Arg Ile Phe Pro Trp Pro Thr Trp Thr Val Asp
                   85                  90                  95
   acc gag gtc gag ttc gac act tac ggt tta gta aag gac gtt aag ata   336
   Thr Glu Val Glu Phe Asp Thr Tyr Gly Leu Val Lys Asp Val Lys Ile
               100                 105                 110
   gac aag tcc acc ctt gct gaa ctc gac agg ctg gcc aac aag gag gag   384
   Asp Lys Ser Thr Leu Ala Glu Leu Asp Arg Leu Ala Asn Lys Glu Glu
           115                 120                 125
   gta atg tac tac agg cgc gtt att cag cat ttg agg gag ctc ggc ttc   432
   Val Met Tyr Tyr Arg Arg Val Ile Gln His Leu Arg Glu Leu Gly Phe
       130                 135                 140
   aag gtc ttc gtt aac ctc aac cac ttc acg ctt cca ata tgg ctc cac   480
   Lys Val Phe Val Asn Leu Asn His Phe Thr Leu Pro Ile Trp Leu His
   145                 150                 155                 160
   gac ccg ata gtg gca agg gag aag gcc ctc aca aac gac aga atc ggc   528
   Asp Pro Ile Val Ala Arg Glu Lys Ala Leu Thr Asn Asp Arg Ile Gly
                   165                 170                 175
   tgg gtc tcc cag agg aca gtt gtt gag ttt gcc aag tat gct gct tac   576
   Trp Val Ser Gln Arg Thr Val Val Glu Phe Ala Lys Tyr Ala Ala Tyr
               180                 185                 190
   atc gcc cat gcg ctc gga gac ctc gtg gac aca tgg agc acc ttc aac   624
   Ile Ala His Ala Leu Gly Asp Leu Val Asp Thr Trp Ser Thr Phe Asn
           195                 200                 205
   gaa cct atg gta gtt gtg gag ctc ggc tac ctc gcc ccc tac tca gga   672
   Glu Pro Met Val Val Val Glu Leu Gly Tyr Leu Ala Pro Tyr Ser Gly
       210                 215                 220
   ttt ccc ccg gga gtc atg aac ccc gag gcc gcg aag ctg gcg atc ctc   720
   Phe Pro Pro Gly Val Met Asn Pro Glu Ala Ala Lys Leu Ala Ile Leu
   225                 230                 235                 240
   aac atg ata aac gcc cac gcc ttg gca tat aag atg ata aag agg ttc    768
   Asn Met Ile Asn Ala His Ala Leu Ala Tyr Lys Met Ile Lys Arg Phe
                   245                 250                 255
   gac acc aag aag gcc gat gag gat agc aag tcc cct gcg gac gtt ggc    816
   Asp Thr Lys Lys Ala Asp Glu Asp Ser Lys Ser Pro Ala Asp Val Gly
               260                 265                 270
   ata att tac aac aac atc ggt gtt gcc tac cct aaa gac cct aac gat    864
   Ile Ile Tyr Asn Asn Ile Gly Val Ala Tyr Pro Lys Asp Pro Asn Asp
           275                 280                 285
   ccc aag gac gtt aaa gca gcc gaa aac gac aac tac ttc cac agc gga    912
   Pro Lys Asp Val Lys Ala Ala Glu Asn Asp Asn Tyr Phe His Ser Gly
       290                 295                 300
   ctg ttc ttt gat gcc atc cac aag ggt aag ctc aac ata gag ttc gac    960
   Leu Phe Phe Asp Ala Ile His Lys Gly Lys Leu Asn Ile Glu Phe Asp
   305                 310                 315                 320
   ggc gaa aac ttt gta aaa gtt aga cac cta aaa ggc aat gac tgg ata    1008
   Gly Glu Asn Phe Val Lys Val Arg His Leu Lys Gly Asn Asp Trp Ile
                   325                 330                 335
   ggc ctc aac tac tac acc cgc gag gtt gtt aga tat tcg gag ccc aag    1056
   Gly Leu Asn Tyr Tyr Thr Arg Glu Val Val Arg Tyr Ser Glu Pro Lys
               340                 345                 350
   ttc cca agt ata ccc ctc ata tcc ttc aag ggc gtt ccc aac tac ggc    1104
   Phe Pro Ser Ile Pro Leu Ile Ser Phe Lys Gly Val Pro Asn Tyr Gly
           355                 360                 365
   tac tcc tgc agg ccc ggc acg acc tcc gcc gat ggc atg ccc gtc agc    1152
   Tyr Ser Cys Arg Pro Gly Thr Thr Ser Ala Asp Gly Met Pro Val Ser
       370                 375                 380
   gat atc ggc tgg gaa gtc tat ccc cag gga atc tac gac tcg ata gtc    1200
   Asp Ile Gly Trp Glu Val Tyr Pro Gln Gly Ile Tyr Asp Ser Ile Val
   385                 390                 395                 400
   gag gcc acc aag tac agt gtt cct gtt tac gtc acc gag aac ggt gtt    1248
   Glu Ala Thr Lys Tyr Ser Val Pro Val Tyr Val Thr Glu Asn Gly Val
                   405                 410                 415
   gcg gat tcc gcg gac acg ctg agg cca tac tac ata gtc agc cac gtc    1296
   Ala Asp Ser Ala Asp Thr Leu Arg Pro Tyr Tyr Ile Val Ser His Val
               420                 425                 430
   tca aag ata gag gaa gcc att gag aat gga tac ccc gta aaa ggc tac    1344
   Ser Lys Ile Glu Glu Ala Ile Glu Asn Gly Tyr Pro Val Lys Gly Tyr
           435                 440                 445
   atg tac tgg gcg ctt acg gat aac tac gag tgg gcc ctc ggc ttc agc    1392
   Met Tyr Trp Ala Leu Thr Asp Asn Tyr Glu Trp Ala Leu Gly Phe Ser
       450                 455                 460
   atg agg ttt ggt ctc tac aag gtc gac ctc atc tcc aag gag agg atc    1440
   Met Arg Phe Gly Leu Tyr Lys Val Asp Leu Ile Ser Lys Glu Arg Ile
   465                 470                 475                 480
   ccg agg gag aga agc gtt gag ata tat cgc agg ata gtg cag tcc aac      1488
   Pro Arg Glu Arg Ser Val Glu Ile Tyr Arg Arg Ile Val Gln Ser Asn
                   485                 490                 495
   ggt gtt cct aag gat atc aaa gag gag ttc ctg aag ggt gag gag aaa      1536
   Gly Val Pro Lys Asp Ile Lys Glu Glu Phe Leu Lys Gly Glu Glu Lys
               500                 505                 510
   tga                                                                  1539
<210> 13
   <211> 1083
   <212> DNA
   <213> Thermotoga sp.
<220>
   <221> CDS
   <222> (1)..(1080)
<400> 13
   atg gta gaa aga cac ttc aga tat gtt ctt att tgc acc ctg ttt ctt    48
   Met Val Glu Arg His Phe Arg Tyr Val Leu Ile Cys Thr Leu Phe Leu
    1               5                  10                  15
   gtt atg ctc cta atc tca tcc act cag tgt gga aaa aat gaa cca aac    96
   Val Met Leu Leu Ile Ser Ser Thr Gln Cys Gly Lys Asn Glu Pro Asn
                20                  25                  30
   aaa aga gtg aat agc atg gaa cag tca gtt gct gaa agt gat agc aac   144
   Lys Arg Val Asn Ser Met Glu Gln Ser Val Ala Glu Ser Asp Ser Asn
            35                  40                  45
   tca gca ttt gaa tac aac aaa atg gta ggt aaa gga gta aat att gga   192
   Ser Ala Phe Glu Tyr Asn Lys Met Val Gly Lys Gly Val Asn Ile Gly
        50                  55                  60
   aat gct tta gaa gct cct ttc gaa gga gct tgg gga gta aga att gag   240
   Asn Ala Leu Glu Ala Pro Phe Glu Gly Ala Trp Gly Val Arg Ile Glu
   65                  70                  75                  80
   gat gaa tat ttt gag ata ata aag aaa agg gga ttt gat tct gtt agg   288
   Asp Glu Tyr Phe Glu Ile Ile Lys Lys Arg Gly Phe Asp Ser Val Arg
                   85                  90                  95
   att ccc ata aga tgg tca gca cat ata tcc gaa aag cca cca tat gat   336
   Ile Pro Ile Arg Trp Ser Ala His Ile Ser Glu Lys Pro Pro Tyr Asp
               100                 105                 110
   att gac agg aat ttc ctc gaa aga gtt aac cat gtt gtc gat agg gct   384
   Ile Asp Arg Asn Phe Leu Glu Arg Val Asn His Val Val Asp Arg Ala
           115                 120                 125
   ctt gag aat aat tta aca gta atc atc aat acg cac cat ttt gaa gaa   432
   Leu Glu Asn Asn Leu Thr Val Ile Ile Asn Thr His His Phe Glu Glu
       130                 135                 140
   ctc tat caa gaa ccg gat aaa tac ggc gat gtt ttg gtg gaa att tgg   480
   Leu Tyr Gln Glu Pro Asp Lys Tyr Gly Asp Val Leu Val Glu Ile Trp
   145                 150                 155                 160
   aga cag att gca aaa ttc ttt aaa gat tac ccg gaa aat ctg ttc ttt     528
   Arg Gln Ile Ala Lys Phe Phe Lys Asp Tyr Pro Glu Asn Leu Phe Phe
                   165                 170                 175
   gaa atc tac aac gag cct gct cag aac ttg aca gct gaa aaa tgg aac     576
   Glu Ile Tyr Asn Glu Pro Ala Gln Asn Leu Thr Ala Glu Lys Trp Asn
               180                 185                 190
   gca ctt tat cca aaa gtg ctc aaa gtt atc agg gag agc aat cca acc     624
   Ala Leu Tyr Pro Lys Val Leu Lys Val Ile Arg Glu Ser Asn Pro Thr
           195                 200                 205
   cgg att gtc att atc gat gct cca aac tgg gca cac tat agc gca gtg     672
   Arg Ile Val Ile Ile Asp Ala Pro Asn Trp Ala His Tyr Ser Ala Val
       210                 215                 220
   aga agt cta aaa tta gtc aac gac aaa cgc atc att gtt tcc ttc cat     720
   Arg Ser Leu Lys Leu Val Asn Asp Lys Arg Ile Ile Val Ser Phe His
   225                 230                 235                 240
   tac tac gaa cct ttc aaa ttc aca cat cag ggt gcc gaa tgg gtt aat     768
   Tyr Tyr Glu Pro Phe Lys Phe Thr His Gln Gly Ala Glu Trp Val Asn
                   245                 250                 255
   ccc atc cca cct gtt agg gtt aag tgg aat ggc gag gaa tgg gaa att     816
   Pro Ile Pro Pro Val Arg Val Lys Trp Asn Gly Glu Glu Trp Glu Ile
               260                 265                 270
   aac caa atc aga agt cat ttc aaa tac gtg agt gac tgg gca aag caa     864
   Asn Gln Ile Arg Ser His Phe Lys Tyr Val Ser Asp Trp Ala Lys Gln
           275                 280                 285
   aat aac gta cca atc ttt ctt ggt gaa ttc ggt gct tat tca aaa gca     912
   Asn Asn Val Pro Ile Phe Leu Gly Glu Phe Gly Ala Tyr Ser Lys Ala
       290                 295                 300
   gac atg gac tca agg gtt aag tgg acc gaa agt gtg aga aaa atg gcg     960
   Asp Met Asp Ser Arg Val Lys Trp Thr Glu Ser Val Arg Lys Met Ala
   305                 310                 315                 320
   gaa gaa ttt gga ttt tca tac gcg tat tgg gaa ttt tgt gca gga ttt    1008
   Glu Glu Phe Gly Phe Ser Tyr Ala Tyr Trp Glu Phe Cys Ala Gly Phe
                   325                 330                 335
   ggc ata tac gat aga tgg tct caa aac tgg atc gaa cca ttg gca aca    1056
   Gly Ile Tyr Asp Arg Trp Ser Gln Asn Trp Ile Glu Pro Leu Ala Thr
               340                 345                 350
   gct gtg gtt ggc aca ggc aaa gag taa                                1083
   Ala Val Val Gly Thr Gly Lys Glu
           355                 360
<210> 14
   <211> 2319
   <212> DNA
   <213> Thermotoga maritima
<220>
   <221> CDS
   <222> (1)..(2316)
<400> 14
   atg gat ctt aca aag gtg ggg atc ata gtg agg ctg aac gag tgg cag     48
   Met Asp Leu Thr Lys Val Gly Ile Ile Val Arg Leu Asn Glu Trp Gln
   1               5                   10                  15
   gca aaa gac gtg gca aaa gac agg ttc ata gag ata aaa gac gga aag     96
   Ala Lys Asp Val Ala Lys Asp Arg Phe Ile Glu Ile Lys Asp Gly Lys
               20                  25                  30
   gct gaa gtg tgg ata ctc cag gga gtg gaa gag att ttc tac gaa aaa    144
   Ala Glu Val Trp Ile Leu Gln Gly Val Glu Glu Ile Phe Tyr Glu Lys
           35                  40                  45
   cca gac aca tct ccc aga atc ttc ttc gca cag gca agg tcg aac aag    192
   Pro Asp Thr Ser Pro Arg Ile Phe Phe Ala Gln Ala Arg Ser Asn Lys
       50                  55                  60
   gtg atc gag gct ttt ctg acc aat cct gtg gat acg aaa aag aaa gaa    240
   Val Ile Glu Ala Phe Leu Thr Asn Pro Val Asp Thr Lys Lys Lys Glu
   65                  70                  75                  80
   ctc ttc aag gtt act gtt gac gga aaa gag att ccc gtc tca aga gtg    288
   Leu Phe Lys Val Thr Val Asp Gly Lys Glu Ile Pro Val Ser Arg Val
                   85                  90                  95
   gaa aag gcc gat ccc acg gac ata gac gtg acg aac tac gtg aga atc    336
   Glu Lys Ala Asp Pro Thr Asp Ile Asp Val Thr Asn Tyr Val Arg Ile
               100                 105                 110
   gtc ctt tct gaa tcc ctg aaa gaa gaa gac ctc aga aaa gac gtg gaa    384
   Val Leu Ser Glu Ser Leu Lys Glu Glu Asp Leu Arg Lys Asp Val Glu
           115                 120                 125
   ctg atc ata gaa ggt tac aaa ccg gca aga gtc atc atg atg gag atc    432
   Leu Ile Ile Glu Gly Tyr Lys Pro Ala Arg Val Ile Met Met Glu Ile
       130                 135                 140
   ctg gac gac tac tat tac gat gga gag ctc gga gcc gta tat tct cca    480
   Leu Asp Asp Tyr Tyr Tyr Asp Gly Glu Leu Gly Ala Val Tyr Ser Pro
   145                 150                 155                 160
   gag aag acg ata ttc aga gtc tgg tcc ccc gtt tct aag tgg gta aag    528
   Glu Lys Thr Ile Phe Arg Val Trp Ser Pro Val Ser Lys Trp Val Lys
                   165                 170                 175
   gtg ctt ctc ttc aaa aac gga gaa gac aca gaa ccg tac cag gtt gtg    576
   Val Leu Leu Phe Lys Asn Gly Glu Asp Thr Glu Pro Tyr Gln Val Val
               180                 185                 190
   aac atg gaa tac aag gga aac ggg gtc tgg gaa gcg gtt gtt gaa ggc    624
   Asn Met Glu Tyr Lys Gly Asn Gly Val Trp Glu Ala Val Val Glu Gly
           195                 200                 205
   gat ctc gac gga gtg ttc tac ctc tat cag ctg gaa aac tac gga aag    672
   Asp Leu Asp Gly Val Phe Tyr Leu Tyr Gln Leu Glu Asn Tyr Gly Lys
       210                 215                 220
   atc aga aca acc gtc gat cct tat tcg aaa gcg gtt tac gca aac aac    720
   Ile Arg Thr Thr Val Asp Pro Tyr Ser Lys Ala Val Tyr Ala Asn Asn
   225                 230                 235                 240
   caa gag agc gcc gtt gtg aat ctt gcc agg aca aac cca gaa gga tgg    768
   Gln Glu Ser Ala Val Val Asn Leu Ala Arg Thr Asn Pro Glu Gly Trp
                   245                 250                 255
   gaa aac gac agg gga ccg aaa atc gaa gga tac gaa gac gcg ata atc    816
   Glu Asn Asp Arg Gly Pro Lys Ile Glu Gly Tyr Glu Asp Ala Ile Ile
               260                 265                 270
   tat gaa ata cac ata gcg gac atc aca gga ctc gaa aac tcc ggg gta    864
   Tyr Glu Ile His Ile Ala Asp Ile Thr Gly Leu Glu Asn Ser Gly Val
           275                 280                 285
   aaa aac aaa ggc ctc tat ctc ggg ctc acc gaa gaa aac acg aaa gga    912
   Lys Asn Lys Gly Leu Tyr Leu Gly Leu Thr Glu Glu Asn Thr Lys Gly
       290                 295                 300
   ccg ggc ggt gtg aca aca ggc ctt tcg cac ctt gtg gaa ctc ggt gtt    960
   Pro Gly Gly Val Thr Thr Gly Leu Ser His Leu Val Glu Leu Gly Val
   305                 310                 315                 320
   aca cac gtt cat ata ctt cct ttc ttt gat ttc tac aca ggc gac gaa   1008
   Thr His Val His Ile Leu Pro Phe Phe Asp Phe Tyr Thr Gly Asp Glu
                   325                 330                 335
   ctc gat aaa gat ttc gag aag tac tac aac tgg ggt tac gat cct tac   1056
   Leu Asp Lys Asp Phe Glu Lys Tyr Tyr Asn Trp Gly Tyr Asp Pro Tyr
               340                 345                 350
   ctg ttc atg gtt ccg gag ggc aga tac tca acc gat ccc aaa aac cca   1104
   Leu Phe Met Val Pro Glu Gly Arg Tyr Ser Thr Asp Pro Lys Asn Pro
           355                 360                 365
   cac acg aga atc aga gaa gtc aaa gaa atg gtc aaa gcc ctt cac aaa   1152
   His Thr Arg Ile Arg Glu Val Lys Glu Met Val Lys Ala Leu His Lys
       370                 375                 380
   cac ggt ata ggt gtg att atg gac atg gtg ttc cct cac acc tac ggt   1200
   His Gly Ile Gly Val Ile Met Asp Met Val Phe Pro His Thr Tyr Gly
   385                 390                 395                 400
   ata ggc gaa ctc tct gcg ttc gat cag acg gtg ccg tac tac ttc tac   1248
   Ile Gly Glu Leu Ser Ala Phe Asp Gln Thr Val Pro Tyr Tyr Phe Tyr
                   405                 410                 415
   aga atc gac aag aca ggt gcc tat ttg aac gaa agc gga tgt ggt aac   1296
   Arg Ile Asp Lys Thr Gly Ala Tyr Leu Asn Glu Ser Gly Cys Gly Asn
               420                 425                 430
   gtc atc gca agc gaa aga ccc atg atg aga aaa ttc ata gtc gat acc   1344
   Val Ile Ala Ser Glu Arg Pro Met Met Arg Lys Phe Ile Val Asp Thr
           435                 440                 445
   gtc acc tac tgg gta aag gag tat cac ata gac gga ttc agg ttc gat   1392
   Val Thr Tyr Trp Val Lys Glu Tyr His Ile Asp Gly Phe Arg Phe Asp
       450                 455                 460
   cag atg ggt ctc atc gac aaa aag aca atg ctc gaa gtc gaa aga gct    1440
   Gln Met Gly Leu Ile Asp Lys Lys Thr Met Leu Glu Val Glu Arg Ala
   465                 470                 475                 480
   ctt cat aaa atc gat cca act atc att ctc tac ggc gaa ccg tgg ggt    1488
   Leu His Lys Ile Asp Pro Thr Ile Ile Leu Tyr Gly Glu Pro Trp Gly
                   485                 490                 495
   gga tgg gga gca ccg atc agg ttt gga aag agc gat gtc gcc ggc aca    1536
   Gly Trp Gly Ala Pro Ile Arg Phe Gly Lys Ser Asp Val Ala Gly Thr
               500                 505                 510
   cac gtg gca gct ttc aac gat gag ttc aga gac gca ata agg ggt tcc    1584
   His Val Ala Ala Phe Asn Asp Glu Phe Arg Asp Ala Ile Arg Gly Ser
           515                 520                 525
   gtg ttc aac ccg agc gtc aag gga ttc gtc atg gga gga tac gga aag    1632
   Val Phe Asn Pro Ser Val Lys Gly Phe Val Met Gly Gly Tyr Gly Lys
       530                 535                 540
   gaa acc aag atc aaa agg ggt gtt gtt gga agc ata aac tac gac gga    1680
   Glu Thr Lys Ile Lys Arg Gly Val Val Gly Ser Ile Asn Tyr Asp Gly
   545                 550                 555                 560
   aaa ctc atc aaa agt ttc gcc ctt gat cca gaa gaa act ata aac tac    1728
   Lys Leu Ile Lys Ser Phe Ala Leu Asp Pro Glu Glu Thr Ile Asn Tyr
                   565                 570                 575
   gca gcg tgt cac gac aac cac aca ctg tgg gac aag aac tac ctt gcc    1776
   Ala Ala Cys His Asp Asn His Thr Leu Trp Asp Lys Asn Tyr Leu Ala
               580                 585                 590
   gcc aaa gct gat aag aaa aag gaa tgg acc gaa gaa gaa ctg aaa aac    1824
   Ala Lys Ala Asp Lys Lys Lys Glu Trp Thr Glu Glu Glu Leu Lys Asn
           595                 600                 605
   gcc cag aaa ctg gct ggt gcg ata ctt ctc act tct caa ggt gtt cct    1872
   Ala Gln Lys Leu Ala Gly Ala Ile Leu Leu Thr Ser Gln Gly Val Pro
       610                 615                 620
   ttc ctc cac gga ggg cag gac ttc tgc agg acg acg aat ttc aac gac    1920
   Phe Leu His Gly Gly Gln Asp Phe Cys Arg Thr Thr Asn Phe Asn Asp
   625                 630                 635                 640
   aac tcc tac aac gcc cct atc tcg ata aac ggc ttc gat tac gaa aga    1968
   Asn Ser Tyr Asn Ala Pro Ile Ser Ile Asn Gly Phe Asp Tyr Glu Arg
                   645                 650                 655
   aaa ctt cag ttc ata gac gtg ttc aat tac cac aag ggt ctc ata aaa    2016
   Lys Leu Gln Phe Ile Asp Val Phe Asn Tyr His Lys Gly Leu Ile Lys
               660                 665                 670
   ctc aga aaa gaa cac cct gct ttc agg ctg aaa aac gct gaa gag atc    2064
   Leu Arg Lys Glu His Pro Ala Phe Arg Leu Lys Asn Ala Glu Glu Ile
           675                 680                 685
   aaa aaa cac ctg gaa ttt ctc ccg ggc ggg aga aga ata gtt gcg ttc    2112
   Lys Lys His Leu Glu Phe Leu Pro Gly Gly Arg Arg Ile Val Ala Phe
       690                 695                 700
   atg ctt aaa gac cac gca ggt ggt gat ccc tgg aaa gac atc gtg gtg     2160
   Met Leu Lys Asp His Ala Gly Gly Asp Pro Trp Lys Asp Ile Val Val
   705                 710                 715                 720
   att tac aat gga aac tta gag aag aca aca tac aaa ctg cca gaa gga     2208
   Ile Tyr Asn Gly Asn Leu Glu Lys Thr Thr Tyr Lys Leu Pro Glu Gly
                   725                 730                 735
   aaa tgg aat gtg gtt gtg aac agc cag aaa gcc gga aca gaa gtg ata     2256
   Lys Trp Asn Val Val Val Asn Ser Gln Lys Ala Gly Thr Glu Val Ile
               740                 745                 750
   gaa acc gtc gaa gga aca ata gaa ctc gat ccg ctt tcc gcg tac gtt     2304
   Glu Thr Val Glu Gly Thr Ile Glu Leu Asp Pro Leu Ser Ala Tyr Val
           755                 760                 765
   ctg tac aga gag tga                                                 2319
   Leu Tyr Arg Glu
       770
<210> 15
   <211> 481
   <212> PRT
   <213> Desulfurococcus sp.
<400> 15
   Met Lys Phe Pro Lys Asp Phe Met Ile Gly Tyr Ser Ser Ser Pro Phe
    1               5                  10                  15
   Gln Phe Glu Ala Gly Ile Pro Gly Ser Glu Asp Pro Asn Ser Asp Trp
               20                  25                  30
   Trp Val Trp Val His Asp Pro Glu Asn Thr Ala Ala Gly Leu Val Ser
           35                  40                  45
   Gly Asp Phe Pro Glu Asn Gly Pro Gly Tyr Trp Asn Leu Asn Gln Asn
       50                  55                  60
   Asp His Asp Leu Ala Glu Lys Leu Gly Val Asn Thr Ile Arg Val Gly
   65                  70                  75                  80
   Val Glu Trp Ser Arg Ile Phe Pro Lys Pro Thr Phe Asn Val Lys Val
                   85                  90                  95
   Pro Val Glu Arg Asp Glu Asn Gly Ser Ile Val His Val Asp Val Asp
               100                 105                 110
   Asp Lys Ala Val Glu Arg Leu Asp Glu Leu Ala Asn Lys Glu Ala Val
           115                 120                 125
   Asn His Tyr Val Glu Met Tyr Lys Asp Trp Val Glu Arg Gly Arg Lys
       130                 135                 140
   Leu Ile Leu Asn Leu Tyr His Trp Pro Leu Pro Leu Trp Leu His Asn
   145                 150                 155                 160
   Pro Ile Met Val Arg Arg Met Gly Pro Asp Arg Ala Pro Ser Gly Trp
                   165                 170                 175
   Leu Asn Glu Glu Ser Val Val Glu Phe Ala Lys Tyr Ala Ala Tyr Ile
   180 185 190
   Ala Trp Lys Met Gly Glu Leu Pro Val Met Trp Ser Thr Met Asn Glu
   195 200 205
   Pro Asn Val Val Tyr Glu Gln Gly Tyr Met Phe Val Lys Gly Gly Phe
   210 215 220
   Pro Pro Gly Tyr Leu Ser Leu Glu Ala Ala Asp Lys Ala Arg Arg Asn
   225 230 235 240
   Met Ile Gln Ala His Ala Arg Ala Tyr Asp Asn Ile Lys Arg Phe Ser
   245 250 255
   Lys Lys Pro Val Gly Leu Ile Tyr Ala Phe Gln Trp Phe Glu Leu Leu
   260 265 270
   Glu Gly Pro Ala Glu Val Phe Asp Lys Phe Lys Ser Ser Lys Leu Tyr
   275 280 285
   Tyr Phe Thr Asp Ile Val Ser Lys Gly Ser Ser Ile Ile Asn Val Glu
   290 295 300
   Tyr Arg Arg Asp Leu Ala Asn Arg Leu Asp Trp Leu Gly Val Asn Tyr
   305 310 315 320
   Tyr Ser Arg Leu Val Tyr Lys Ile Val Asp Asp Lys Pro Ile Ile Leu
   325 330 335
   His Gly Tyr Gly Phe Leu Cys Thr Pro Gly Gly Ile Ser Pro Ala Glu
   340 345 350
   Asn Pro Cys Ser Asp Phe Gly Trp Glu Val Tyr Pro Glu Gly Leu Tyr
   355 360 365
   Leu Leu Leu Lys Glu Leu Tyr Asn Arg Tyr Gly Val Asp Leu Ile Val
   370 375 380
   Thr Glu Asn Gly Val Ser Asp Ser Arg Asp Ala Leu Arg Pro Ala Tyr
   385 390 395 400
   Leu Val Ser His Val Tyr Ser Val Trp Lys Ala Ala Asn Glu Gly Ile
   405 410 415
   Pro Val Lys Gly Tyr Leu His Trp Ser Leu Thr Asp Asn Tyr Glu Trp
   420 425 430
   Ala Gln Gly Phe Arg Gln Lys Phe Gly Leu Val Met Val Asp Phe Lys
   435 440 445
   Thr Lys Lys Arg Tyr Leu Arg Pro Ser Ala Leu Val Phe Arg Glu Ile
   450 455 460
   Ala Thr His Asn Gly Ile Pro Asp Glu Leu Gln His Leu Thr Leu Ile
   465 470 475 480
   Gln
<210> 16
   <211> 438
   <212> PRT
   <213> Thermotoga sp.
<400> 16
   Met Ile Arg Arg Ser Asp Phe Pro Lys Asp Phe Ile Phe Gly Thr Ala
   1               5                   10                  15
   Thr Ala Ala Tyr Gln Ile Glu Gly Ala Ala Asn Glu Asp Gly Arg Gly
               20                  25                  30
   Pro Ser Ile Trp Asp Val Phe Ser His Thr Pro Gly Lys Thr Leu Asn
           35                  40                  45
   Gly Asp Thr Gly Asp Val Ala Cys Asp His Tyr His Arg Tyr Lys Glu
       50                  55                  60
   Asp Ile Gln Leu Met Lys Glu Ile Gly Leu Asp Ala Tyr Arg Phe Ser
   65                  70                  75                  80
   Ile Ser Trp Pro Arg Ile Met Pro Asp Gly Lys Asn Ile Asn Gln Lys
                   85                  90                  95
   Gly Val Asp Phe Tyr Asn Arg Leu Val Asp Glu Leu Leu Lys Asn Asp
               100                 105                 110
   Ile Ile Pro Phe Val Thr Leu Tyr His Trp Asp Leu Pro Tyr Ala Leu
           115                 120                 125
   Tyr Glu Lys Gly Gly Trp Leu Asn Pro Asp Ile Ala Leu Tyr Phe Arg
       130                 135                 140
   Ala Tyr Ala Thr Phe Met Phe Asn Glu Leu Gly Asp Arg Val Lys His
   145                 150                 155                 160
   Trp Ile Thr Leu Asn Glu Pro Trp Cys Ser Ser Phe Ser Gly Tyr Tyr
                   165                 170                 175
   Thr Gly Glu His Ala Pro Gly His Gln Asn Leu Gln Glu Ala Ile Ile
               180                 185                 190
   Ala Ala His Asn Leu Leu Arg Glu His Gly His Ala Val Gln Ala Ser
           195                 200                 205
   Arg Glu Glu Val Lys Asp Gly Glu Val Gly Leu Thr Asn Val Val Met
       210                 215                 220
   Lys Ile Glu Pro Gly Asp Ala Lys Pro Glu Ser Phe Leu Val Ala Ser
   225                 230                 235                 240
   Leu Val Asp Lys Phe Val Asn Ala Trp Ser His Asp Pro Val Val Phe
                   245                 250                 255
   Gly Lys Tyr Pro Glu Glu Ala Val Ala Leu Tyr Thr Glu Lys Gly Leu
               260                 265                 270
   Gln Val Leu Asp Ser Asp Met Asn Ile Ile Ser Thr Pro Ile Asp Phe
           275                 280                 285
   Phe Gly Val Asn Tyr Tyr Thr Arg Thr Leu Val Val Phe Asp Met Asn
       290                 295                 300
   Asn Pro Leu Gly Phe Ser Tyr Val Gln Gly Asp Leu Pro Lys Thr Glu
   305                 310                 315                 320
   Met Gly Trp Glu Ile Tyr Pro Gln Gly Leu Phe Asp Met Leu Val Tyr
                   325                 330                 335
   Leu Lys Glu Arg Tyr Lys Leu Pro Leu Tyr Ile Thr Glu Asn Gly Met
               340                 345                 350
   Ala Gly Pro Asp Lys Leu Glu Asn Gly Arg Val His Asp Asn Tyr Arg
           355                 360                 365
   Ile Glu Tyr Leu Glu Lys His Phe Glu Lys Ala Leu Glu Ala Ile Asn
       370                 375                 380
   Ala Asp Val Asp Leu Lys Gly Tyr Phe Ile Trp Ser Leu Met Asp Asn
   385                 390                 395                 400
   Phe Glu Trp Ala Cys Gly Tyr Ser Lys Arg Phe Gly Ile Ile Tyr Val
                   405                 410                 415
   Asp Tyr Asn Thr Pro Lys Arg Ile Leu Lys Asp Ser Ala Met Trp Leu
               420                 425                 430
   Lys Glu Phe Leu Lys Ser
           435
<210> 17
   <211> 421
   <212> PRT
   <213> Staphylothermus marinus
<400> 17
   Met Ile Arg Phe Pro Asp Tyr Phe Leu Phe Gly Thr Ala Thr Ser Ser
   1 5 10 15
   His Gln Ile Glu Gly Asn Asn Ile Phe Asn Asp Trp Trp Glu Trp Glu
   20 25 30
   Thr Lys Gly Arg Ile Lys Val Arg Ser Gly Lys Ala Cys Asn His Trp
   35 40 45
   Glu Leu Tyr Lys Glu Asp Ile Glu Leu Met Ala Glu Leu Gly Tyr Asn
   50 55 60
   Ala Tyr Arg Phe Ser Ile Glu Trp Ser Arg Ile Phe Pro Arg Lys Asp
   65 70 75 80
   His Ile Asp Tyr Glu Ser Leu Asn Lys Tyr Lys Glu Ile Val Asn Leu
   85 90 95
   Leu Arg Lys Tyr Gly Ile Glu Pro Val Ile Thr Leu His His Phe Thr
   100 105 110
   Asn Pro Gln Trp Phe Met Lys Ile Gly Gly Trp Thr Arg Glu Glu Asn
   115 120 125
   Ile Lys Tyr Phe Ile Lys Tyr Val Glu Leu Ile Ala Ser Glu Ile Lys
   130 135 140
   Asp Val Lys Ile Trp Ile Thr Ile Asn Glu Pro Ile Ile Tyr Val Leu
   145 150 155 160
   Gln Gly Tyr Ile Ser Gly Glu Trp Pro Pro Gly Ile Lys Asn Leu Lys
   165 170 175
   Ile Ala Asp Gln Val Thr Lys Asn Leu Leu Lys Ala His Asn Glu Ala
   180 185 190
   Tyr Asn Ile Leu His Lys His Gly Ile Val Gly Ile Ala Lys Asn Met
   195 200 205
   Ile Ala Phe Lys Pro Gly Ser Asn Arg Gly Lys Asp Ile Asn Ile Tyr
   210 215 220
   His Lys Val Asp Lys Ala Phe Asn Trp Gly Phe Leu Asn Gly Ile Leu
   225 230 235 240
   Arg Gly Glu Leu Glu Thr Leu Arg Gly Lys Tyr Arg Val Glu Pro Gly
   245 250 255
   Asn Ile Asp Phe Ile Gly Ile Asn Tyr Tyr Ser Ser Tyr Ile Val Lys
   260 265 270
   Tyr Thr Trp Asn Pro Phe Lys Leu His Ile Lys Val Glu Pro Leu Asp
   275 280 285
   Thr Gly Leu Trp Thr Thr Met Gly Tyr Cys Ile Tyr Pro Arg Gly Ile
   290 295 300
   Tyr Glu Val Val Met Lys Thr His Glu Lys Tyr Gly Lys Glu Ile Ile
   305 310 315 320
   Ile Thr Glu Asn Gly Val Ala Val Glu Asn Asp Glu Leu Arg Ile Leu
   325 330 335
   Ser Ile Ile Arg His Leu Gln Tyr Leu Tyr Lys Ala Met Asn Glu Gly
   340 345 350
   Ala Lys Val Lys Gly Tyr Phe Tyr Trp Ser Phe Met Asp Asn Phe Glu
   355 360 365
   Trp Asp Lys Gly Phe Asn Gln Arg Phe Gly Leu Val Glu Val Asp Tyr
   370 375 380
   Lys Thr Phe Glu Arg Lys Pro Arg Lys Ser Ala Tyr Val Tyr Ser Gln
   385 390 395 400
   Ile Ala Arg Thr Lys Thr Ile Ser Asp Glu Tyr Leu Glu Lys Tyr Gly
   405 410 415
   Leu Lys Asn Leu Glu
   420
<210> 18
   <211> 509
   <212> PRT
   <213> Thermococcus sp.
<400> 18
   Met Leu Pro Glu Gly Phe Leu Trp Gly Val Ser Gln Ser Gly Phe Gln
    1               5                  10                  15
   Phe Glu Met Gly Asp Lys Leu Arg Arg Asn Ile Asp Pro Asn Thr Asp
               20                  25                  30
   Trp Trp Lys Trp Val Arg Asp Pro Phe Asn Ile Lys Arg Glu Leu Val
           35                  40                  45
   Ser Gly Asp Leu Pro Glu Glu Gly Ile Asn Asn Tyr Glu Leu Tyr Glu
       50                  55                  60
   Lys Asp His Arg Leu Ala Arg Asp Leu Gly Leu Asn Val Tyr Arg Ile
   65                  70                  75                  80
   Gly Ile Glu Trp Ser Arg Ile Phe Pro Trp Pro Thr Trp Phe Val Glu
                   85                  90                  95
   Val Asp Val Glu Arg Asp Ser Tyr Gly Leu Val Lys Asp Val Lys Ile
               100                 105                 110
   Asp Lys Asp Thr Leu Glu Glu Leu Asp Glu Ile Ala Asn His Gln Glu
           115                 120                 125
   Ile Ala Tyr Tyr Arg Arg Val Ile Glu His Leu Arg Glu Leu Gly Phe
       130                 135                 140
   Lys Val Ile Val Asn Leu Asn His Phe Thr Leu Pro Leu Trp Leu His
   145                 150                 155                 160
   Asp Pro Ile Ile Ala Arg Glu Lys Ala Leu Thr Asn Gly Arg Ile Gly
                   165                 170                 175
   Trp Val Gly Gln Glu Ser Val Val Glu Phe Ala Lys Tyr Ala Ala Tyr
               180                 185                 190
   Ile Ala Asn Ala Leu Gly Asp Leu Val Asp Met Trp Ser Thr Phe Asn
           195                 200                 205
   Glu Pro Met Val Val Val Glu Leu Gly Tyr Leu Ala Pro Tyr Ser Gly
       210                 215                 220
   Phe Pro Pro Gly Val Met Asn Pro Glu Ala Ala Lys Leu Ala Ile Leu
   225                 230                 235                 240
   Asn Met Ile Asn Ala His Ala Leu Ala Tyr Lys Met Ile Lys Lys Phe
                   245                 250                 255
   Asp Arg Val Lys Ala Asp Lys Asp Ser Arg Ser Glu Ala Glu Val Gly
               260                 265                 270
   Ile Ile Tyr Asn Asn Ile Gly Val Ala Tyr Pro Tyr Asp Ser Asn Asp
           275                 280                 285
   Pro Lys Asp Val Lys Ala Ala Glu Asn Asp Asn Tyr Phe His Ser Gly
   290 295 300
   Leu Phe Phe Asp Ala Ile His Lys Gly Lys Leu Asn Ile Glu Phe Asp
   305 310 315 320
   Gly Glu Thr Phe Val Lys Val Arg His Leu Arg Gly Asn Asp Trp Ile
   325 330 335
   Gly Val Asn Tyr Tyr Thr Arg Glu Val Val Arg Tyr Ser Glu Pro Lys
   340 345 350
   Phe Pro Ser Ile Pro Leu Ile Ser Phe Arg Gly Val His Asn Tyr Gly
   355 360 365
   Tyr Ala Cys Arg Pro Gly Ser Ser Ser Ala Asp Gly Arg Pro Val Ser
   370 375 380
   Asp Ile Gly Trp Glu Ile Tyr Pro Glu Gly Ile Tyr Asp Ser Ile Arg
   385 390 395 400
   Glu Ala Asn Lys Tyr Gly Val Pro Val Tyr Val Thr Glu Asn Gly Ile
   405 410 415
   Ala Asp Ser Thr Asp Thr Leu Arg Pro Tyr Tyr Leu Ala Ser His Val
   420 425 430
   Ala Lys Ile Glu Glu Ala Tyr Glu Ala Gly Tyr Asp Val Arg Gly Tyr
   435 440 445
   Leu Tyr Trp Ala Leu Thr Asp Asn Tyr Glu Trp Ala Leu Gly Phe Arg
   450 455 460
   Met Arg Phe Gly Leu Tyr Lys Val Asp Leu Ile Thr Lys Glu Arg Thr
   465 470 475 480
   Pro Arg Glu Glu Ser Val Lys Val Tyr Arg Gly Ile Val Glu Asn Asn
   485 490 495
   Gly Val Ser Lys Glu Ile Arg Glu Lys Phe Gly Leu Gly
   500 505
<210> 19
   <211> 721
   <212> PRT
   <213> Thermotoga maritima
<400> 19
   Met Glu Arg Ile Asp Glu Ile Leu Ser Gln Leu Thr Thr Glu Glu Lys
    1               5                  10                  15
   Val Lys Leu Val Val Gly Val Gly Leu Pro Gly Leu Phe Gly Asn Pro
               20                  25                  30
   His Ser Arg Val Ala Gly Ala Ala Gly Glu Thr His Pro Val Pro Arg
           35                  40                  45
   Leu Gly Ile Pro Ala Phe Val Leu Ala Asp Gly Pro Ala Gly Leu Arg
       50                  55                  60
   Ile Asn Pro Thr Arg Glu Asn Asp Glu Asn Thr Tyr Tyr Thr Thr Ala
   65                  70                  75                  80
   Phe Pro Val Glu Ile Met Leu Ala Ser Thr Trp Asn Arg Asp Leu Leu
                   85                  90                  95
   Glu Glu Val Gly Lys Ala Met Gly Glu Glu Val Arg Glu Tyr Gly Val
               100                 105                 110
   Asp Val Leu Leu Ala Pro Ala Met Asn Ile His Arg Asn Pro Leu Cys
           115                 120                 125
   Gly Arg Asn Phe Glu Tyr Tyr Ser Glu Asp Pro Val Leu Ser Gly Glu
       130                 135                 140
   Met Ala Ser Ala Phe Val Lys Gly Val Gln Ser Gln Gly Val Gly Ala
   145                 150                 155                 160
   Cys Ile Lys His Phe Val Ala Asn Asn Gln Glu Thr Asn Arg Met Val
                   165                 170                 175
   Val Asp Thr Ile Val Ser Glu Arg Ala Leu Arg Glu Ile Tyr Leu Lys
               180                 185                 190
   Gly Phe Glu Ile Ala Val Lys Lys Ala Arg Pro Trp Thr Val Met Ser
           195                 200                 205
   Ala Tyr Asn Lys Leu Asn Gly Lys Tyr Cys Ser Gln Asn Glu Trp Leu
       210                 215                 220
   Leu Lys Lys Val Leu Arg Glu Glu Trp Gly Phe Gly Gly Phe Val Met
   225                 230                 235                 240
   Ser Asp Trp Tyr Ala Gly Asp Asn Pro Val Glu Gln Leu Lys Ala Gly
                   245                 250                 255
   Asn Asp Met Ile Met Pro Gly Lys Ala Tyr Gln Val Asn Thr Glu Arg
               260                 265                 270
   Arg Asp Glu Ile Glu Glu Ile Met Glu Ala Leu Lys Glu Gly Lys Leu
           275                 280                 285
   Ser Glu Glu Val Leu Asp Glu Cys Val Arg Asn Ile Leu Lys Val Leu
       290                 295                 300
   Val Asn Ala Pro Ser Phe Lys Gly Tyr Arg Tyr Ser Asn Lys Pro Asp
   305                 310                 315                 320
   Leu Glu Ser His Ala Glu Val Ala Tyr Glu Ala Gly Ala Glu Gly Val
                   325                 330                 335
   Val Leu Leu Glu Asn Asn Gly Val Leu Pro Phe Asp Glu Asn Thr His
               340                 345                 350
   Val Ala Val Phe Gly Thr Gly Gln Ile Glu Thr Ile Lys Gly Gly Thr
           355                 360                 365
   Gly Ser Gly Asp Thr His Pro Arg Tyr Thr Ile Ser Ile Leu Glu Gly
       370                 375                 380
   Ile Lys Glu Arg Asn Met Lys Phe Asp Glu Glu Leu Ala Ser Thr Tyr
   385 390 395 400
   Glu Glu Tyr Ile Lys Lys Met Arg Glu Thr Glu Glu Tyr Lys Pro Arg
   405 410 415
   Thr Asp Ser Trp Gly Thr Val Ile Lys Pro Lys Leu Pro Glu Asn Phe
   420 425 430
   Leu Ser Glu Lys Glu Ile Lys Lys Pro Pro Lys Lys Asn Asp Val Ala
   435 440 445
   Val Val Val Ile Ser Arg Ile Ser Gly Glu Gly Tyr Asp Arg Lys Pro
   450 455 460
   Val Lys Gly Asp Phe Tyr Leu Ser Asp Asp Glu Leu Glu Leu Ile Lys
   465 470 475 480
   Thr Val Ser Lys Glu Phe His Asp Gln Gly Lys Lys Val Val Val Leu
   485 490 495
   Leu Asn Ile Gly Ser Pro Ile Glu Val Ala Ser Trp Arg Asp Leu Val
   500 505 510
   Asp Gly Ile Leu Leu Val Trp Gln Ala Gly Gln Glu Met Gly Arg Ile
   515 520 525
   Val Ala Asp Val Leu Val Gly Lys Ile Asn Pro Ser Gly Lys Leu Pro
   530 535 540
   Thr Thr Phe Pro Lys Asp Tyr Ser Asp Val Pro Ser Trp Thr Phe Pro
   545 550 555 560
   Gly Glu Pro Lys Asp Asn Pro Gln Arg Val Val Tyr Glu Glu Asp Ile
   565 570 575
   Tyr Val Gly Tyr Arg Tyr Tyr Asp Thr Phe Gly Val Glu Pro Ala Tyr
   580 585 590
   Glu Phe Gly Tyr Gly Leu Ser Tyr Thr Lys Phe Glu Tyr Lys Asp Leu
   595 600 605
   Lys Ile Ala Ile Asp Gly Glu Thr Leu Arg Val Ser Tyr Thr Ile Thr
   610 615 620
   Asn Thr Gly Asp Arg Ala Gly Lys Glu Val Ser Gln Val Tyr Ile Lys
   625 630 635 640
   Ala Pro Lys Gly Lys Ile Asp Lys Pro Phe Gln Glu Leu Lys Ala Phe
   645 650 655
   His Lys Thr Lys Leu Leu Asn Pro Gly Glu Ser Glu Glu Ile Ser Leu
   660 665 670
   Glu Ile Pro Leu Arg Asp Leu Ala Ser Phe Asp Gly Lys Glu Trp Val
   675 680 685
   Val Glu Ser Gly Glu Tyr Glu Val Arg Val Gly Ala Ser Ser Arg Asp
   690 695 700
   Ile Arg Leu Arg Asp Ile Phe Leu Val Glu Gly Glu Lys Arg Phe Lys
   705                 710                 715                 720
   Pro
<210> 20
   <211> 454
   <212> PRT
   <213> Thermococcus alcaliphilus
<400> 20
   Met Ile His Cys Pro Val Lys Gly Ile Ile Ser Glu Ala Arg Gly Ile
   1 5 10 15
   Thr Ile Thr Ile Asp Leu Ser Phe Gln Gly Gln Ile Asn Asn Leu Val
   20 25 30
   Asn Ala Met Ile Val Phe Pro Glu Phe Phe Leu Phe Gly Thr Ala Thr
   35 40 45
   Ser Ser His Gln Ile Glu Gly Asp Asn Lys Trp Asn Asp Trp Trp Tyr
   50 55 60
   Tyr Glu Glu Ile Gly Lys Leu Pro Tyr Lys Ser Gly Lys Ala Cys Asn
   65 70 75 80
   His Trp Glu Leu Tyr Arg Glu Asp Ile Glu Leu Met Ala Gln Leu Gly
   85 90 95
   Tyr Asn Ala Tyr Arg Phe Ser Ile Glu Trp Ser Arg Leu Phe Pro Glu
   100 105 110
   Glu Gly Lys Phe Asn Glu Glu Ala Phe Asn Arg Tyr Arg Glu Ile Ile
   115 120 125
   Glu Ile Leu Leu Glu Lys Gly Ile Thr Pro Asn Val Thr Leu His His
   130 135 140
   Phe Thr Ser Pro Leu Trp Phe Met Arg Lys Gly Gly Phe Leu Lys Glu
   145 150 155 160
   Glu Asn Leu Lys Tyr Trp Glu Gln Tyr Val Asp Lys Ala Ala Glu Leu
   165 170 175
   Leu Lys Gly Val Lys Leu Val Ala Thr Phe Asn Glu Pro Met Val Tyr
   180 185 190
   Val Met Met Gly Tyr Leu Thr Ala Tyr Trp Pro Pro Phe Ile Lys Ser
   195 200 205
   Pro Phe Lys Ala Phe Lys Val Ala Ala Asn Leu Leu Lys Ala His Ala
   210 215 220
   Met Ala Tyr Asp Ile Leu His Gly Asn Phe Asp Val Gly Ile Val Lys
   225 230 235 240
   Asn Ile Pro Ile Met Leu Pro Ala Ser Asn Arg Glu Lys Asp Val Glu
   245 250 255
   Ala Ala Gln Lys Ala Asp Asn Leu Phe Asn Trp Asn Phe Leu Asp Ala
   260 265 270
   Ile Trp Ser Gly Lys Tyr Lys Gly Ala Phe Gly Thr Tyr Lys Thr Pro
   275 280 285
   Glu Ser Asp Ala Asp Phe Ile Gly Ile Asn Tyr Tyr Thr Ala Ser Glu
   290 295 300
   Val Arg His Ser Trp Asn Pro Leu Lys Phe Phe Phe Asp Ala Lys Leu
   305 310 315 320
   Ala Asp Leu Ser Glu Arg Lys Thr Asp Met Gly Trp Ser Val Tyr Pro
   325 330 335
   Lys Gly Ile Tyr Glu Ala Ile Ala Lys Val Ser His Tyr Gly Lys Pro
   340 345 350
   Met Tyr Ile Thr Glu Asn Gly Ile Ala Thr Leu Asp Asp Glu Trp Arg
   355 360 365
   Ile Glu Phe Ile Ile Gln His Leu Gln Tyr Val His Lys Ala Leu Asn
   370 375 380
   Asp Gly Phe Asp Leu Arg Gly Tyr Phe Tyr Trp Ser Phe Met Asp Asn
   385 390 395 400
   Phe Glu Trp Ala Glu Gly Phe Arg Pro Arg Phe Gly Leu Val Glu Val
   405 410 415
   Asp Tyr Thr Thr Phe Lys Arg Arg Pro Arg Lys Ser Ala Tyr Ile Tyr
   420 425 430
   Gly Glu Ile Ala Arg Glu Lys Lys Ile Lys Asp Glu Leu Leu Ala Lys
   435 440 445
   Tyr Gly Leu Pro Glu Leu
   450
<210> 21
   <211> 511
   <212> PRT
   <213> Thermococcus chitonophagus
<400> 21
   Met Leu Pro Glu Asn Phe Leu Trp Gly Val Ser Gln Ser Gly Phe Gln
    1               5                  10                  15
   Phe Glu Met Gly Asp Arg Leu Arg Arg His Ile Asp Pro Asn Thr Asp
               20                  25                  30
   Trp Trp Tyr Trp Val Arg Asp Glu Tyr Asn Ile Lys Lys Gly Leu Val
           35                  40                  45
   Ser Gly Asp Leu Pro Glu Asp Gly Ile Asn Ser Tyr Glu Leu Tyr Glu
       50                  55                  60
   Arg Asp Gln Glu Ile Ala Lys Asp Leu Gly Leu Asn Thr Tyr Arg Ile
   65                  70                  75                  80
   Gly Ile Glu Trp Ser Arg Val Phe Pro Trp Pro Thr Thr Phe Val Asp
                   85                  90                  95
   Val Glu Tyr Glu Ile Asp Glu Ser Tyr Gly Leu Val Lys Asp Val Lys
               100                 105                 110
   Ile Ser Lys Asp Ala Leu Glu Lys Leu Asp Glu Ile Ala Asn Gln Arg
           115                 120                 125
   Glu Ile Ile Tyr Tyr Arg Asn Leu Ile Asn Ser Leu Arg Lys Arg Gly
       130                 135                 140
   Phe Lys Val Ile Leu Asn Leu Asn His Phe Thr Leu Pro Ile Trp Leu
   145                 150                 155                 160
   His Asp Pro Ile Glu Ser Arg Glu Lys Ala Leu Thr Asn Lys Arg Asn
                   165                 170                 175
   Gly Trp Val Ser Glu Arg Ser Val Ile Glu Phe Ala Lys Phe Ala Ala
               180                 185                 190
   Tyr Leu Ala Tyr Lys Phe Gly Asp Ile Val Asp Met Trp Ser Thr Phe
           195                 200                 205
   Asn Glu Pro Met Val Val Ala Glu Leu Gly Tyr Leu Ala Pro Tyr Ser
       210                 215                 220
   Gly Phe Pro Pro Gly Val Met Asn Pro Glu Ala Ala Lys Leu Val Met
   225                 230                 235                 240
   Leu His Met Ile Asn Ala His Ala Leu Ala Tyr Arg Met Ile Lys Lys
                   245                 250                 255
   Phe Asp Arg Lys Lys Ala Asp Pro Glu Ser Lys Glu Pro Ala Glu Ile
               260                 265                 270
   Gly Ile Ile Tyr Asn Asn Ile Gly Val Thr Tyr Pro Phe Asn Pro Lys
           275                 280                 285
   Asp Ser Lys Asp Leu Gln Ala Ser Asp Asn Ala Asn Phe Phe His Ser
       290                 295                 300
   Gly Leu Phe Leu Thr Ala Ile His Arg Gly Lys Leu Asn Ile Glu Phe
   305                 310                 315                 320
   Asp Gly Glu Thr Phe Val Tyr Leu Pro Tyr Leu Lys Gly Asn Asp Trp
                   325                 330                 335
   Leu Gly Val Asn Tyr Tyr Thr Arg Glu Val Val Lys Tyr Gln Asp Pro
               340                 345                 350
   Met Phe Pro Ser Ile Pro Leu Ile Ser Phe Lys Gly Val Pro Asp Tyr
           355                 360                 365
   Gly Tyr Gly Cys Arg Pro Gly Thr Thr Ser Lys Asp Gly Asn Pro Val
       370                 375                 380
   Ser Asp Ile Gly Trp Glu Val Tyr Pro Lys Gly Met Tyr Asp Ser Ile
   385                 390                 395                 400
   Val Ala Ala Asn Glu Tyr Gly Val Pro Val Tyr Val Thr Glu Asn Gly
                   405                 410                 415
   Ile Ala Asp Ser Lys Asp Val Leu Arg Pro Tyr Tyr Ile Ala Ser His
               420                 425                 430
   Ile Glu Ala Met Glu Glu Ala Tyr Glu Asn Gly Tyr Asp Val Arg Gly
           435                 440                 445
   Tyr Leu His Trp Ala Leu Thr Asp Asn Tyr Glu Trp Ala Leu Gly Phe
       450                 455                 460
   Arg Met Arg Phe Gly Leu Tyr Glu Val Asn Leu Ile Thr Lys Glu Arg
   465                 470                 475                 480
   Lys Pro Arg Lys Lys Ser Val Arg Val Phe Arg Glu Ile Val Ile Asn
                   485                 490                 495
   Asn Gly Leu Thr Ser Asn Ile Arg Lys Glu Ile Leu Glu Glu Gly
               500                 505                 510
<210> 22
   <211> 510
   <212> PRT
   <213> Pyrococcus furiosus
<400> 22
   Met Phe Pro Glu Lys Phe Leu Trp Gly Val Ala Gln Ser Gly Phe Gln
    1               5                  10                  15
   Phe Glu Met Gly Asp Lys Leu Arg Arg Asn Ile Asp Thr Asn Thr Asp
               20                  25                  30
   Trp Trp His Trp Val Arg Asp Lys Thr Asn Ile Glu Lys Gly Leu Val
           35                  40                  45
   Ser Gly Asp Leu Pro Glu Glu Gly Ile Asn Asn Tyr Glu Leu Tyr Glu
       50                  55                  60
   Lys Asp His Glu Ile Ala Arg Lys Leu Gly Leu Asn Ala Tyr Arg Ile
   65                  70                  75                  80
   Gly Ile Glu Trp Ser Arg Ile Phe Pro Trp Pro Thr Thr Phe Ile Asp
                   85                  90                  95
   Val Asp Tyr Ser Tyr Asn Glu Ser Tyr Asn Leu Ile Glu Asp Val Lys
               100                 105                 110
   Ile Thr Lys Asp Thr Leu Glu Glu Leu Asp Glu Ile Ala Asn Lys Arg
           115                 120                 125
   Glu Val Ala Tyr Tyr Arg Ser Val Ile Asn Ser Leu Arg Ser Lys Gly
       130                 135                 140
   Phe Lys Val Ile Val Asn Leu Asn His Phe Thr Leu Pro Tyr Trp Leu
   145 150 155 160
   His Asp Pro Ile Glu Ala Arg Glu Arg Ala Leu Thr Asn Lys Arg Asn
   165 170 175
   Gly Trp Val Asn Pro Arg Thr Val Ile Glu Phe Ala Lys Tyr Ala Ala
   180 185 190
   Tyr Ile Ala Tyr Lys Phe Gly Asp Ile Val Asp Met Trp Ser Thr Phe
   195 200 205
   Asn Glu Pro Met Val Val Val Glu Leu Gly Tyr Leu Ala Pro Tyr Ser
   210 215 220
   Gly Phe Pro Pro Gly Val Leu Asn Pro Glu Ala Ala Lys Leu Ala Ile
   225 230 235 240
   Leu His Met Ile Asn Ala His Ala Leu Ala Tyr Arg Gln Ile Lys Lys
   245 250 255
   Phe Asp Thr Glu Lys Ala Asp Lys Asp Ser Lys Glu Pro Ala Glu Val
   260 265 270
   Gly Ile Ile Tyr Asn Asn Ile Gly Val Ala Tyr Pro Lys Asp Pro Asn
   275 280 285
   Asp Ser Lys Asp Val Lys Ala Ala Glu Asn Asp Asn Phe Phe His Ser
   290 295 300
   Gly Leu Phe Phe Glu Ala Ile His Lys Gly Lys Leu Asn Ile Glu Phe
   305 310 315 320
   Asp Gly Glu Thr Phe Ile Asp Ala Pro Tyr Leu Lys Gly Asn Asp Trp
   325 330 335
   Ile Gly Val Asn Tyr Tyr Thr Arg Glu Val Val Thr Tyr Gln Glu Pro
   340 345 350
   Met Phe Pro Ser Ile Pro Leu Ile Thr Phe Lys Gly Val Gln Gly Tyr
   355 360 365
   Gly Tyr Ala Cys Arg Pro Gly Thr Leu Ser Lys Asp Asp Arg Pro Val
   370 375 380
   Ser Asp Ile Gly Trp Glu Leu Tyr Pro Glu Gly Met Tyr Asp Ser Ile
   385 390 395 400
   Val Glu Ala His Lys Tyr Gly Val Pro Val Tyr Val Thr Glu Asn Gly
   405 410 415
   Ile Ala Asp Ser Lys Asp Ile Leu Arg Pro Tyr Tyr Ile Ala Ser His
   420 425 430
   Ile Lys Met Ile Glu Lys Ala Phe Glu Asp Gly Tyr Glu Val Lys Gly
   435 440 445
   Tyr Phe His Trp Ala Leu Thr Asp Asn Phe Glu Trp Ala Leu Gly Phe
   450 455 460
   Arg Met Arg Phe Gly Leu Tyr Glu Val Asn Leu Ile Thr Lys Glu Arg
   465 470 475 480
   Ile Pro Arg Glu Lys Ser Val Ser Ile Phe Arg Glu Ile Val Ala Asn
   485 490 495
   Asn Gly Val Thr Lys Lys Ile Glu Glu Glu Leu Leu Arg Gly
   500 505 510
<210> 23
   <211> 537
   <212> PRT
   <213> Bankia gouldi
<400> 23
   Met Arg Ile Arg Leu Ala Thr Leu Ala Leu Cys Ala Ala Leu Ser Pro
    1               5                  10                  15
   Val Thr Phe Ala Asp Asn Val Thr Val Gln Ile Asp Ala Asp Gly Gly
               20                  25                  30
   Lys Lys Leu Ile Ser Arg Ala Leu Tyr Gly Met Asn Asn Ser Asn Ala
           35                  40                  45
   Glu Ser Leu Thr Asp Thr Asp Trp Gln Arg Phe Arg Asp Ala Gly Val
       50                  55                  60
   Arg Met Leu Arg Glu Asn Gly Gly Asn Asn Ser Thr Lys Tyr Asn Trp
   65                  70                  75                  80
   Gln Leu His Leu Ser Ser His Pro Asp Trp Tyr Asn Asn Val Tyr Ala
                   85                  90                  95
   Gly Asn Asn Asn Trp Asp Asn Arg Val Ala Leu Ile Gln Glu Asn Leu
               100                 105                 110
   Pro Gly Ala Asp Thr Met Trp Ala Phe Gln Leu Ile Gly Lys Val Ala
           115                 120                 125
   Ala Thr Ser Ala Tyr Asn Phe Asn Asp Trp Glu Phe Asn Gln Ser Gln
       130                 135                 140
   Trp Trp Thr Gly Val Ala Gln Asn Leu Ala Gly Gly Gly Glu Pro Asn
   145                 150                 155                 160
   Leu Asp Gly Gly Gly Glu Ala Leu Val Glu Gly Asp Pro Asn Leu Tyr
                   165                 170                 175
   Leu Met Asp Trp Ser Pro Ala Asp Thr Val Gly Ile Leu Asp His Trp
               180                 185                 190
   Phe Gly Val Asn Gly Leu Gly Val Arg Arg Gly Lys Ala Lys Tyr Trp
           195                 200                 205
   Ser Met Asp Asn Glu Pro Gly Ile Trp Val Gly Thr His Asp Asp Val
       210                 215                 220
   Val Lys Glu Gln Thr Pro Val Glu Asp Phe Leu His Thr Tyr Phe Glu
   225                 230                 235                 240
   Thr Ala Lys Lys Ala Arg Ala Lys Phe Pro Gly Ile Lys Ile Thr Gly
   245 250 255
   Pro Val Pro Ala Asn Glu Trp Gln Trp Tyr Ala Trp Gly Gly Phe Ser
   260 265 270
   Val Pro Gln Glu Gln Gly Phe Met Ser Trp Met Glu Tyr Phe Ile Lys
   275 280 285
   Arg Val Ser Glu Glu Gln Arg Ala Ser Gly Val Arg Leu Leu Asp Val
   290 295 300
   Leu Asp Leu His Tyr Tyr Pro Gly Ala Tyr Asn Ala Glu Asp Ile Val
   305 310 315 320
   Gln Leu His Arg Thr Phe Phe Asp Arg Asp Phe Val Ser Leu Asp Ala
   325 330 335
   Asn Gly Val Lys Met Val Glu Gly Gly Trp Asp Asp Ser Ile Asn Lys
   340 345 350
   Glu Tyr Ile Phe Gly Arg Val Asn Asp Trp Leu Glu Glu Tyr Met Gly
   355 360 365
   Pro Asp His Gly Val Thr Leu Gly Leu Thr Glu Met Cys Val Arg Asn
   370 375 380
   Val Asn Pro Met Thr Thr Ala Ile Trp Tyr Ala Ser Met Leu Gly Thr
   385 390 395 400
   Phe Ala Asp Asn Gly Val Glu Ile Phe Thr Pro Trp Cys Trp Asn Thr
   405 410 415
   Gly Met Trp Glu Thr Leu His Leu Phe Ser Arg Tyr Asn Lys Pro Tyr
   420 425 430
   Arg Val Ala Ser Ser Ser Ser Leu Glu Glu Phe Val Ser Ala Tyr Ser
   435 440 445
   Ser Ile Asn Glu Ala Glu Asp Ala Met Thr Val Leu Leu Val Asn Arg
   450 455 460
   Ser Thr Ser Glu Thr His Thr Ala Thr Val Ala Ile Asp Asp Phe Pro
   465 470 475 480
   Leu Asp Gly Pro Tyr Arg Thr Leu Arg Leu His Asn Leu Pro Gly Glu
   485 490 495
   Glu Thr Phe Val Ser His Arg Asp Asn Ala Leu Glu Lys Gly Thr Val
   500 505 510
   Arg Ala Ser Asp Asn Thr Val Thr Leu Glu Leu Pro Pro Leu Ser Val
   515 520 525
   Thr Ala Ile Leu Leu Lys Ala Arg Pro
   530 535
<210> 24
   <211> 555
   <212> PRT
   <213> Thermotoga maritima
<400> 24
   Val Ile Cys Val Glu Ile Phe Gly Lys Thr Phe Arg Glu Gly Arg Phe
   1               5                   10                  15
   Val Leu Lys Glu Lys Asn Phe Thr Val Glu Phe Ala Val Glu Lys Ile
               20                  25                  30
   His Leu Gly Trp Lys Ile Ser Gly Arg Val Lys Gly Ser Pro Gly Arg
           35                  40                  45
   Leu Glu Val Leu Arg Thr Lys Ala Pro Glu Lys Val Leu Val Asn Asn
       50                  55                  60
   Trp Gln Ser Trp Gly Pro Cys Arg Val Val Asp Ala Phe Ser Phe Lys
   65                  70                  75                  80
   Pro Pro Glu Ile Asp Pro Asn Trp Arg Tyr Thr Ala Ser Val Val Pro
                   85                  90                  95
   Asp Val Leu Glu Arg Asn Leu Gln Ser Asp Tyr Phe Val Ala Glu Glu
               100                 105                 110
   Gly Lys Val Tyr Gly Phe Leu Ser Ser Lys Ile Ala His Pro Phe Phe
           115                 120                 125
   Ala Val Glu Asp Gly Glu Leu Val Ala Tyr Leu Glu Tyr Phe Asp Val
       130                 135                 140
   Glu Phe Asp Asp Phe Val Pro Leu Glu Pro Leu Val Val Leu Glu Asp
   145                 150                 155                 160
   Pro Asn Thr Pro Leu Leu Leu Glu Lys Tyr Ala Glu Leu Val Gly Met
                   165                 170                 175
   Glu Asn Asn Ala Arg Val Pro Lys His Thr Pro Thr Gly Trp Cys Ser
               180                 185                 190
   Trp Tyr His Tyr Phe Leu Asp Leu Thr Trp Glu Glu Thr Leu Lys Asn
           195                 200                 205
   Leu Lys Leu Ala Lys Asn Phe Pro Phe Glu Val Phe Gln Ile Asp Asp
       210                 215                 220
   Ala Tyr Glu Lys Asp Ile Gly Asp Trp Leu Val Thr Arg Gly Asp Phe
   225                 230                 235                 240
   Pro Ser Val Glu Glu Met Ala Lys Val Ile Ala Glu Asn Gly Phe Ile
                   245                 250                 255
   Pro Gly Ile Trp Thr Ala Pro Phe Ser Val Ser Glu Thr Ser Asp Val
               260                 265                 270
   Phe Asn Glu His Pro Asp Trp Val Val Lys Glu Asn Gly Glu Pro Lys
           275                 280                 285
   Met Ala Tyr Arg Asn Trp Asn Lys Lys Ile Tyr Ala Leu Asp Leu Ser
   290 295 300
   Lys Asp Glu Val Leu Asn Trp Leu Phe Asp Leu Phe Ser Ser Leu Arg
   305 310 315 320
   Lys Met Gly Tyr Arg Tyr Phe Lys Ile Asp Phe Leu Phe Ala Gly Ala
   325 330 335
   Val Pro Gly Glu Arg Lys Lys Asn Ile Thr Pro Ile Gln Ala Phe Arg
   340 345 350
   Lys Gly Ile Glu Thr Ile Arg Lys Ala Val Gly Glu Asp Ser Phe Ile
   355 360 365
   Leu Gly Cys Gly Ser Pro Leu Leu Pro Ala Val Gly Cys Val Asp Gly
   370 375 380
   Met Arg Ile Gly Pro Asp Thr Ala Pro Phe Trp Gly Glu His Ile Glu
   385 390 395 400
   Asp Asn Gly Ala Pro Ala Ala Arg Trp Ala Leu Arg Asn Ala Ile Thr
   405 410 415
   Arg Tyr Phe Met His Asp Arg Phe Trp Leu Asn Asp Pro Asp Cys Leu
   420 425 430
   Ile Leu Arg Glu Glu Lys Thr Asp Leu Thr Gln Lys Glu Lys Glu Leu
   435 440 445
   Tyr Ser Tyr Thr Cys Gly Val Leu Asp Asn Met Ile Ile Glu Ser Asp
   450 455 460
   Asp Leu Ser Leu Val Arg Asp His Gly Lys Lys Val Leu Lys Glu Thr
   465 470 475 480
   Leu Glu Leu Leu Gly Gly Arg Pro Arg Val Gln Asn Ile Met Ser Glu
   485 490 495
   Asp Leu Arg Tyr Glu Ile Val Ser Ser Gly Thr Leu Ser Gly Asn Val
   500 505 510
   Lys Ile Val Val Asp Leu Asn Ser Arg Glu Tyr His Leu Glu Lys Glu
   515 520 525
   Gly Lys Ser Ser Leu Lys Lys Arg Val Val Lys Arg Glu Asp Gly Arg
   530 535 540
   Asn Phe Tyr Phe Tyr Glu Glu Gly Glu Arg Glu
   545 550 555
<210> 25
   <211> 680
   <212> PRT
   <213> Thermotoga maritima
<400> 25
   Met Gly Ile Gly Gly Asp Asp Ser Trp Ser Pro Ser Val Ser Ala Glu
   1 5 10 15
   Phe Leu Leu Leu Ile Val Glu Leu Ser Phe Val Leu Phe Ala Ser Asp
                   20                  25                  30
   Glu Phe Val Lys Val Glu Asn Gly Lys Phe Ala Leu Asn Gly Lys Glu
               35                  40                  45
   Phe Arg Phe Ile Gly Ser Asn Asn Tyr Tyr Met His Tyr Lys Ser Asn
       50                  55                  60
   Gly Met Ile Asp Ser Val Leu Glu Ser Ala Arg Asp Met Gly Ile Lys
   65                  70                  75                  80
   Val Leu Arg Ile Trp Gly Phe Leu Asp Gly Glu Ser Tyr Cys Arg Asp
                   85                  90                  95
   Lys Asn Thr Tyr Met His Pro Glu Pro Gly Val Phe Gly Val Pro Glu
               100                 105                 110
   Gly Ile Ser Asn Ala Gln Ser Gly Phe Glu Arg Leu Asp Tyr Thr Val
           115                 120                 125
   Ala Lys Ala Lys Glu Leu Gly Ile Lys Leu Val Ile Val Leu Val Asn
       130                 135                 140
   Asn Trp Asp Asp Phe Gly Gly Met Asn Gln Tyr Val Arg Trp Phe Gly
   145                 150                 155                 160
   Gly Thr His His Asp Asp Phe Tyr Arg Asp Glu Lys Ile Lys Glu Glu
                   165                 170                 175
   Tyr Lys Lys Tyr Val Ser Phe Leu Val Asn His Val Asn Thr Tyr Thr
               180                 185                 190
   Gly Val Pro Tyr Arg Glu Glu Pro Thr Ile Met Ala Trp Glu Leu Ala
           195                 200                 205
   Asn Glu Pro Arg Cys Glu Thr Asp Lys Ser Gly Asn Thr Leu Val Glu
       210                 215                 220
   Trp Val Lys Glu Met Ser Ser Tyr Ile Lys Ser Leu Asp Pro Asn His
   225                 230                 235                 240
   Leu Val Ala Val Gly Asp Glu Gly Phe Phe Ser Asn Tyr Glu Gly Phe
                   245                 250                 255
   Lys Pro Tyr Gly Gly Glu Ala Glu Trp Ala Tyr Asn Gly Trp Ser Gly
               260                 265                 270
   Val Asp Trp Lys Lys Leu Leu Ser Ile Glu Thr Val Asp Phe Gly Thr
           275                 280                 285
   Phe His Leu Tyr Pro Ser His Trp Gly Val Ser Pro Glu Asn Tyr Ala
       290                 295                 300
   Gln Trp Gly Ala Lys Trp Ile Glu Asp His Ile Lys Ile Ala Lys Glu
   305                 310                 315                 320
   Ile Gly Lys Pro Val Val Leu Glu Glu Tyr Gly Ile Pro Lys Ser Ala
                   325                 330                 335
   Pro Val Asn Arg Thr Ala Ile Tyr Arg Leu Trp Asn Asp Leu Val Tyr
   340 345 350
   Asp Leu Gly Gly Asp Gly Ala Met Phe Trp Met Leu Ala Gly Ile Gly
   355 360 365
   Glu Gly Ser Asp Arg Asp Glu Arg Gly Tyr Tyr Pro Asp Tyr Asp Gly
   370 375 380
   Phe Arg Ile Val Asn Asp Asp Ser Pro Glu Ala Glu Leu Ile Arg Glu
   385 390 395 400
   Tyr Ala Lys Leu Phe Asn Thr Gly Glu Asp Ile Arg Glu Asp Thr Cys
   405 410 415
   Ser Phe Ile Leu Pro Lys Asp Gly Met Glu Ile Lys Lys Thr Val Glu
   420 425 430
   Val Arg Ala Gly Val Phe Asp Tyr Ser Asn Thr Phe Glu Lys Leu Ser
   435 440 445
   Val Lys Val Glu Asp Leu Val Phe Glu Asn Glu Ile Glu His Leu Gly
   450 455 460
   Tyr Gly Ile Tyr Gly Phe Asp Leu Asp Thr Thr Arg Ile Pro Asp Gly
   465 470 475 480
   Glu His Glu Met Phe Leu Glu Gly His Phe Gln Gly Lys Thr Val Lys
   485 490 495
   Asp Ser Ile Lys Ala Lys Val Val Asn Glu Ala Arg Tyr Val Leu Ala
   500 505 510
   Glu Glu Val Asp Phe Ser Ser Pro Glu Glu Val Lys Asn Trp Trp Asn
   515 520 525
   Ser Gly Thr Trp Gln Ala Glu Phe Gly Ser Pro Asp Ile Glu Trp Asn
   530 535 540
   Gly Glu Val Gly Asn Gly Ala Leu Gln Leu Asn Val Lys Leu Pro Gly
   545 550 555 560
   Lys Ser Asp Trp Glu Glu Val Arg Val Ala Arg Lys Phe Glu Arg Leu
   565 570 575
   Ser Glu Cys Glu Ile Leu Glu Tyr Asp Ile Tyr Ile Pro Asn Val Glu
   580 585 590
   Gly Leu Lys Gly Arg Leu Arg Pro Tyr Ala Val Leu Asn Pro Gly Trp
   595 600 605
   Val Lys Ile Gly Leu Asp Met Asn Asn Ala Asn Val Glu Ser Ala Glu
   610 615 620
   Ile Ile Thr Phe Gly Gly Lys Glu Tyr Arg Arg Phe His Val Arg Ile
   625 630 635 640
   Glu Phe Asp Arg Thr Ala Gly Val Lys Glu Leu His Ile Gly Val Val
   645 650 655
   Gly Asp His Leu Arg Tyr Asp Gly Pro Ile Phe Ile Asp Asn Val Arg
   660 665 670
   Leu Tyr Lys Arg Thr Gly Gly Met
   675 680
<210> 26
   <211> 512
   <212> PRT
   <213> Thermococcus chitonophagus
<400> 26
   Met Leu Pro Glu Glu Phe Leu Trp Gly Val Gly Gln Ser Gly Phe Gln
   1               5                   10                  15
   Phe Glu Met Gly Asp Lys Leu Arg Arg His Ile Asp Pro Asn Thr Asp
               20                  25                  30
   Trp Trp Lys Trp Val Arg Asp Pro Phe Asn Ile Lys Lys Glu Leu Val
           35                  40                  45
   Ser Gly Asp Leu Pro Glu Asp Gly Ile Asn Asn Tyr Glu Leu Phe Glu
       50                  55                  60
   Asn Asp His Lys Leu Ala Lys Gly Leu Gly Leu Asn Ala Tyr Arg Ile
   65                  70                  75                  80
   Gly Ile Glu Trp Ser Arg Ile Phe Pro Trp Pro Thr Trp Thr Val Asp
                   85                  90                  95
   Thr Glu Val Glu Phe Asp Thr Tyr Gly Leu Val Lys Asp Val Lys Ile
               100                 105                 110
   Asp Lys Ser Thr Leu Ala Glu Leu Asp Arg Leu Ala Asn Lys Glu Glu
           115                 120                 125
   Val Met Tyr Tyr Arg Arg Val Ile Gln His Leu Arg Glu Leu Gly Phe
       130                 135                 140
   Lys Val Phe Val Asn Leu Asn His Phe Thr Leu Pro Ile Trp Leu His
   145                 150                 155                 160
   Asp Pro Ile Val Ala Arg Glu Lys Ala Leu Thr Asn Asp Arg Ile Gly
                   165                 170                 175
   Trp Val Ser Gln Arg Thr Val Val Glu Phe Ala Lys Tyr Ala Ala Tyr
               180                 185                 190
   Ile Ala His Ala Leu Gly Asp Leu Val Asp Thr Trp Ser Thr Phe Asn
           195                 200                 205
   Glu Pro Met Val Val Val Glu Leu Gly Tyr Leu Ala Pro Tyr Ser Gly
       210                 215                 220
   Phe Pro Pro Gly Val Met Asn Pro Glu Ala Ala Lys Leu Ala Ile Leu
   225                 230                 235                 240
   Asn Met Ile Asn Ala His Ala Leu Ala Tyr Lys Met Ile Lys Arg Phe
   245 250 255
   Asp Thr Lys Lys Ala Asp Glu Asp Ser Lys Ser Pro Ala Asp Val Gly
   260 265 270
   Ile Ile Tyr Asn Asn Ile Gly Val Ala Tyr Pro Lys Asp Pro Asn Asp
   275 280 285
   Pro Lys Asp Val Lys Ala Ala Glu Asn Asp Asn Tyr Phe His Ser Gly
   290 295 300
   Leu Phe Phe Asp Ala Ile His Lys Gly Lys Leu Asn Ile Glu Phe Asp
   305 310 315 320
   Gly Glu Asn Phe Val Lys Val Arg His Leu Lys Gly Asn Asp Trp Ile
   325 330 335
   Gly Leu Asn Tyr Tyr Thr Arg Glu Val Val Arg Tyr Ser Glu Pro Lys
   340 345 350
   Phe Pro Ser Ile Pro Leu Ile Ser Phe Lys Gly Val Pro Asn Tyr Gly
   355 360 365
   Tyr Ser Cys Arg Pro Gly Thr Thr Ser Ala Asp Gly Met Pro Val Ser
   370 375 380
   Asp Ile Gly Trp Glu Val Tyr Pro Gln Gly Ile Tyr Asp Ser Ile Val
   385 390 395 400
   Glu Ala Thr Lys Tyr Ser Val Pro Val Tyr Val Thr Glu Asn Gly Val
   405 410 415
   Ala Asp Ser Ala Asp Thr Leu Arg Pro Tyr Tyr Ile Val Ser His Val
   420 425 430
   Ser Lys Ile Glu Glu Ala Ile Glu Asn Gly Tyr Pro Val Lys Gly Tyr
   435 440 445
   Met Tyr Trp Ala Leu Thr Asp Asn Tyr Glu Trp Ala Leu Gly Phe Ser
   450 455 460
   Met Arg Phe Gly Leu Tyr Lys Val Asp Leu Ile Ser Lys Glu Arg Ile
   465 470 475 480
   Pro Arg Glu Arg Ser Val Glu Ile Tyr Arg Arg Ile Val Gln Ser Asn
   485 490 495
   Gly Val Pro Lys Asp Ile Lys Glu Glu Phe Leu Lys Gly Glu Glu Lys
   500 505 510
<210> 27
   <211> 360
   <212> PRT
   <213> Thermotoga sp.
<400> 27
   Met Val Glu Arg His Phe Arg Tyr Val Leu Ile Cys Thr Leu Phe Leu
   1 5 10 15
   Val Met Leu Leu Ile Ser Ser Thr Gln Cys Gly Lys Asn Glu Pro Asn
   20 25 30
   Lys Arg Val Asn Ser Met Glu Gln Ser Val Ala Glu Ser Asp Ser Asn
   35 40 45
   Ser Ala Phe Glu Tyr Asn Lys Met Val Gly Lys Gly Val Asn Ile Gly
   50 55 60
   Asn Ala Leu Glu Ala Pro Phe Glu Gly Ala Trp Gly Val Arg Ile Glu
   65 70 75 80
   Asp Glu Tyr Phe Glu Ile Ile Lys Lys Arg Gly Phe Asp Ser Val Arg
   85 90 95
   Ile Pro Ile Arg Trp Ser Ala His Ile Ser Glu Lys Pro Pro Tyr Asp
   100 105 110
   Ile Asp Arg Asn Phe Leu Glu Arg Val Asn His Val Val Asp Arg Ala
   115 120 125
   Leu Glu Asn Asn Leu Thr Val Ile Ile Asn Thr His His Phe Glu Glu
   130 135 140
   Leu Tyr Gln Glu Pro Asp Lys Tyr Gly Asp Val Leu Val Glu Ile Trp
   145 150 155 160
   Arg Gln Ile Ala Lys Phe Phe Lys Asp Tyr Pro Glu Asn Leu Phe Phe
   165 170 175
   Glu Ile Tyr Asn Glu Pro Ala Gln Asn Leu Thr Ala Glu Lys Trp Asn
   180 185 190
   Ala Leu Tyr Pro Lys Val Leu Lys Val Ile Arg Glu Ser Asn Pro Thr
   195 200 205
   Arg Ile Val Ile Ile Asp Ala Pro Asn Trp Ala His Tyr Ser Ala Val
   210 215 220
   Arg Ser Leu Lys Leu Val Asn Asp Lys Arg Ile Ile Val Ser Phe His
   225 230 235 240
   Tyr Tyr Glu Pro Phe Lys Phe Thr His Gln Gly Ala Glu Trp Val Asn
   245 250 255
   Pro Ile Pro Pro Val Arg Val Lys Trp Asn Gly Glu Glu Trp Glu Ile
   260 265 270
   Asn Gln Ile Arg Ser His Phe Lys Tyr Val Ser Asp Trp Ala Lys Gln
   275 280 285
   Asn Asn Val Pro Ile Phe Leu Gly Glu Phe Gly Ala Tyr Ser Lys Ala
   290 295 300
   Asp Met Asp Ser Arg Val Lys Trp Thr Glu Ser Val Arg Lys Met Ala
   305 310 315 320
   Glu Glu Phe Gly Phe Ser Tyr Ala Tyr Trp Glu Phe Cys Ala Gly Phe
   325 330 335
   Gly Ile Tyr Asp Arg Trp Ser Gln Asn Trp Ile Glu Pro Leu Ala Thr
   340 345 350
   Ala Val Val Gly Thr Gly Lys Glu
   355 360
<210> 28
   <211> 772
   <212> PRT
   <213> Thermotoga maritima
<400> 28
   Met Asp Leu Thr Lys Val Gly Ile Ile Val Arg Leu Asn Glu Trp Gln
   1 5 10 15
   Ala Lys Asp Val Ala Lys Asp Arg Phe Ile Glu Ile Lys Asp Gly Lys
   20 25 30
   Ala Glu Val Trp Ile Leu Gln Gly Val Glu Glu Ile Phe Tyr Glu Lys
   35 40 45
   Pro Asp Thr Ser Pro Arg Ile Phe Phe Ala Gln Ala Arg Ser Asn Lys
   50 55 60
   Val Ile Glu Ala Phe Leu Thr Asn Pro Val Asp Thr Lys Lys Lys Glu
   65 70 75 80
   Leu Phe Lys Val Thr Val Asp Gly Lys Glu Ile Pro Val Ser Arg Val
   85 90 95
   Glu Lys Ala Asp Pro Thr Asp Ile Asp Val Thr Asn Tyr Val Arg Ile
   100 105 110
   Val Leu Ser Glu Ser Leu Lys Glu Glu Asp Leu Arg Lys Asp Val Glu
   115 120 125
   Leu Ile Ile Glu Gly Tyr Lys Pro Ala Arg Val Ile Met Met Glu Ile
   130 135 140
   Leu Asp Asp Tyr Tyr Tyr Asp Gly Glu Leu Gly Ala Val Tyr Ser Pro
   145 150 155 160
   Glu Lys Thr Ile Phe Arg Val Trp Ser Pro Val Ser Lys Trp Val Lys
   165 170 175
   Val Leu Leu Phe Lys Asn Gly Glu Asp Thr Glu Pro Tyr Gln Val Val
   180 185 190
   Asn Met Glu Tyr Lys Gly Asn Gly Val Trp Glu Ala Val Val Glu Gly
   195 200 205
   Asp Leu Asp Gly Val Phe Tyr Leu Tyr Gln Leu Glu Asn Tyr Gly Lys
   210 215 220
   Ile Arg Thr Thr Val Asp Pro Tyr Ser Lys Ala Val Tyr Ala Asn Asn
   225 230 235 240
   Gln Glu Ser Ala Val Val Asn Leu Ala Arg Thr Asn Pro Glu Gly Trp
   245 250 255
   Glu Asn Asp Arg Gly Pro Lys Ile Glu Gly Tyr Glu Asp Ala Ile Ile
   260 265 270
   Tyr Glu Ile His Ile Ala Asp Ile Thr Gly Leu Glu Asn Ser Gly Val
   275 280 285
   Lys Asn Lys Gly Leu Tyr Leu Gly Leu Thr Glu Glu Asn Thr Lys Gly
   290 295 300
   Pro Gly Gly Val Thr Thr Gly Leu Ser His Leu Val Glu Leu Gly Val
   305 310 315 320
   Thr His Val His Ile Leu Pro Phe Phe Asp Phe Tyr Thr Gly Asp Glu
   325 330 335
   Leu Asp Lys Asp Phe Glu Lys Tyr Tyr Asn Trp Gly Tyr Asp Pro Tyr
   340 345 350
   Leu Phe Met Val Pro Glu Gly Arg Tyr Ser Thr Asp Pro Lys Asn Pro
   355 360 365
   His Thr Arg Ile Arg Glu Val Lys Glu Met Val Lys Ala Leu His Lys
   370 375 380
   His Gly Ile Gly Val Ile Met Asp Met Val Phe Pro His Thr Tyr Gly
   385 390 395 400
   Ile Gly Glu Leu Ser Ala Phe Asp Gln Thr Val Pro Tyr Tyr Phe Tyr
   405 410 415
   Arg Ile Asp Lys Thr Gly Ala Tyr Leu Asn Glu Ser Gly Cys Gly Asn
   420 425 430
   Val Ile Ala Ser Glu Arg Pro Met Met Arg Lys Phe Ile Val Asp Thr
   435 440 445
   Val Thr Tyr Trp Val Lys Glu Tyr His Ile Asp Gly Phe Arg Phe Asp
   450 455 460
   Gln Met Gly Leu Ile Asp Lys Lys Thr Met Leu Glu Val Glu Arg Ala
   465 470 475 480
   Leu His Lys Ile Asp Pro Thr Ile Ile Leu Tyr Gly Glu Pro Trp Gly
   485 490 495
   Gly Trp Gly Ala Pro Ile Arg Phe Gly Lys Ser Asp Val Ala Gly Thr
   500 505 510
   His Val Ala Ala Phe Asn Asp Glu Phe Arg Asp Ala Ile Arg Gly Ser
   515 520 525
   Val Phe Asn Pro Ser Val Lys Gly Phe Val Met Gly Gly Tyr Gly Lys
   530 535 540
   Glu Thr Lys Ile Lys Arg Gly Val Val Gly Ser Ile Asn Tyr Asp Gly
   545 550 555 560
   Lys Leu Ile Lys Ser Phe Ala Leu Asp Pro Glu Glu Thr Ile Asn Tyr
   565 570 575
   Ala Ala Cys His Asp Asn His Thr Leu Trp Asp Lys Asn Tyr Leu Ala
               580                 585                 590
   Ala Lys Ala Asp Lys Lys Lys Glu Trp Thr Glu Glu Glu Leu Lys Asn
           595                 600                 605
   Ala Gln Lys Leu Ala Gly Ala Ile Leu Leu Thr Ser Gln Gly Val Pro
       610                 615                 620
   Phe Leu His Gly Gly Gln Asp Phe Cys Arg Thr Thr Asn Phe Asn Asp
   625                 630                 635                 640
   Asn Ser Tyr Asn Ala Pro Ile Ser Ile Asn Gly Phe Asp Tyr Glu Arg
                   645                 650                 655
   Lys Leu Gln Phe Ile Asp Val Phe Asn Tyr His Lys Gly Leu Ile Lys
               660                 665                 670
   Leu Arg Lys Glu His Pro Ala Phe Arg Leu Lys Asn Ala Glu Glu Ile
           675                 680                 685
   Lys Lys His Leu Glu Phe Leu Pro Gly Gly Arg Arg Ile Val Ala Phe
       690                 695                 700
   Met Leu Lys Asp His Ala Gly Gly Asp Pro Trp Lys Asp Ile Val Val
   705                 710                 715                 720
   Ile Tyr Asn Gly Asn Leu Glu Lys Thr Thr Tyr Lys Leu Pro Glu Gly
                   725                 730                 735
   Lys Trp Asn Val Val Val Asn Ser Gln Lys Ala Gly Thr Glu Val Ile
               740                 745                 750
   Glu Thr Val Glu Gly Thr Ile Glu Leu Asp Pro Leu Ser Ala Tyr Val
           755                 760                 765
   Leu Tyr Arg Glu
       770
<210> 29
   <211> 52
   **<212>** DNA
   <213> Artificial Sequence
<220>
   <223> Description of Artificial Sequence: artificial sequence
<400> 29
   ccgagaattc attaaagagg agaaattaac tatggtgaat gctatgattg tc          52
<210> 30
   <211> 31
   <212> DNA
   <213> Artificial Sequence
<220>
   <223> Description of Artificial Sequence: artificial sequence
<400 > 30
   cggaagatct tcatagctcc ggaagcccat a 31
<210> 31
   <211> 54
   <212> DNA
   <213> Artificial Sequence
<220>
   <223> Description of Artificial Sequence: artificial sequence
<400> 31
   ccgagaattc attaaagagg agaaattaac tatgataaga aggtccgatt ttcc 54
<210> 32
   <211> 31
   <212> DNA
   <213> Artificial Sequence
<220>
   <223> Description of Artificial Sequence: artificial sequence
<400> 32
   cggaagatct ttaagatttt agaaattcct t 31
<210> 33
   <211> 52
   <212> DNA
   <213> Artificial Sequence
<220>
   <223> Description of Artificial Sequence:artificial sequence
<400> 33
   ccgagaattc attaaagagg agaaattaac tatgctacca gaaggctttc tc 52
<210> 34
   <211> 31
   <212> DNA
   <213> Artificial Sequence
<220>
   <223> Description of Artificial Sequence:artificial sequence
<400> 34
   cggaggtacc tcacccaagt ccgaacttct c 31
<210> 35
   <211> 52
   <212> DNA
   <213> Artificial Sequence
<220>
   <223> Description of Artificial Sequence: artificial sequence
<400> 35
   ccgagaattc attaaagagg agaaattaac tatgataagg tttcctgatt at 52
<210> 36
   <211> 31
   <212> DNA
   <213> Artificial Sequence
<220>
   <223> Description of Artificial Sequence: artificial sequence
<400> 36
   cggaagatct ttattcgagg ttctttaatc c 31
<210> 37
   <211> 57
   <212> DNA
   <213> Artificial Sequence
<220>
   <223> Description of Artificial Sequence: artificial sequence
<400> 37
   ccgagaattc attcattaaa gaggagaaat taactatgct tccaggagaa ctttctc 57
<210> 38
   <211> 31
   <212> DNA
   <213> Artificial Sequence
<220>
   <223> Description of Artificial Sequence: artificial sequence
<400> 38
   cggaggatcc ctacccctcc tctaagatct c 31
<210> 39
   <211> 41
   <212> DNA
   <213> Artificial Sequence
<220>
   <223> Description of Artificial Sequence: artificial sequence
<400> 39
   aataatctag agcatgcaat tccccaaaga cttcatgata g 41
<210> 40
   <211> 32
   <212> DNA
   <213> Artificial Sequence
<220>
   <223> Description of Artificial Sequence: artificial sequence
<400> 40
   aataaaagct tactggatca gtgtaagatg ct 32
<210> 41
   <211> 52
   <212> DNA
   <213> Artificial Sequence
<220>
   <223> Description of Artificial Sequence: artificial sequence
<400> 41
   ccgacaattg attaaagagg agaaattaac tatggaaagg atcgatgaaa tt 52
<210> 42
   <211> 31
   <212> DNA
   <213> Artificial Sequence
<220>
   <223> Description of Artificial Sequence: artificial sequence
<400> 42
   cggaggtacc tcatggtttg aatctcttct c 31
<210> 43
   <211> 52
   <212> DNA
   <213> Artificial Sequence
<220>
   <223> Description of Artificial Sequence: artificial sequence
<400> 43
   ccgacaattg attaaagagg agaaattaac tatgttccct gaaaagttcc tt 52
<210> 44
   <211> 31
   <212> DNA
   <213> Artificial Sequence
<220>
   <223> Description of Artificial Sequence: artificial sequence
<400> 44
   cggaggtacc tcatcccctc agcaattcct c 31
<210> 45
   <211> 27
   <212> DNA
   <213> Artificial Sequence
<220>
   <223> Description of Artificial Sequence: artificial sequence
<90.0> 45
<210> 46
   <211> 35
   <212> DNA
   <213> Artificial Sequence
<220>
   <223> Description of Artificial Sequence: artificial sequence
<400> 46
   aataaaagct tccgggttgt acagcggtaa taggc 35
<210> 47
   <211> 60
   <212> DNA
   <213> Artificial Sequence
<220>
   <223> Description of Artificial Sequence: artificial sequence
<400> 47
   tttattgaat tcattaaaga ggagaaatta actatgatct gtgtggaaat attcggaaag 60
<210> 48
   <211> 40
   <212> DNA
   <213> Artificial Sequence
<220>
   <223> Description of Artificial Sequence: artificial sequence
<400> 48
   tctataaagc tttcattctc tctcaccctc ttcgtagaag 40
<210> 49
   <211> 54
   <212> DNA
   <213> Artificial Sequence
<220>
   <223> Description of Artificial Sequence: artificial sequence
<400> 49
   tttattcaat tgattaaaga ggagaaatta actatgggga ttggtggcga cgac 54
<210> 50
   <211> 36
   <212> DNA
   <213> Artificial Sequence
<220>
   <223> Description of Artificial Sequence: artificial sequence
<400> 50
   tttattaagc ttatcttttc atattcacat acctcc 36
<210> 51
   <211> 60
   <212> DNA
   <213> Artificial Sequence
<220>
   <223> Description of Artificial Sequence: artificial sequence
<400> 51
   tttattgaat tcattaaaga ggagaaatta actatgctac cagaagagtt cctatggggc 60
<210> 52
   <211> 39
   <212> DNA
   <213> Artificial Sequence
<220>
   <223> Description of Artificial Sequence: artificial sequence
<400> 52
   tttattaagc ttctcatcaa cggctatggt cttcatttc 39
<210> 53
   <211> 68
   <212> DNA
   <213> Artificial Sequence
<220>
   <223> Description of Artificial Sequence: artificial sequence
<400> 53
   aaaaaacaat tgaattcatt aaagaggaga aattaactat ggtagaaaga cacttcagat 60
   atgttctt 68
<210> 54
   <211> 36
   <212> DNA
   <213> Artificial Sequence
<220>
   <223> Description of Artificial Sequence: artificial sequence
<400> 54
   tttttcggat ccaattcttc atttactctt tgcctg 36
<210> 55
   <211> 56
   <212> DNA
   <213> Artificial Sequence
<220>
   <223> Description of Artificial Sequence: artificial sequence
<400> 55
   ttttggaatt cattaaagag gagaaattaa ctatggaact gatcatagaa ggttac 56
<210> 56
   <211> 36
   <212> DNA
   <213> Artificial Sequence
<220>
   <223> Description of Artificial Sequence: artificial sequence
<400> 56
   ataagaagct tttcactctc tgtacagaac gtacgc                               36